# EUROPÄISCHE PATENTSCHRIFT

(11) **EP 2 065 361 B1**
(45) Veröffentlichungstag und Bekanntmachung des Hinweises auf die Patenterteilung: **17.12.2014**
(21) Anmeldenummer: 08019433.5
(22) Anmeldetag: 06.11.2008
(51) Int. Cl.: C07C 69/54, C07C 39/367, C09K 19/12, G02F 1/1334

(54) **Polymerisierbare Verbindungen**
Polymerisable compounds
Composés polymérisables

(30) Priorität: 30.11.2007 DE 102007057680
(43) Veröffentlichungstag der Anmeldung: 03.06.2009
(73) Patentinhaber: Merck Patent GmbH, 64293 Darmstadt (DE)
(72) Erfinder: Bremer, Matthias, Dr., 64295 Darmstadt (DE); Goetz, Achim, 64665 Alsbach-Hähnlein (DE); Derow, Stephan, Dr., 64347 Griesheim (DE)

(56) Entgegenhaltungen:
- EP-A1- 1 378 557
- EP-A1- 1 498 468
- DE-A1- 4 434 976
- DE-A1- 19 521 457

## Beschreibung

Die vorliegende Erfindung betrifft neue polymerisierbare Verbindungen, Verfahren und Zwischenprodukte zu ihrer Herstellung, und ihre Verwendung für optische, elektrooptische und elektronische Zwecke, insbesondere in Flüssigkristall (FK)-Medien und FK-Anzeigen, vor allem in FK-Anzeigen des PS- (polymer stabilized) und PSA- (polymer sustained alignment) Typs.

Die derzeit verwendeten Flüssigkristallanzeigen (FK-Anzeigen) sind meist solche des TN-Typs (twisted nematic). Diese weisen allerdings den Nachteil einer starken Blickwinkelabhängigkeit des Kontrastes auf. Daneben sind sogenannte VA-Anzeigen (vertical alignment) bekannt, die einen breiteren Blickwinkel aufweisen. Die FK-Zelle einer VA-Anzeige enthält eine Schicht eines FK-Mediums zwischen zwei transparenten Elektroden, wobei das FK-Medium üblicherweise einen negativen Wert der dielektrischen (DK-) Anisotropie aufweist. Die Moleküle der FK-Schicht sind im ausgeschalteten Zustand senkrecht zu den Elektrodenflächen (homöotrop) oder gekippt homöotrop (engl. "tilted") orientiert. Bei Anlegen einer elektrischen Spannung an die Elektroden findet eine Umorientierung der FK-Moleküle parallel zu den Elektrodenflächen statt. Weiterhin sind OCB-Anzeigen (optically compensated bend) bekannt, die auf einem Doppelbrechungseffekt beruhen und eine FK-Schicht mit einer sogenannten "bend"-Orientierung und üblicherweise positiver (DK-) Anisotropie aufweisen. Bei Anlegen einer elektrischen Spannung findet eine Umorientierung der FK-Moleküle senkrecht zu den Elektrodenflächen statt. Darüber hinaus enthalten OCB-Anzeigen normalerweise einen oder mehrere doppelbrechende optische Retardationsfilme, um unerwünschte Lichtdurchlässigkeit der "bend"-Zelle im dunklen Zustand zu vermeiden. OCB-Anzeigen besitzen gegenüber TN-Anzeigen einen weiteren Blickwinkel und kürzere Schaltzeiten. Weiterhin sind IPS-Anzeigen (In-Plane-Switching) bekannt, die eine FK-Schicht zwischen zwei Substraten enthalten, wovon nur eines eine Elektrodenschicht mit üblicherweise kammförmiger Struktur aufweist. Dadurch wird bei Anlegen einer Spannung ein elektrisches Feld erzeugt, welches eine signifikante Komponente parallel zur FK-Schicht aufweist. Dies bewirkt eine Umorientierung der FK-Moleküle in der Schichtebene. Des weiteren wurden sogenannte FFS-Anzeigen (Fringe-Field-Switching) vorgeschlagen (siehe u.a. S.H. Jung et al., Jpn. J. Appl. Phys., Band 43, No. 3, 2004, 1028), die ebenfalls zwei Elektroden auf dem gleichen Substrat beinhalten, wovon jedoch im Gegensatz zu IPS-Anzeigen nur eine als strukturierte (kammförmige) Elektrode ausgebildet ist, und die andere Elektrode unstrukturiert ist. Dadurch wird ein starkes sogenanntes "fringe field" erzeugt, also ein starkes elektrisches Feld nahe am Rand der Elektroden und in der gesamten Zelle ein elektrisches Feld, welches sowohl eine starke vertikale als auch eine starke horizontale Komponente aufweist. Sowohl IPS-Anzeigen als auch FFS-Anzeigen weisen eine geringe Blickwinkelabhängigkeit des Kontrastes auf.

In VA-Anzeigen des neueren Typs ist die einheitliche Ausrichtung der FK-Moleküle auf mehrere kleinere Domänen innerhalb der FK-Zelle beschränkt. Zwischen diesen Domänen, auch als Tilt-Domänen (engl. "tilt domains") bezeichnet, können Disklinationen existieren. VA-Anzeigen mit Tilt-Domänen weisen, verglichen mit herkömmlichen VA-Anzeigen, eine größere Blickwinkelunabhängigkeit des Kontrastes und der Graustufen auf. Außerdem sind solche Anzeigen einfacher herzustellen, da eine zusätzliche Behandlung der Elektrodenoberfläche zur einheitlichen Orientierung der Moleküle im eingeschalteten Zustand, wie z.B. durch Reiben, nicht mehr notwendig ist. Stattdessen wird die Vorzugsrichtung des Kipp- oder Tiltwinkels (engl. "pretilt") durch eine spezielle Ausgestaltung der Elektroden kontrolliert. In den sogenannten MVA-Anzeigen (multidomain vertical alignment) wird dies üblicherweise dadurch erreicht, dass die Elektroden Erhebungen oder Vorsprünge (engl. "protrusions") aufweisen, die einen lokalen pretilt verursachen. Als Folge werden die FK-Moleküle beim Anlegen einer Spannung in verschiedenen, definierten Regionen der Zelle in unterschiedliche Richtungen parallel zu den Elektrodenflächen orientiert. Dadurch wird ein "kontrolliertes" Schalten erreicht und das Entstehen störender Disklinationslinien vermieden. Diese Anordnung verbessert zwar den Blickwinkel der Anzeige, führt aber zu einer Verringerung ihrer Lichtdurchlässigkeit. Ein Weiterentwicklung von MVA verwendet Protrusions nur auf einer Elektroden-Seite, die gegenüberliegende Elektrode weist hingegen Schlitze (engl. "slits") auf, was die Lichtdurchlässigkeit verbessert. Die geschlitzten Elektroden erzeugen beim Anlegen einer Spannung ein inhomogenes elektrisches Feld in der FK-Zelle, so dass weiterhin ein kontrolliertes Schalten erreicht wird. Zur weiteren Verbesserung der Lichtdurchlässigkeit können die Abstände zwischen den slits und protrusions vergrößert werden, was jedoch wiederum zu einer Verlängerung der Schaltzeiten führt. Beim sogenannten PVA (Patterned VA) kommt man ganz ohne Protrusions aus, indem man beide Elektroden auf den gegenüberliegenden Seiten durch Schlitze strukturiert, was zu einem erhöhten Kontrast und verbesserter Lichtdurchlässigkeit führt, aber technologisch schwierig ist und das Display empfindlicher gegen mechanische Einflüsse macht (Klopfen, engl. "tapping", etc.). Für viele Anwendungen, wie beispielsweise Monitore und vor allem TV-Bildschirme, ist jedoch eine Verkürzung der Schaltzeiten sowie eine Verbesserung des Kontrastes und der Luminanz (Transmission) der Anzeige gefragt.

Eine Weiterentwicklung stellen die sogenannten PS-Anzeigen (polymer stabilized) dar, die auch unter dem Begriff "PSA" (polymer sustained alignment) bekannt sind. Darin wird dem FK-Medium eine geringe Menge (zum Beispiel 0.3 Gew.%, typischerweise <1 Gew.%) einer polymerisierbaren Verbindung zugesetzt, welche nach Einfüllen in die FK-Zelle bei angelegter elektrischer Spannung zwischen den Elektroden in situ polymerisiert bzw. vernetzt wird, üblicherweise durch UV-Photopolymerisation. Als besonders geeignet hat sich der Zusatz von polymerisierbaren mesogenen oder flüssigkristallinen Verbindungen, auch als "reaktive Mesogene" (RM) bezeichnet, zur FK-Mischung erwiesen. Mittlerweile wird das PS(A)-Prizip in diversen klassischen FK-Anzeigen angenwendet. So sind beispielsweise PSA-VA-, PSA-OCB-, PS-IPS- und PS-TN-Anzeigen bekannt. Wie man in Testzellen nachweisen kann, führt das PSA-Verfahren zu einem pretilt in der Zelle. Bei PSA-OCB-Anzeigen kann man daher erreichen, dass die Bend-Struktur stabilisiert wird, so dass man ohne Offset-Spannung auskommt oder diese reduzieren kann. Im Falle von PSA-VA-Anzeigen wirkt sich dieser Pretilt positiv auf die Schaltzeiten aus. Für PSA-VA-Anzeigen kann ein Standard-MVA- bzw -PVA Pixel- und Elektroden-Layout verwendet werden. Darüber hinaus kann man aber beispielsweise mit nur einer strukturierten Elektrodenseite und ohne Protrusions auskommen, was die Herstellung wesentlich vereinfacht und gleichzeitig zu einem sehr guten Kontrast bei sehr guter Lichtdurchlässigkeit führt. PSA-VA-Anzeigen sind beispielsweise in JP 10-036847 A, EP 1 170 626 A2, EP 1 378 557 A1, EP 1 498 468 A1, US 2004/0191428 A1, US 2006/0066793 A1 und US 2006/0103804 A1 beschrieben. PSA-OCB-Anzeigen sind beispielsweise in T.-J- Chen et al., Jpn. J. Appl. Phys. 45, 2006, 2702-2704 und S. H. Kim, L.-C- Chien, Jpn. J. Appl. Phys. 43, 20Ö4, 7643-7647 beschrieben. PS-IPS-Anzeigen sind zum Beispiel in US 6,177,972 und Appl. Phys. Lett. 1999, 75(21), 3264 beschrieben. PS-TN-Anzeigen sind zum Beispiel in Optics Express 2004, 12(7), 1221 beschrieben.

Insbesondere für Monitor- und vor allem TV-Anwendungen ist nach wie vor die Optimierung der Schaltzeiten, wie aber auch des Kontrastes und der Luminanz (also auch Transmission) der FK-Anzeige gefragt. Hier kann das PSA-Verfahren entscheidende Vorteile bringen. Insbesondere beim PSA-VA kann man ohne nennenswerte Einbußen sonstiger Parameter eine Verkürzung der Schaltzeiten erreichen, die mit einem in Testzellen messbaren Pretilt korrelieren.

Es hat sich jedoch gezeigt, dass bei Verwendung in PS(A)-Anzeigen die aus dem Stand der Technik bekannten FK-Mischungen und RMs noch einige Nachteile aufweisen. So eignet sich bei weitem nicht jedes beliebige lösliche RM zur Verwendung in PS(A)-Anzeigen, und es ist oft schwierig, geeignetere Auswahlkriterien als eben das direkte PSA-Experiment mit pretilt-Messung zu finden. Noch kleiner wird die Auswahl, wenn eine Polymerisation mittels UV-Licht ohne den Zusatz von Photoinitiatoren gewünscht ist, was für bestimmte Anwendungen von Vorteil sein kann. Darüber hinaus sollte das gewählte "Materialsystem" FK-Mischung (nachfolgend auch als "FK-Hostmischung" bezeichnet) + polymerisierbare Komponente eine möglichst geringe Rotationsviskosität sowie möglichst gute elektrische Eigenschaften aufweisen, hierbei ist die sog. "Voltage Holding Ratio" (HR oder VHR) hervorzuheben. Im Zusammenhang mit PSA-VA ist insbesondere eine hohe HR nach Bestrahlung mit (UV-) Licht von zentraler Bedeutung, da dies ein unverzichtbarer Teil des Prozesses ist, aber natürlich auch als "normale" Belastung im fertigen Display auftritt.

Es ergibt sich jedoch das Problem, dass bei weitem nicht alle Kombinationen FK-Mischung + polymerisierbare Komponente für PS(A)-Anzeigen geeignet sind, weil sich zum Beispiel kein oder kein ausreichender Tilt einstellt, oder weil zum Beispiel die HR für TFT-Display-Anwendungen unzureichend ist.

Es besteht somit immer noch ein großer Bedarf nach PS(A)-Anzeigen, insbesondere vom VA- und OCB-Typ, sowie FK-Medien und polymerisierbaren Verbindungen zur Verwendung in solchen Anzeigen, welche die oben beschriebenen Nachteile nicht oder nur in geringem Maße zeigen und verbesserte Eigenschaften besitzen. Insbesondere besteht ein großer Bedarf nach PS(A)-Anzeigen bzw. -Materialien mit einem hohen spezifischen Widerstand bei gleichzeitig großem Arbeitstemperaturbereich, kurzen Schaltzeiten auch bei tiefen Temperaturen und niedriger Schwellenspannung, die eine Vielzahl von Graustufen, einen hohen Kontrast und einen weiten Blickwinkel ermöglichen, sowie hohe Werte der "voltage holding ratio" (HR) nach UV-Belastung aufweisen.

Der Erfindung liegt die Aufgabe zugrunde, neue geeignete Materialien, insbesondere polymerisierbare Verbindungen und diese enthaltende FK-Medien, für die Verwendung in PS(A)-Anzeigen bereitzustellen, welche die oben angegebenen Nachteile nicht oder nur in geringerem Maße aufweisen, die Einstellung eines Pretilt-Winkels ermöglichen und vorzugsweise gleichzeitig sehr hohe spezifische Widerstände, niedrige Schwellenspannungen und niedrige Schaltzeiten besitzen.

Weitere Aufgabe der Erfindung ist die Bereitstellung von neuen polymerisierbaren mesogenen Verbindungen (reaktive Mesogene, "RM"), insbesondere für optische, elektrooptische, elektronische, dekorative und kosmetische Anwendungen, sowie von geeigneten Verfahren und Zwischenprodukten zu ihrer Herstellung.

Diese Aufgabe wurde erfindungsgemäß gelöst durch Materialien und Verfahren wie in der vorliegenden Anmeldung beschrieben. Insbesondere wurde überraschend gefunden, dass erfindungsgemäße polymerisierbare Verbindungen bei der Verwendung in PS(A)-Anzeigen die Einstellung der gewünschten Tiltwinkel ermöglichen. Dies konnte in Verbindung mit einem FK-Medium mittels Pretilt-Messungen nachgewiesen werden. Insbesondere konnte ein Pretilt ohne den Zusatz von Photoinitiator erreicht werden.

DE 44 34 976 A1 offenbart FK-Verbindungen mit einer Alkenoatseitenkette. DE 195 21 457 offenbart polymerisierbare Biphenyle mit Fluoratomen in 2,3-Position. Verbindungen wie in der vorliegenden Anmeldung beschrieben und beansprucht werden in diesen Dokumenten jedoch nicht offenbart.

### Gegenstand der Erfindung sind somit Verbindungen der Formel I

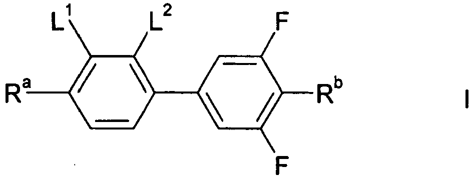

worin die einzelnen Reste folgende Bedeutung besitzen
- R^{a} und R^{b}: P-Sp-,
- P: bei jedem Auftreten gleich oder verschieden Vinyloxy, Acrylat. Methacrylat, Fluoracrylat, Chloracrylat, Oetan oder Epoxy,
- Sp: eine Einfachbindung,
- L¹ und L²: jeweils unabhängig voneinander H oder F.

Weiterer Gegenstand der Erfindung sind neue Verfahren zur Herstellung von Verbindungen der Formel I, sowie darin erhaltene bzw. verwendete Zwischenprodukte, insbesondere Verbindungen der Formel Ia 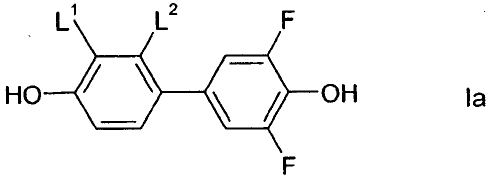 worin L¹ und L² die in Formel I angegebene Bedeutung besitzen.

Ein weiterer Gegenstand der Erfindung ist ein FK-Medium enthaltend eine oder mehrere polymerisierbare Verbindungen der Formel I und eine oder mehrere zusätzliche Verbindungen, welche auch mesogen, flüssigkristallin und/oder polymerisierbar sein können.

Ein weiterer Gegenstand der Erfindung ist ein FK-Medium enthaltend
- eine polymerisierbare Komponente A), enthaltend eine oder mehrere polymerisierbare Verbindungen der Formel I, sowie
- eine flüssigkristalline Komponente B), im Folgenden auch als "FK-Hostmischung" bezeichnet, enthaltend eine oder mehrere, vorzugsweise zwei oder mehr niedermolekulare (d.h. monomere bzw. unpolymerisierte) Verbindungen wie vor- und nachstehend beschrieben.

Weiterer Gegenstand der Erfindung ist ein Polymer erhältlich durch Polymerisation einer oder mehrerer Verbindungen der Formel I oder eines erfindungsgemäßen FK-Mediums.

Weiterer Gegenstand der Erfindung ist eine Polymerfolie erhältlich durch Polymerisation einer Schicht, enthaltend eine oder mehrere Verbindungen der Formel I oder ein erfindungsgemäßes FK-Medium, im einheitlich ausgerichteten Zustand in der FK-Phase, vorzugsweise in der nematischen oder cholesterischen Phase.

Weiterer Gegenstand der Erfindung ist die Verwendung von Verbindungen der Formel I, erfindungsgemäßen FK-Medien, Polymeren und Polymerfolien in elektrooptischen Anzeigen, FK-Anzeigen, optischen Filmen, Polarisatoren, Kompensatoren, Strahlenteilern, Reflektivpolarisatoren, Orientierungsschichten, Farbfilteren, holographischen Elementen, Heissprägefolien, Adhäsionsfolien, optischen Datenspeichern, in der nichtlinearen Optik, Effektpigmenten, dekorativen Elementen, Sicherheitselementen, Sicherheitsmarkierungen, elektrischen Halbleitern, organischen Feldeffekttransistoren (OFET), integrierten Schaltkreise (IC), Dünnfilmtransistoren (TFT), Radiofrequenz-Identifikationselementen (RFID), organischen Leuchtdioden (OLED), elektrolumineszierenden Anzeigen, Beleuchtungselementen, photovoltaischen Vorrichtungen, optischen Sensoren, Photoleitern, elektrophotographischen Anwendungen, oder kosmetischen Formulierungen oder Anwendungen.

Weiterer Gegenstand der Erfindung ist die Verwendung von Verbindungen der Formel I, erfindungsgemäßen FK-Medien und Polymeren in PS- und PSA-Anzeigen. Weiterer Gegenstand der Erfindung ist eine FK-Anzeige enthaltend eine oder mehrere Verbindungen der Formel I oder ein erfindungsgemäßes FK-Medium, insbesondere eine PS-oder PSA-Anzeige, besonders bevorzugt eine PSA-VA-, PSA-OCB-, PS-IPS-, PS-FFS- oder PS-TN-Anzeige.

Weiterer Gegenstand der Erfindung ist eine PS- oder PSA-Anzeige enthaltend eine FK-Zelle bestehend aus zwei Substraten, wobei mindestens ein Substrat lichtdurchlässig ist und mindestens ein Substrat eine Elektrodenschicht aufweist, sowie einer zwischen den Substraten befindlichen Schicht eines FK-Mediums enthaltend eine polymerisierte Komponente und eine niedermolekulare Komponente, wobei die polymerisierte Komponente erhältlich ist durch Polymerisation einer oder mehrerer polymerisierbarer Verbindungen zwischen den Substraten der FK-Zelle im FK-Medium unter Anlegen einer elektrischen Spannung, dadurch gekennzeichnet, dass sie eine oder mehrere Verbindungen der Formel I oder ein erfindungsgemäßes FK-Medium enthält.

Besonders bevorzugte Verbindungen der Formel I sind solche worin
- R^{a} und R^{b} gleiche oder verschiedene Reste P-Sp- bedeuten,
- R^{a} und R^{b} gleiche oder verschiedene Reste P-Sp- bedeuten, worin einer oder beide Reste Sp eine Einfachbindung bedeuten,
- Sp eine Einfachbindung bedeutet,
   Besonders bevorzugte Verbindungen der Formel I und Ia sind solche worin mindestens einer der Reste L¹ und L² F bedeutet, insbesondere worin
- einer der Reste L¹ und L² F und der andere H bedeutet,
- L¹ F und L² H bedeutet,
- L¹ H und L² F bedeutet,
- L¹ und L² F bedeuten.
Besonders bevorzugte Verbindungen der Formel I sind ausgewählt aus den folgenden Unterformeln 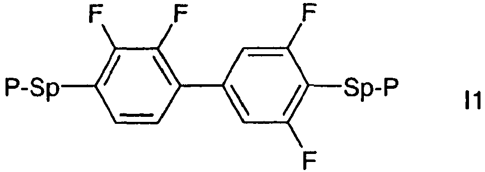 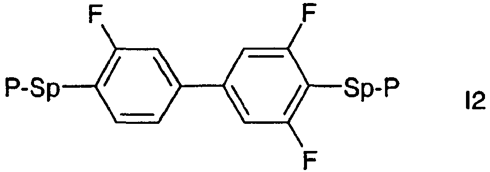 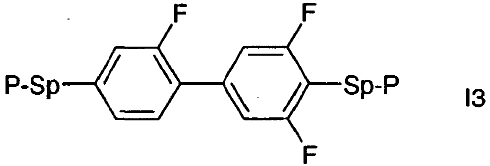 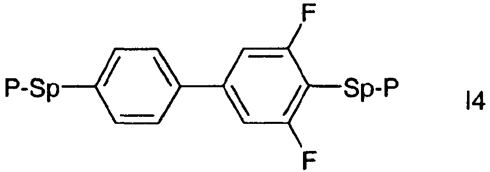 worin P und Sp jeweils unabhängig voneinander eine der vor- und nachstehend angegebenen Bedeutungen besitzen.

Besonders bevorzugte Verbindungen der Formel Ia sind ausgewählt aus den folgenden Unterformeln

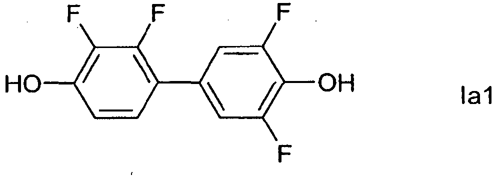

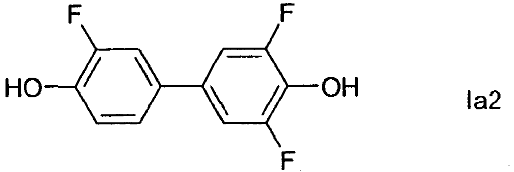

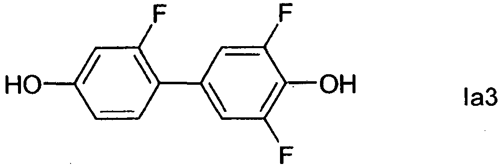

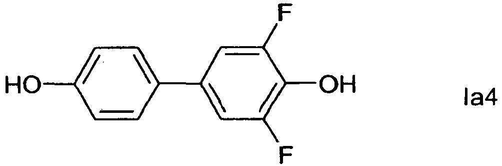

Vor- und nachstehend gelten folgende Bedeutungen:
Der Begriff "PSA" wird, falls nicht anders angegeben, stellvertretend für PS-Anzeigen und PSA-Anzeigen verwendet.

Der Begriff "mesogene Gruppe" ist dem Fachmann bekannt und in der Literatur beschrieben, und bedeutet eine Gruppe, die durch die Anisotropie ihrer anziehenden und abstoßenden Wechselwirkungen wesentlich dazu beiträgt, in niedermolekularen oder polymeren Substanzen eine Flüssigkristall(FK-)Phase hervorzurufen. Verbindungen enthaltend mesogene Gruppen (mesogene Verbindungen) müssen nicht unbedingt selbst eine FK-Phase aufweisen. Es ist auch möglich, dass mesogene Verbindungen FK-Phasenverhalten nur nach Vermischung mit anderen Verbindungen und/oder nach Polymerisation zeigen. Typische mesogene Gruppen sind beispielsweise starre stäbchen- oder scheibchenförmige Einheiten. Ein Überblick über die im Zusammenhang mit mesogenen bzw. FK-Verbindungen verwendeten Begriffe und Definitionen findet sich in Pure Appl. Chem. 73(5), 888 (2001) und C. Tschierske, G. Pelzl, S. Diele, Angew. Chem. 2004, 116, 6340-6368.

Der Begriff "Abstandsgruppe" (engl. "spacer" oder "spacer group"), vor- und nachstehend auch als "Sp" bezeichnet, ist dem Fachmann bekannt und in der Literatur beschrieben, siehe beispielsweise Pure Appl. Chem. 73(5), 888 (2001) und C. Tschierske, G. Pelzl, S. Diele, Angew. Chem. 2004, 116, 6340-6368. Falls nicht anders angegeben, bezeichnet der Begriff "Abstandsgruppe" bzw. "Spacer" vor- und nachstehend eine flexible Gruppe, die in einer polymerisierbaren mesogenen Verbindung ("RM") die mesogene Gruppe und die polymerisierbare(n) Gruppe(n) miteinander verbindet.

Bevorzugte Reste R^{a} und R^{b} sind optional substituiertes Alkyl, Alkenyl, Alkinyl, Alkoxy, Alkylcarbonyl, Alkoxycarbonyl, Alkylcarbonyloxy und Alkoxycarbonyloxy mit 1 bis 20, vorzugsweise 1 bis 12 C-Atomen.

Bevorzugte Alkylgruppen sind beispielsweise Methyl, Ethyl, n-Propyl, Isopropyl, n-Butyl, Isobutyl, s-Butyl, t-Butyl, 2-Methylbutyl, n-Pentyl, s-Pentyl, Cyclopentyl, n-Hexyl, Cyclohexyl, 2-Ethylhexyl, n-Heptyl, Cycloheptyl, n-Octyl, Cyclooctyl, n-Nonyl, n-Decyl, n-Undecyl, n-Dodecyl, Dodecanyl, Trifluoromethyl, Perfluoro-n-butyl, 2,2,2-Trifluoroethyl, Perfluorooctyl, Perfluorohexyl etc.

Bevorzugte Alkenylgruppen sind beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl etc.

Bevorzugte Alkinylgruppen sind beispielsweise Ethinyl, Propinyl, Butinyl, Pentinyl, Hexinyl, Octinyl etc.

Bevorzugte Alkoxygruppen sind beispielsweise Methoxy, Ethoxy, 2-Methoxyethoxy, n-Propoxy, i-Propoxy, n-Butoxy, i-Butoxy, s-Butoxy, t-Butoxy, 2-Methylbutoxy, n-Pentoxy, n-Hexoxy, n-Heptoxy, n-Octoxy, n-Nonoxy, n-Decoxy, n-Undecoxy, n-Dodecoxy, etc.

Die Verbindungen der Formel I und Ia können in Analogie zu dem Fachmann bekannten und in Standardwerken der organischen Chemie beschriebenen Verfahren, wie beispielsweise in Houben-Weyl, Methoden der organischen Chemie, Thieme-Verlag, Stuttgart, hergestellt werden. Die Synthese von polymerisierbaren Verbdinungen der Formel I, worin P eine Acrylatgruppe oder ein Acrylatderivat bedeutet (wie zum Beispiel Methacrylat) kann in Analogie zu den in US 5,723,066 beschriebenen Methoden durchgeführt werden. Bevorzugte Methoden finden sich in den Beispielen. Beispielsweise erfolgt die Synthese von Verbindungen der Formel I durch Veresterung oder Veretherung von Diolen der Formel Ia, wie zum Beispiel 2,3,3',5'-Tetrafluoro-4,4'-dihydroxybiphenyl, mit entsprechenden Säuren, Säurederivaten, oder halogenierten Verbindungen enthaltend eine Gruppe P, wie zum Beispiel (Meth)acrylsäurechlorid oder (Meth)acrylsäure, in Gegenwart von einem wasserentziehenden Reagens wie zum Beispiel DCC (Dicyclohexylcarbodiimid).

Ein besonders bevorzugtes Verfahren zur Herstellung von Verbindungen der Formel Ia1 ist in Schema 1 beispielhaft dargestellt und enthält folgende Schritte:
a) Reaktion der OH-Gruppe von 2,3-Difluorphenol (1) mit einer Schutzgruppe, zum Beispiel ein Trialkylsilylether,
b) Metallierung des Produkts (2) aus Schritt a) in para-Position zur geschützten Phenolgruppe und anschließende Reaktion mit einer Boronsäure oder einem Boronsäureester,
c) Kopplung des Produkts (3) aus Schritt b) mit 4-Halogen-2,6-difluorphenol (4) in Gegenwart eines Übergangsmetallkatalysators und Abspaltung der Schutzgruppe zu 2,3,3',5'-Tetrafluoro-biphenyl-4,4'-diol (5).

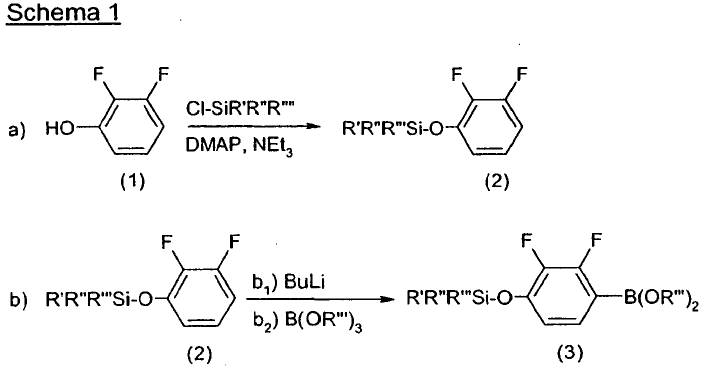

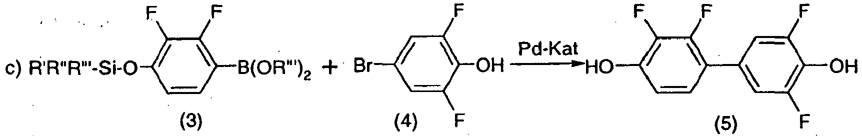

Darin bedeuten R', R" = Alkyl, zum Beispiel Methyl; R"' = Alkyl, beispielsweise t-butyl, R"" = H oder Alkyl, zwei Reste OR"" können auch zusammen mit dem Boratom einen cyclischen Rest bilden; DMAP = Dimethylamino-pyridin; NEt₃ = Triethylamin; BuLi = n-Butyllithium; Pd-Kat = Pd(II)-Katalysator, beispielsweise Bis(triphenylphosphino)palladium(II)-chlorid.

Der Schutz der Phenolgruppe in Schritt a) kann zum Beispiel durch Veretherung mit Trialkylchlorsilan erfolgen. In Schritt b) kann eine Boronsäure oder ein offenkettiger oder cyclischer Boronsäureester verwendet werden. Das Halogenid (4) in Schritt c) ist zum Beispiel 4-Brom-2,6,-difluorphenol, anstelle des Bromids kann aber auch ein Iodid oder eine andere geeignete Abgangsgruppe mit vergleichbarer Reaktivität (wie zum Beispiel Triflat) verwendet werden. Der in Schritt c) eingesetzte Übergangsmetallkatalysator ist bevorzugt ein Palladiumkomplex der Oxidationsstufen 0, II oder IV. Die Reaktion erfolgt bevorzugt in homogener Phase mit einem löslichen Katalysator. Ein bevorzugter Komplex ist zum Beispiel Bis(triphenylphosphin)palladium(II)chlorid.

Alternativ zu dem vor- und nachstehend beschriebenen Verfahren können die reaktiven Gruppen der Reaktionspartner (3) und (4) (Boronsäure-derivat und Halogenid) auch ausgetauscht werden, oder es wird in Schritt a) 2,6-Difluorphenol anstelle von Verbindung (1), und in Schritt c) 4-Brom(oder Iod)-2,3-difluorphenol anstelle von Verbindung (4) verwendet.

Ein besonders bevorzugtes Verfahren zur Herstellung der Verbindungen der Formel Ia2 und Ia3 ist in Schema 2 beispielhaft dargestellt und enthält folgende Schritte:
d) Reaktion der OH-Gruppe von 4-Halogen-2,6-Difluorphenol (6) mit einer Schutzgruppe, zum Beispiel ein Benzylhalogenid.
e) Dehalogenierung des Produkts (7) aus Schritt d) in para-Position zur geschützten Phenolethergruppe und anschließende Reaktion mit einer Boronsäure oder einem Boronsäureester,
f) Kopplung des Produkts (8) aus Schritt e) mit 4-Halogen-2-fluorphenol (9) bzw. 4-Halogen-3-fluorphenol (10) in Gegenwart eines Übergangsmetallkatalysators und Abspaltung der Schutzgruppe zu 2,3',5'-Trifluoro-biphenyl-4,4'-diol (11) bzw. 3,3',5'-Trifluoro-biphenyl-4,4'-diol (12).

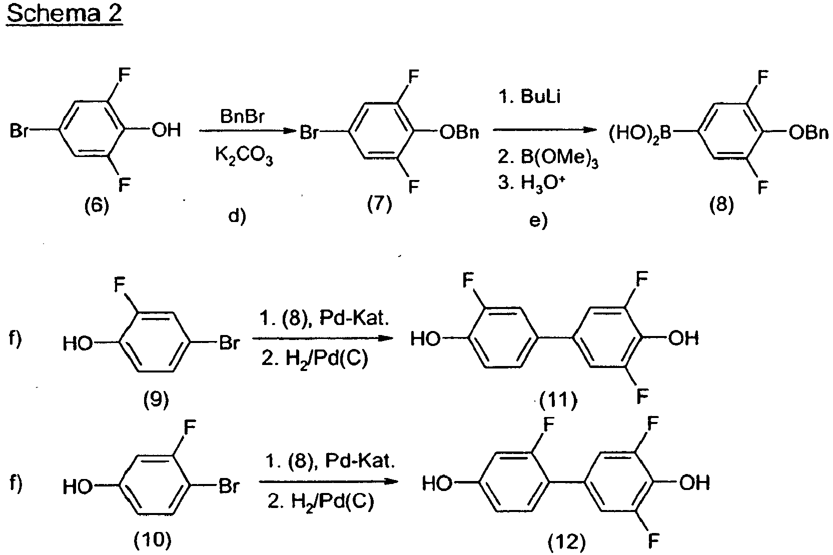

Darin bedeuten Bn = Benzyl; BuLi = n-Butyllithium; Pd-Kat = Pd(II)-Katalysator, beispielsweise Bis(triphenylphosphino) palladium(II)-chlorid.

In Schritt e) kann eine Boronsäure oder ein offenkettiger oder cyclischer Boronsäureester verwendet werden. Das Halogenid (4) in Schritt f) ist zum Beispiel 4-Brom-2- oder 3-fluorphenol, anstelle des Bromids kann aber auch ein Iodid oder eine andere geeignete Abgangsgruppe mit vergleichbarer Reaktivität (wie zum Beispiel Triflat) verwendet werden. Der in Schritt f) eingesetzte Übergangsmetallkatalysator ist bevorzugt ein Palladiumkomplex der Oxidationsstufen 0, II oder IV. Die Reaktion erfolgt bevorzugt in homogener Phase mit einem löslichen Katalysator. Ein bevorzugter Komplex ist zum Beispiel Bis(triphenylphosphin)palladium(II)-chlorid.

Alternativ zu dem vor- und nachstehend beschriebenen Verfahren können die reaktiven Gruppen der Reaktionspartner (8) und (9) bzw. (8) und (10) (Boronsäure-derivat und Halogenid) auch ausgetauscht werden, oder es wird in Schritt d) 4-Br(oder lod)-2 (oder 3)-fluorphenol anstelle von Verbindung (6), und in Schritt f) 4-Br(oder lod)-2,6-difluorphenol anstelle von Verbindung (9) bzw. (10) verwendet.

Die Herstellung von Verbindungen der Formel I aus Verbindungen der Formel Ia erfolgt vorzugsweise durch ein Verfahren, wie in Schema 3 beispielhaft dargestellt, enthaltend entweder Schritt g) oder h):
g) Reaktion der phenolischen OH-Gruppen mit Chlorpropionsäurechlorid und anschließender HCl-Abspaltung,
   oder
h) Veretherung oder Veresterung der phenolischen OH-Gruppen mit einer Säure, einem Säurederivat oder einer halogenierten Verbindung enthaltend eine Gruppe P.

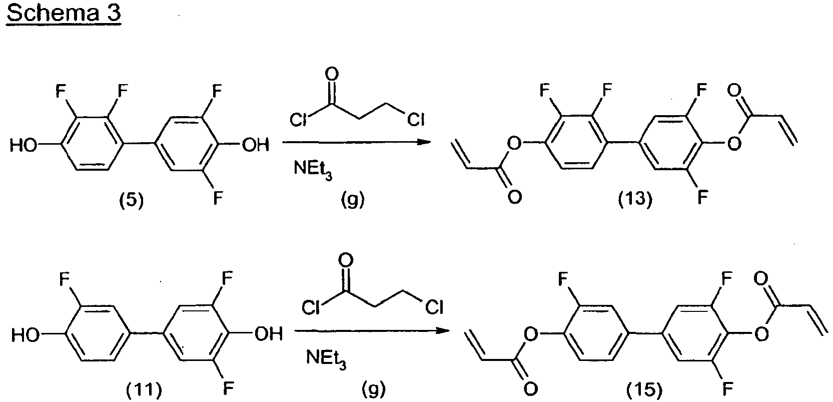

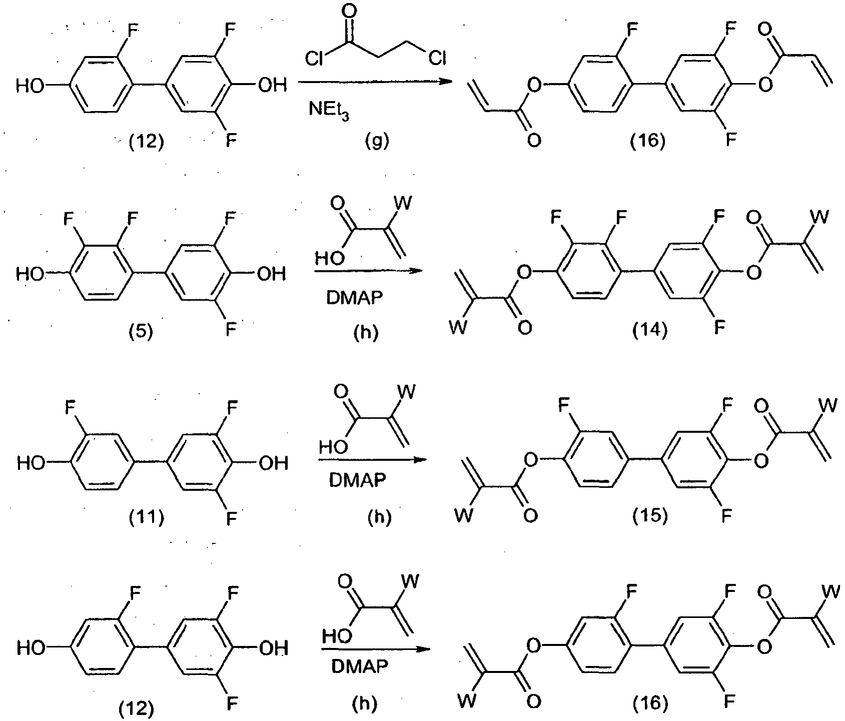

Darin bedeutet W = H, CH₃, F oder Cl

Für Schritt h) geeignete Säuren, Säurederivate oder halogenierte Verbindungen enthaltend eine Gruppe P sind beispielsweise Acrylsäure, Methacrylsäure, Fluoracrylsäure oder Chloracrylsäure oder entsprechende Säurederivate, zum Beispiel das Säurechlorid.

Zur Herstellung von PS(A)-Anzeigen werden die polymerisierbaren Verbindungen im FK-Medium zwischen den Substraten der FK-Anzeige unter Anlegen einer Spannung durch in-situ-Polymerisation polymerisiert oder vernetzt (falls eine Verbindung zwei oder mehr polymerisierbare Gruppen enthält). Geeignete und bevorzugte Polymerisationsmethoden sind beispielsweise die thermische oder Photopolymerisation, vorzugsweise Photopolymerisation, insbesondere UV-Photopolymerisation. Dabei können gegebenfalls auch ein oder mehrere Initiatoren zugesetzt werden. Geeignete Bedingungen für die Polymerisation, sowie geeignete Arten und Mengen der Initiatoren, sind dem Fachmann bekannt und in der Literatur beschrieben. Für die radikalische Polymerisation eignen sich zum Beispiel die kommerziell erhältlichen Photoinitiatoren Irgacure651®, Irgacure184®, oder Darocure1173® (Ciba AG). Falls ein Initiator eingesetzt wird, beträgt dessen Anteil vorzugsweise 0,001 bis 5 Gew.-%, besonders bevorzugt 0,001 bis 1 Gew.-%. Die Polymerisation kann aber auch ohne Zusatz eines Initiators erfolgen. In einer weiteren bevorzugten Ausführungsform enthält das FK-Medium keinen Polymerisationsinitiator.

Die polymerisierbare Komponente A) oder das FK-Medium können auch einen oder mehrere Stabilisatoren enthalten, um eine unerwünschte spontane Polymerisation der RMs, beispielsweise während der Lagerung oder des Transports, zu verhindern. Geeignete Arten und Mengen der Stabilisatoren sind dem Fachmann bekannt und in der Literatur beschrieben. Besonders geeignet sind zum Beispiel die kommerziell erhältlichen Stabilisatoren der Serie Irganox ® (Ciba AG). Falls Stabilisatoren eingesetzt werden, beträgt deren Anteil, bezogen auf die Gesamtmenge der RMs beziehungsweise der polymerisierbaren Komponente A), vorzugsweise 10 - 5000 ppm, besonders bevorzugt 50 - 500 ppm.

Die erfindungsgemäßen polymerisierbaren Verbindungen eignen sich auch für die Polymerisation ohne Initiator, was erhebliche Vorteile mit sich bringt, wie beispielsweise geringere Materialkosten und insbesondere eine geringere Verunreinigung des FK-Mediums durch mögliche Restmengen des Initiators oder dessen Abbauprodukte.

Die erfindungsgemäßen FK-Medien zur Verwendung in PSA-Anzeigen enthalten vorzugsweise < 5 Gew.-%, besonders bevorzugt < 1 Gew.-%, ganz besonders bevorzugt < 0.5 Gew.-% an polymerisierbaren Verbindungen, insbesondere polymerisierbaren Verbindungen der oben genannten Formeln.

Besonders bevorzugt sind FK-Medien enthaltend eine, zwei oder drei polymerisierbare Verbindungen der Formel I.

Ferner bevorzugt sind FK-Medien, worin die polymerisierbare Komponente A) ausschließlich polymerisierbare Verbindungen der Formel I enthält.

Ferner bevorzugt sind FK-Medien, worin Komponente B) eine FK-Verbindung oder eine FK-Mischung ist, die eine nematische Flüssigkristallphase aufweist.

Ferner bevorzugt sind achirale polymerisierbare Verbindungen der Formel I, sowie FK-Medien enthaltend, vorzugsweise ausschließlich bestehend aus, achiralen Verbindungen.

Ferner bevorzugt sind FK-Medien, worin die polymerisierbare Komponente bzw. Komponente A) eine oder mehrere polymerisierbare Verbindungen mit einer polymerisierbaren Gruppe (monoreaktiv) und eine oder mehrere polymerisierbare Verbindungen mit zwei oder mehr, vorzugsweise zwei polymerisierbaren Gruppen (di- oder multireaktiv) enthält.

Ferner bevorzugt sind PS(A)-Anzeigen und FK-Medien, worin die polymerisierbare Komponente bzw. Komponente A) ausschließlich polymerisierbare Verbindungen mit zwei polymerisierbaren Gruppen (direaktiv) enthält.

Die polymerisierbaren Verbindungen der Formel I können einzeln polymerisiert werden, es können aber auch Mischungen polymerisiert werden, welche zwei oder mehr Verbindungen der Formel I enthaltend, oder Mischungen enthaltend eine oder mehrere Verbindungen der Formel I und eine oder mehrere weitere polymerisierbare Verbindungen (Comonomere), welche vorzugsweise mesogen oder flüssigkristallin sind. Bei Polymerisation solcher Mischungen entstehen Copolymere. Die vor- und nachstehend genannten polymerisierbaren Mischungen sind ein weiterer Gegenstand der Erfindung. Die polymerisierbaren Verbindungen und Comonomere sind mesogen oder nicht-mesogen, vorzugsweise mesogen oder flüssigkristallin.

Geeignete und bevorzugte mesogene Comonomere, besonders für die Verwendung in PS(A)-Anzeigen, sind beispielsweise ausgewählt aus den folgenden Formeln: 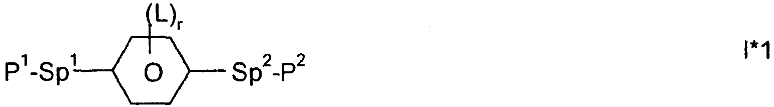 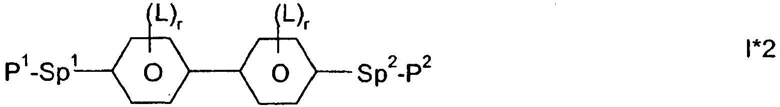 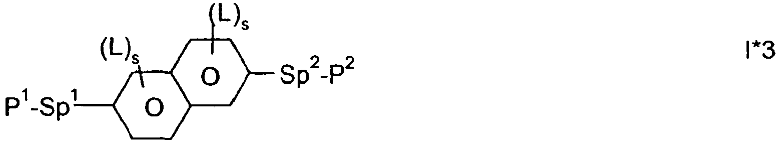 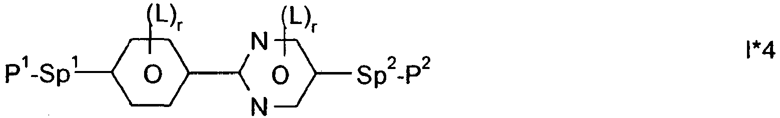 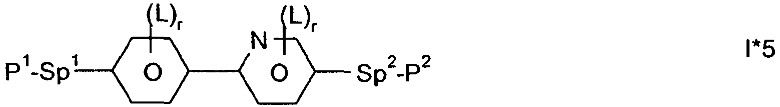 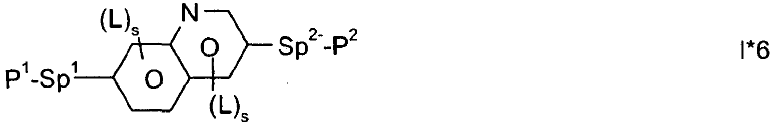 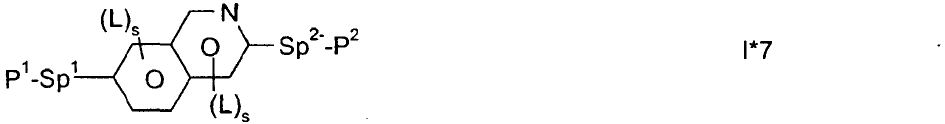 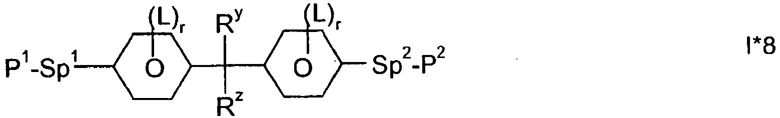 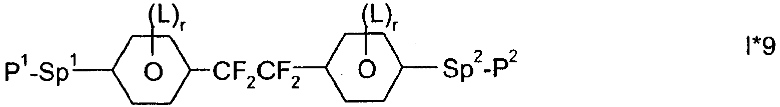 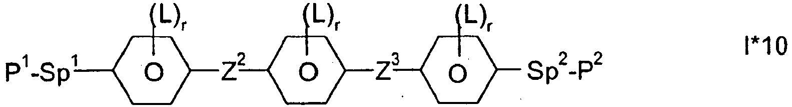 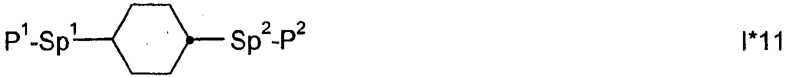 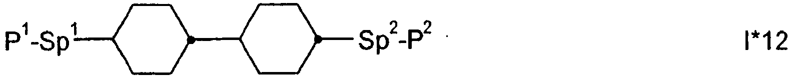 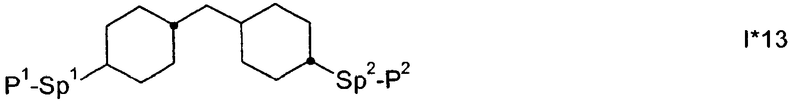 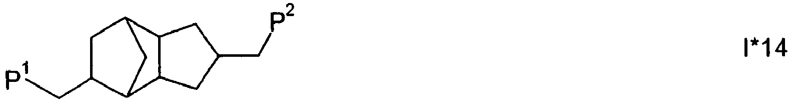 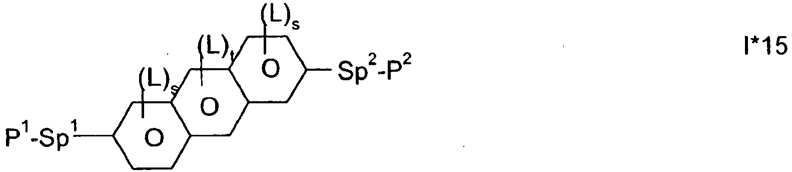 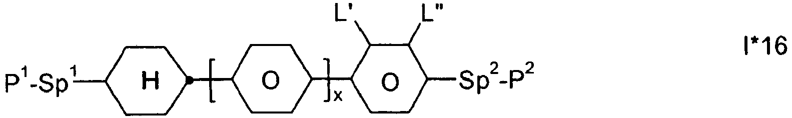 worin
- P¹ und P²: eine der für P angegebenen Bedeutungen besitzen und vorzugsweise Acrylat oder Methacrylat bedeuten,
- Sp¹ und Sp²: eine der für Sp angegebenen Bedeutungen besitzen oder eine Einfachbindung bedeuten,
- Z² und Z³: jeweils unabhängig voneinander -COO- oder -OCO bedeuten,
- L: P-Sp-, F, Cl, Br, I, -CN, -NO₂, -NCO, -NCS, -OCN, -SCN, - C(=O)N(R^{x})₂, -C(=O)Y¹, -C(=O)R^{x}, -N(R^{x})₂, optional substituiertes Silyl, optional substituiertes Aryl mit 6 bis 20 C Atomen, oder geradkettiges oder verzweigtes Alkyl, Alkoxy, Alkylcarbonyl, Alkoxycarbonyl, Alkylcarbonlyoxy oder Alkoxycarbonyloxy mit 1 bis 25 C-Atomen, worin auch ein oder mehrere H-Atome durch F, Cl oder P-Sp- ersetzt sein können, bedeutet,
- L' und L": jeweils unabhängig voneinander H, F oder Cl bedeuten,
- r: 0, 1, 2, 3 oder 4 bedeutet,
- s 0, 1, 2: oder 3 bedeutet,
- t: 0, 1 oder 2 bedeutet,
- x: 0 oder 1, und
- R^{y} und R^{z}: jeweils unabhängig voneinander H oder CH₃ bedeuten.

Eine weitere Verwendung betrifft Polymere erhältlich durch Polymerisation einer oder mehrerer Verbindungen der Formel I oder eines erfindungsgemäßen polymerisierbaren FK-Mediums, insbesondere Polymerfilme oder -folien erhältlich durch Polymerisation einer Schicht, enthaltend eine oder mehrere Verbindungen der Formel I oder ein erfindungsgemäßes FK-Medium, im einheitlich ausgerichteten Zustand in der FK-Phase.

Auch für diese Verwendung können die Verbindungen der Formel I einzeln oder in Mischung polymerisiert werden, welche zwei oder mehr Verbindungen der Formel I enthalten, oder eine oder mehrere Verbindungen der Formel I und eine oder mehrere weitere Comonomere enthalten, vorzugsweise mesogene oder flüssigkristalline Comonomere (RMs). Die Verbindungen der Formel I und/oder die Comonomere oder RMs können monoreaktiv, di- oder multireaktiv sein.

Die Polymere und Polymerfolien eignen sich besonders zur Verwendung als optische Filme wie Polarisatoren, Kompensatoren, Strahlenteiler oder Reflektivpolarisatoren, ferner als Orientierungsschichten, Farbfilter, holographische Elemente oder Heissprägefolien, zur Herstellung von Effektpigmenten mit winkelabhängiger Selektivreflexion, für dekorative Elemente, oder in Sicherheitselementen oder Sicherheitsmarkierungen für fälschungssichere Dokumente, Ausweise oder Wertpapiere.

Als Comonomere geeignete RMs sind dem Fachmann bekannt oder können nach an sich bekannten Methoden hergestellt werden, die in Standardwerken der organischen Chemie beschrieben sind, wie beispielsweise Houben-Weyl, Methoden der organischen Chemie, Thieme-Verlag, Stuttgart. Typische Beispiele für geeignete RMs sind offenbart in WO 93/22397, EP 0 261 712, DE 195 04 224, WO 95/22586, WO 97/00600, US 5,518,652, US 5,750,051, US 5,770,107 and US 6,514,578. Weitere Beispiele für besonders geeignete und bevorzugte monoreaktive (MR), direaktive (DR) und chirale (CR) RMs sind in der folgenden Liste gezeigt: 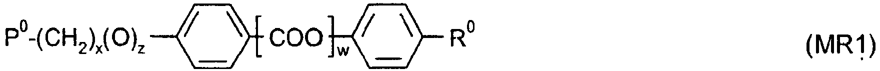 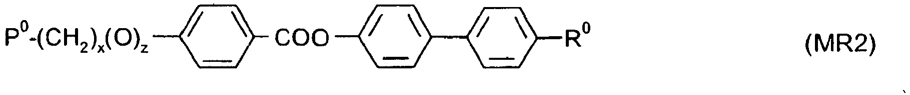 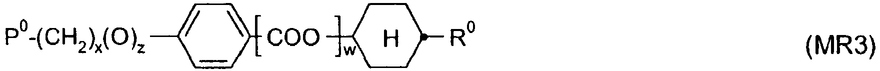 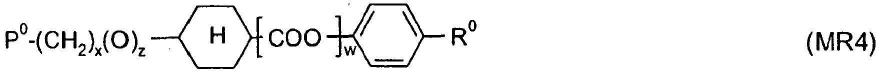 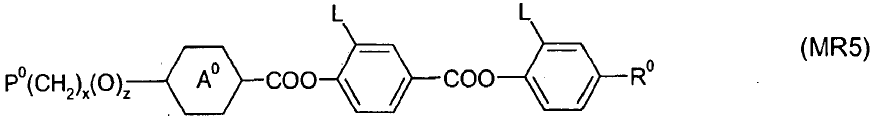 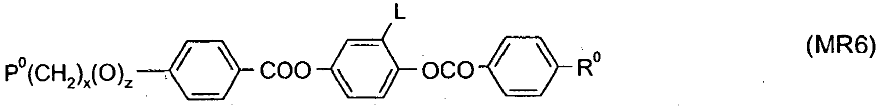 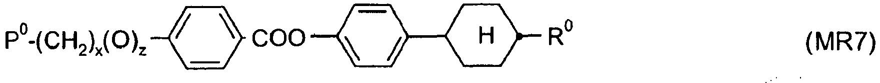 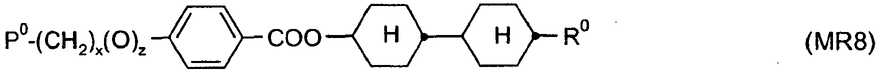 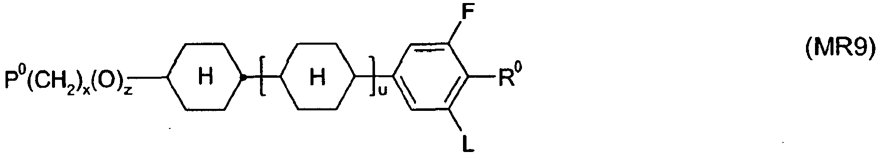 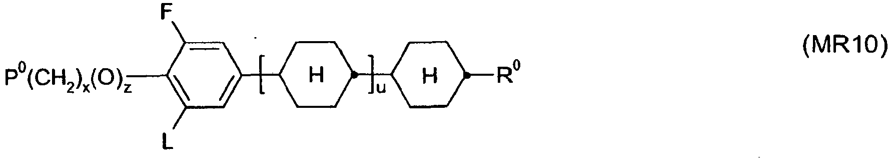 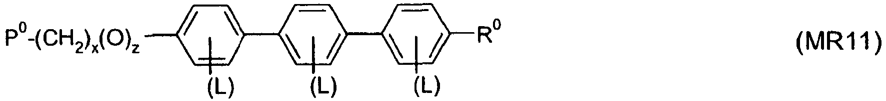 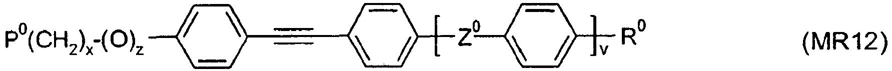 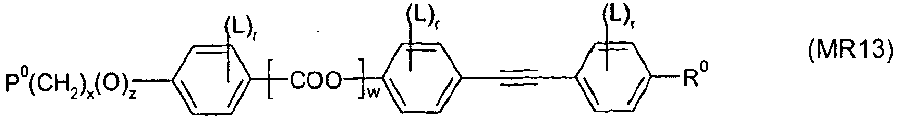 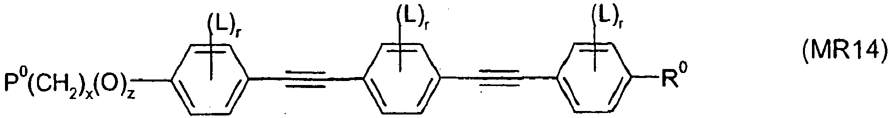 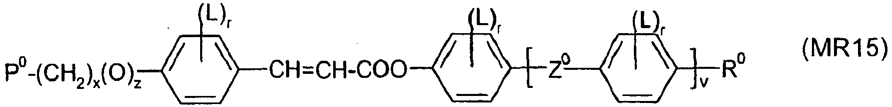 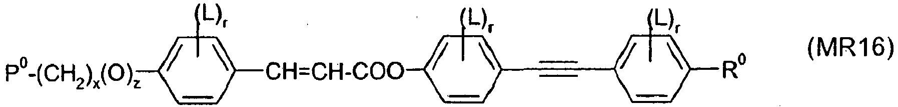 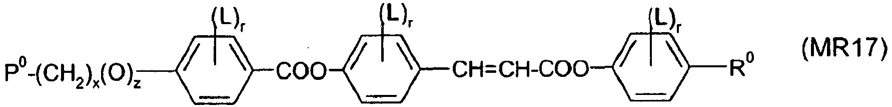 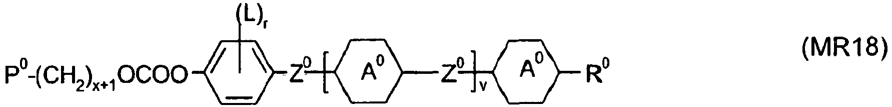 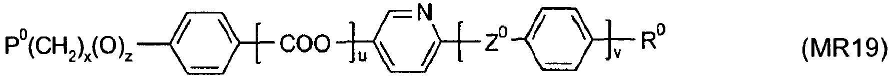 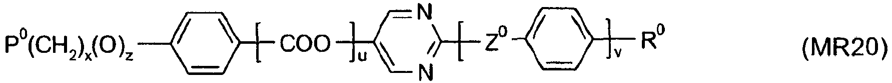 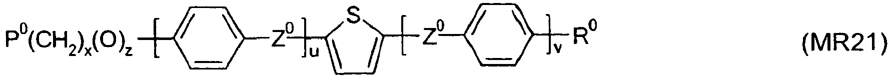 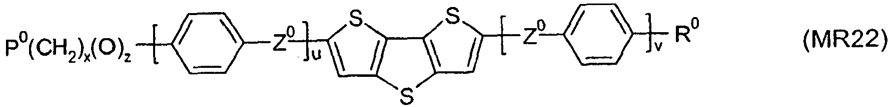 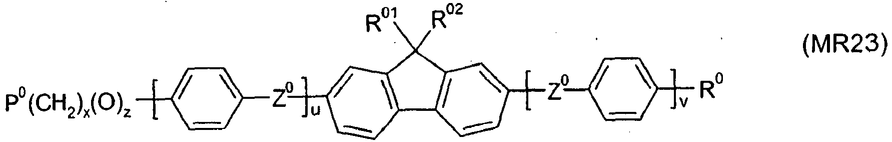 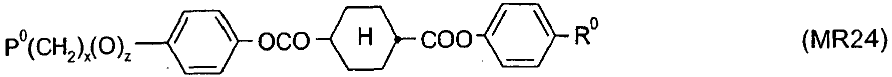 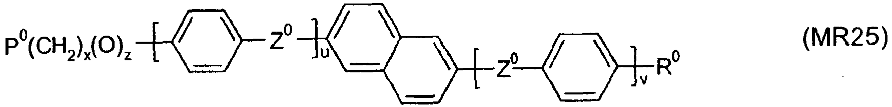 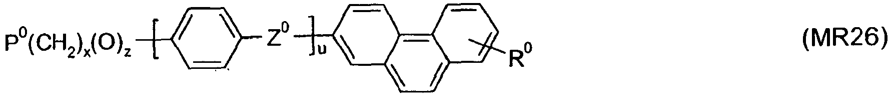 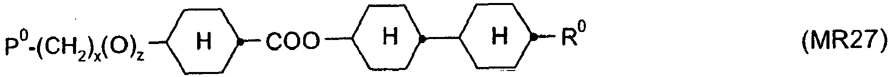 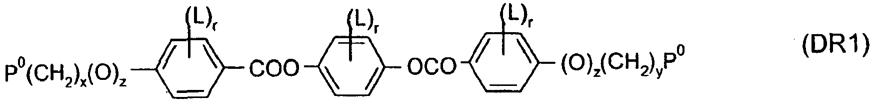 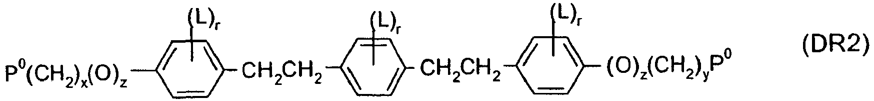 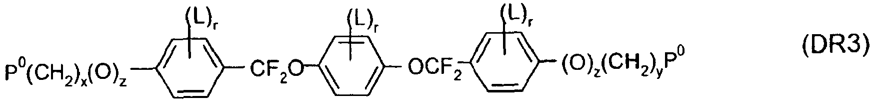 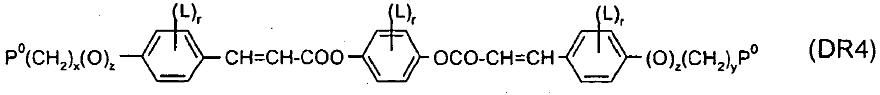 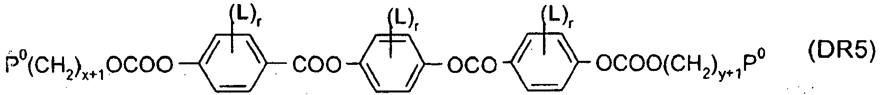 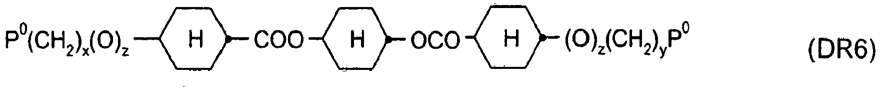 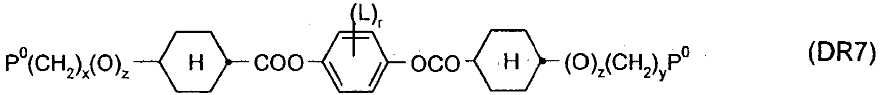 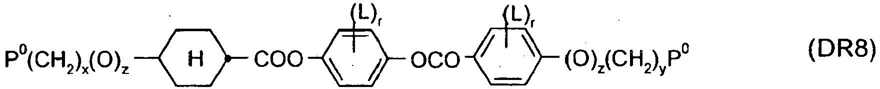 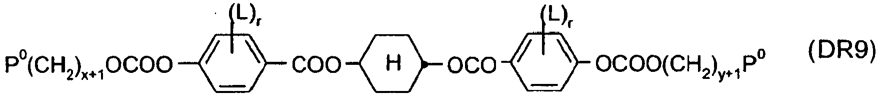 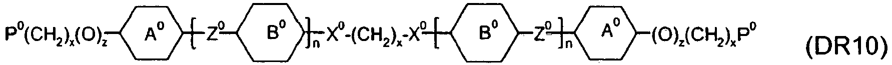 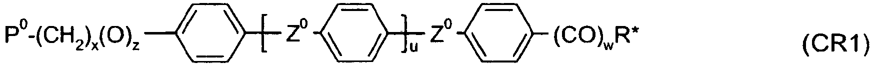 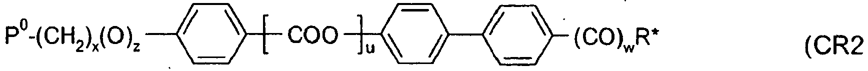 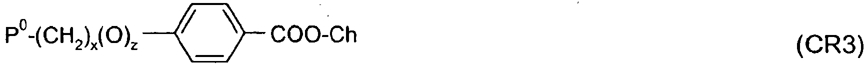 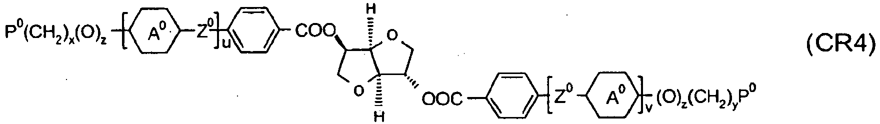 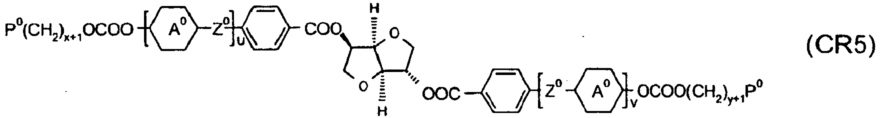 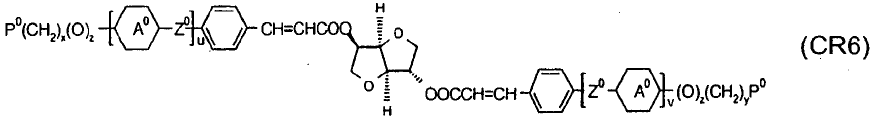 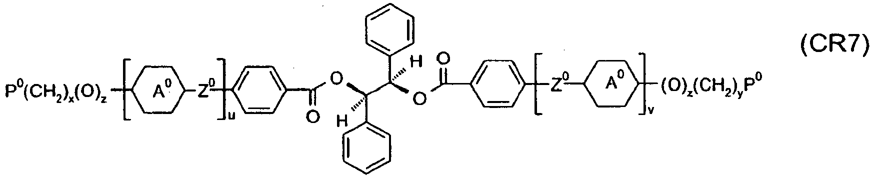 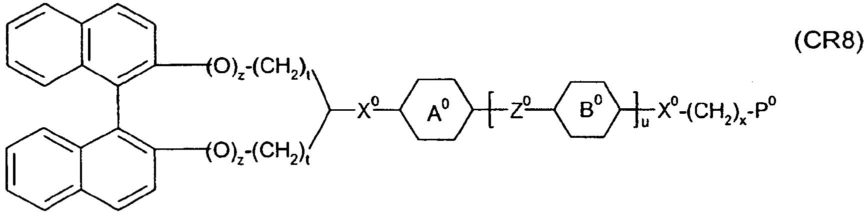 worin die einzelnen Reste, jeweils unabhängig voneinander und bei mehrfachem Auftreten gleich oder verschieden, folgende Bedeutung haben
- P⁰: eine polymerisierbare Gruppe, vorzugsweise ausgewählt aus Acryl, Methacryl, Oxetan, Epoxy, Vinyloxy und Styryl,
- A⁰ und B⁰: 1,4-Phenylen welches optional durch 1, 2, 3 oder 4 Reste L substituiert ist, oder trans-1,4-Cyclohexylen,
- Z⁰: -COO-, -OCO-, -CH₂CH₂-, -C≡C-, -CH=CH-, -CH=CH-COO-, - OCO-CH=CH- oder eine Einfachbindung,
- R⁰: Alkyl, Alkoxy, Thioalkyl, Alkylcarbonyl, Alkoxycarbonyl, Alkylcarbonyloxy oder Alkoxycarbonyloxy mit 1 oder mehr, vorzugsweise 1 bis 15 C-Atomen, welches optional mono- oder polyfluoriert ist, oder Y⁰ oder P⁰-(CH₂)_{y}-(O)_{z}-,
- Y⁰: F, Cl, CN, NO₂, OCH₃, OCN, SCN, NCO, NCS, SF₅, optional fluoriertes Alkylcarbonyl, Alkoxycarbonyl, Alkylcarbonyloxy oder Alkoxycarbonyloxy mit 1 bis 4 C-Atomen, oder mono-, oligo- oder polyfluoriertes Alkyl oder Alkoxy mit 1 bis 4 C-Atomen,
- R^{01,02}: H, R⁰ oder Y⁰,
- R*: chirales Alkyl oder Alkoxy mit 4 oder mehr, vorzugsweise 4 bis 12 C-Atomen, wie zum Beispiel 2-Methylbutyl, 2-Methyloctyl, 2-Methylbutoxy oder 2-Methyloctoxy,
- Ch: eine chirale Gruppe ausgewählt aus Cholesteryl, Östradiol und Terpenoidresten wie zum Beispiel Menthyl oder Citronellyl,
- L: H, F, Cl, CN oder optional fluoriertes oder chloriertes Alkyl, Alkoxy, Alkylcarbonyl, Alkoxycarbonyl, Alkylcarbonyloxy oder Alkoxycarbonyloxy mit 1 bis 4 C-Atomen,
- r: 0, 1, 2, 3 oder 4,
- t: 0, 1, 2 oder 3,
- u und v: 0, 1 oder 2,
- w: 0 oder 1,
- x und y: 0 oder eine ganze Zahl von 1 bis 12,
- z: 0 oder 1, wobei z 0 ist falls im angrenzenden Rest x oder y 0 ist,
und worin die Benzol- und Naphthylringe auch zusätzlich durch ein oder mehrere, gleiche oder verschiedene Reste L substitutiert sein können.

Die erfindungsgemäßen polymerisierbaren Mischungen können auch eine oder mehrere chirale Verbindungen oder chirale Dotierstoffe enthalten und chiral flüssigkristalline Phasen aufweisen, wie zum Beispiel eine cholesterische Phase. Die chiralen Verbindungen können polymerisierbar und/oder mesogen oder flüssigkristallin sein. Geeignete chirale RMs sind beispielsweise solche der oben gezeigten Formeln CR1-CR8. Geeignete chirale Dotierstoffe sind beispielsweise ausgewählt aus den kommerziell erhältlichen Verbindungen Cholesterylnonanoat (CN), CB 15, R/S-811, R/S-1011, R/S-2011, R/S-3011 oder R/S-4011 (Merck KGaA, Darmstadt). Besonders geeignet sind Dotierstoffe mit hohem Verdrillungsvermögen, zum Beispiel chirale Zuckerderivate, insbesondere Derivate von Dianhydrohexitolen wie Isosorbit, Isomannit oder Isoidit, besonders bevorzugt Isosorbit-Derivative wie zum Beispiel in WO 98/00428 offenbart. Ferner bevorzugt sind Hydrobenzoinderivate wie zum Beispiel in GB 2,328,207 beschrieben, chirale Binaphthyle wie zum Beispiel in WO 02/94805 beschrieben, chirale Binaphthole wie zum Beispiel in WO 02/34739 beschrieben, chirale TADDOLe wie zum Beispiel in WO 02/06265 beschrieben, und chirale Verbindungen mit einer fluorierten Brückengruppe und einer terminalen oder zentralen chiralen Gruppe wie zum Beispiel in WO 02/06196 oder WO 02/06195 beschrieben.

Geeignete Verfahren und Hilfsmittel zur Herstellung von flüssigkristallinen Polymeren, Polymerfilmen oder Beschichtungen sind dem Fachmann bekannt und in der Literatur beschrieben, beispielsweise in D. J. Broer, G. Challa, G. N. Mol, Macromol. Chem, 1991, 192, 59. Die Herstellung der erfindungsgemäßen Polymere erfolgt vorzugsweise in Form von dünnen Filmen. Hierzu wird das polymerisierbare flüssigkristalline Material zunächst hilfsweise auf ein Substrat gebracht, zum Beispiel durch bekannte Beschichtungs- oder Bedruckungsverfahren, von dem der Polymerfilm dann später gegebenfalls wieder abgelöst werden kann. Dann wird im Material eine einheitliche molekulare Ausrichtung in der FK-Phase, beispielsweise in der nematischen, smektischen oder cholesterischen Phase, induziert und bei einer geeigneten Temperatur durch Polymerisation oder Vernetzung in situ fixiert.

Geeignete Substrate sind zum Beispiel Filme oder Folien aus Kunststoff, Papier, Karton, Leder, Zellstoff, Textilien, Glas, Keramik oder Metall. Geeignete Kunststoffe sind zum Beispiel Polyester wie Polyethylenterephthalat (PET) oder Polyethylennaphthalat (PEN), Polyvinylalkohol (PVA), Polycarbonat (PC), Di- oder Triacetylcellulose (DAC, TAC), insbesondere PET, TAC, Polyimide oder Polyamide, die in FK-Anzeigen eingesetzt werden. Das polymerisierbare Material kann auch mit einem zweiten Substrat bedeckt werden.

Geeignete Beschichtungsverfahren sind beispielsweise Walzen, Sprühen, Rakeln, Schleudern (Spin-Coating) oder Tauchen, geeignete Druckverfahren sind zum Beispiel Flexodruck, Offsetdruck, Tiefdruck, Hochdruck oder Tintenstrahldruck.

Das polymerisierbare Material kann auch als Lösung oder Suspension in einem organischen Lösungsmittel aufgebracht und das Lösungsmittel anschließend entfernt werden, zum Beispiel durch Erwärmen und/oder Druckverminderung oder Anlegen eines Vakuums. Geeignete Lösungsmittel sind zum Beispiel Toluol, Xylol, MEK, Aceton, Cyclohexanon, Isopropylalkohol, Methyl-, Ethyl-, Propyl- oder Butylacetat, PGMEA (Propylenglykolmonomethyletheracetat) oder deren Gemische.

Eine einheitliche Ausrichtung der flüssigkristallinen Moleküle kann beispielsweise erzielt werden, indem man auf die zu beschichtende Oberfläche des Substrats eine Orientierungsschicht aufbringt, wie beispielsweise eine Polyimidschicht oder eine Schicht aus photoorientierten und vernetzten FK-Molekülen. Geeignete Materialien und Verfahren zum Aufbringen von Orientierungsschichten sind in der Literatur beschrieben, zum Beispiel in US 5,602,661, US 5,389,698 oder US 6,717,644. Zusätzlich oder alternativ dazu kann die zu beschichtende Oberfläche des Substrats oder die Orientierungsschicht vor Aufbringen des polymerisierbaren Materials in einer Vorzugsrichtung gerieben werden. Oft genügt auch eine leicht Scherung des polymerisierbaren Materials zwischen zwei Substraten zur einheitlichen Ausrichtung. Weitere Verfahren zur Induzierung oder Verbesserung der einheitlichen Ausrichtung sind Tempern des polymerisierbaren Materials, oder Anlegen eines elektrischen oder magnetischen Feldes an das polymerisierbare Material. In einer weiteren bevorzugten Ausführungsform enthält die polymerisierbare Mischung eine oder mehrere Additive, zum Beispiel oberflächenaktive Substanzen, die eine einheitliche Ausrichtung der flüssigkristallinen Moleküle auf dem Substrat induzieren oder verbessern. Geeignete Substanzen sind dem Fachmann bekannt und in der Literatur beschrieben, zum Beispiel in J. Cognard, Mol.Cryst.Liq.Cryst. 78, Supplement 1, 1-77 (1981) oder T. Uchida und H. Seki in "Liquid Crystals - Applications and Uses Vol. 3", B. Bahadur, World Scientific Publishing, Singapur 1992, Seite 1-63. Besonders bevorzugt sind nichtionische Verbindungen, zum Beispiel nichtionische Fluorkohlenwasserstoffe wie das kommerziell erhältliche Fluorad FC-171® (3M), Zonyl FSN ® (DuPont), oder Verbindungen wie in GB 2 383 040 A oder EP 1 256 617 A1 beschrieben.

Die Polymerisation oder Vernetzung des polymerisierbaren Materials erfolgt vorzugsweise in situ, nach Aufbringen und Orientieren und gegebenfalls Tempern in der FK-Phase, beispielsweise durch thermische Polymerisation oder durch Behandlung mit aktinischer Strahlung wie zum Beispiel UV-Licht, IR-Licht oder sichtbarem Licht, Röntgenstrahlen, Gammastrahlen oder hochenergetischen Partikeln wie Ionen oder Elektronen. Besonders bevorzugt ist Photopolymerisation, insbesondere Polymerisation mit UV-Licht. Als Strahlungsquelle kann zum Beispiel eine einzelne UV-Lampe oder eine Reihe von UV-Lampen verwendet werden. Andere mögliche Strahlungsquellen sind zum Beispiel Laser, wie UV-Laser, IR-Laser oder Laser im sichtbaren Wellenlägenbereich.

Die Polymerisation wird vorzugsweise in Gegenwart eines Initiators durchgeführt, der die aktinische Strahlung absorbiert. Bei UV-Photopolymerisation wird zum Beispiel ein Photoinitiator verwendet, der bei UV-Bestrahlung zerfällt und dabei freie Radikale oder Ionen freiisetzt, welche eine Polymerisationsreaktion auslösen. UV-Photoinitiatoren sind besonders bevorzugt. Solche Photoinitiatoren sind dem Fachmann bekannt und kommerziell erhältlich, wie zum Beispiel Irgacure® 907, Irgacure® 651, Irgacure® 184, Irgacure907®, Irgacure369®, Darocure® 1173 oder Darocure® 4205 (Ciba AG) oder UVI 6974 (Union Carbide). Besonders bevorzugt zur Herstellung erfindungsgemäßer Polymere ist die thermisch oder photochemisch induzierte radikalische Polymerisation. Vorzugsweise enthält das polymerisierbare Material einen oder mehrere Initiatoren. Das polymerisierbare Material enthält vorzugsweise von 0.01 bis 10 Gew.-%, besonders bevorzugt von 0.05 bis 5 Gew.-% an Initiatoren.

In einer weiteren bevorzugten Ausführungsform enthält das polymerisierbare Material einen oder mehrere Stabilisatoren, um eine unerwünschte spontane Polymerisation zu vermeiden, wie beispielsweise aus kommerziell erhältlichen Serie Irganox® (Ciba Geigy AG, Basel, Switzerland).

In einer weiteren bevorzugten Ausführungsform enthält das polymerisierbare Material ein oder mehrere Kettenübertragungsreagenzien, zum Beispiel Thiolverbindungen wie Dodekanthiol oder Trimethylpropan-tri(3-mercaptopropionat), insbesondere flüssigkristalline Thiolverbindungen. Besonders bevorzugt sind mesogene oder flüssigkristalline Thiolverbindungen wie zum Beispiel in WO 96/12209 A1, WO 96/25470 A2 oder US 6,420,001 offenbart. Durch Zugabe solcher Reagenzien kann zum Beispiel die freie Kettenlänge der Polymere, oder die Kettenlänge zwischen zwei Vernetzungspunkten, reduziert werden.

In einer weiteren bevorzugten Ausführungsform enthält das polymerisierbare Material ein oder mehrere mono-, di- oder multireaktive polymerisierbare nicht-mesogene Verbindungen, wie zum Beispiel Alkyl(meth)acrylate oder Alkyldi(meth)acrylate, vorzugsweise mit Alkylresten enthaltend 1 bis 20 C-Atome. Typische Beispiele für geeignete multireaktive Verbindungen sind Trimethylpropantrimethacrylat oder Pentaerythritoltetraacrylat. Das polymerisierbare Material enthält vorzugsweise von 1 bis 50 Gew.-%, besonders bevorzugt von 2 bis 20 Gew.-% solcher nicht-mesogener polymerisierbarer Verbindungen.

In einer weiteren bevorzugten Ausführungsform enthält das polymerisierbare Material ein oder mehrere polymere oder polymerisierbare Bindemittel oder Dispergierhilfsmittel, wie zum Beispiel in WO 96/02597 beschrieben.

In einer weiteren bevorzugten Ausführungsform enthält das polymerisierbare Material eine oder mehrere Komponenten oder Hilfsmittel ausgewählt aus der Gruppe enthaltend Katalysatoren, Sensibilisatoren, Stabilisatoren, Kettenübertragungsreagentien, Inhibitoren, Comonomere, oberflächenaktive Substanzen, Weichmacher, Benetzungsmittel, Dispergierhilfsmittel, Verlaufsmittel, Fließmittel, Viskositätsminderer, Hydrophobisierungsmittel, Adhäsionsmittel, Entschäumungsmittel, Entlüftungs- oder Entgasungsmittel, Verdünner, Reaktivverdünner, Farbstoffe, Farbmittel, Pigmente und Nanopartikel.

Die erfindungsgemäßen Polymerfilme und -folien haben vorzugsweise eine Dicke von 0.3 bis 10 Mikrometer, besonders bevorzugt von 0.5 bis 5 Mikrometer. Für die Verwendung als Orientierungsschicht sind Filme mit einer Dicke von 0.05 bis 1, insbesondere 0.1 bis 0.5 Mikrometer bevorzugt.

Die FK-Medien zur Verwendung in den erfindungsgemäßen FK-Anzeigen enthalten, neben den oben beschriebenen polymerisierbaren Verbindungen, eine FK-Mischung ("Host-Mischung") enthaltend eine oder mehr, vorzugsweise zwei oder mehr niedermolekulare (d.h. monomere bzw. unpolymerisierte) Verbindungen. Letztere sind stabil bzw. unreaktiv gegenüber einer Polymerisationsreaktion unter den zur Polymerisation der polymerisierbaren Verbindungen verwendeten Bedingungen. Prinzipiell eignet sich als Host-Mischung jede zur Verwendung in herkömmlichen VA- und OCB-Anzeigen geeignete FK-Mischung. Geeignete FK-Mischungen sind dem Fachmann bekannt und in der Literatur beschrieben, beispielsweise Mischungen in VA-Anzeigen in EP 1 378 557 A1, und Mischungen für OCB-Anzeigen in EP 1 306 418 A1 und DE 102 24 046 A1. Besonders bevorzugte Host-Mischungen und FK-Medien werden im Folgenden genannt:
a) FK-Medium, welches eine oder mehrere Verbindungen ausgewählt aus den folgenden Formeln enthält: 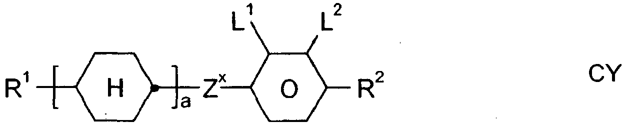 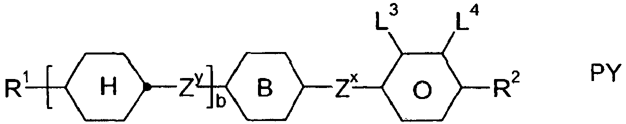 worin die einzelnen Reste folgende Bedeutung besitzen
   - a: 1 oder 2,
   - b: 0 oder 1, 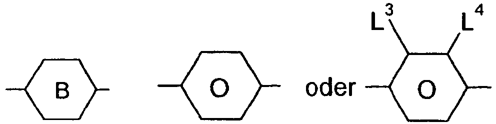
   - R¹ und R²: jeweils unabhängig voneinander Alkyl mit 1 bis 12 C-Atomen, wobei auch eine oder zwei nicht benachbarte CH₂-Gruppen durch -O-, -CH=CH-, -CO-, -OCO- oder - COO- so ersetzt sein können, dass O-Atome nicht direkt miteinander verknüpft sind, vorzugsweise Alkyl oder Alkoxy mit 1 bis 6 C-Atomen,
   - Z^{x} und Z^{y}: jeweils unabhängig voneinander -CH₂CH₂-, -CH=CH-, -CF₂O-, -OCF₂-, -CH₂O-, -OCH₂-, -COO-, -OCO-, -C₂F₄-, -CF=CF-, -CH=CHCH₂O-, oder eine Einfachbindung, vorzugsweise eine Einfachbindung,
   - L¹⁻⁴: jeweils unabhängig voneinander F, Cl, OCF₃, CF₃, CH₃, CH₂F, CHF₂.

Vorzugsweise bedeuten beide Reste L¹ und L² F, oder einer der Reste L¹ und L² F und der andere Cl, bzw. beide Reste L³ und L⁴F, oder einer der Reste L³ und L⁴F und der andere Cl.

Die Verbindungen der Formel CY sind vorzugsweise ausgewählt aus den folgenden Unterformeln 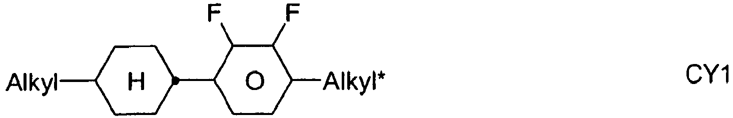 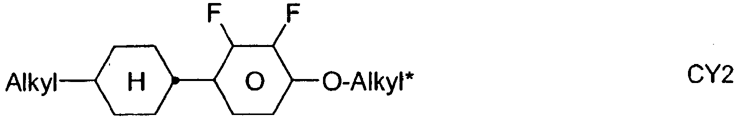 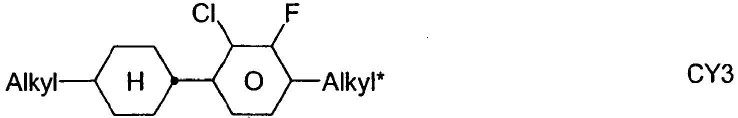 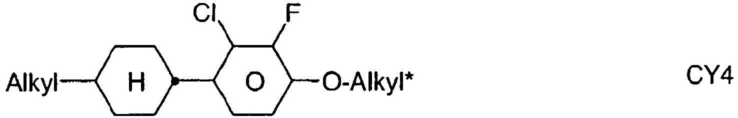 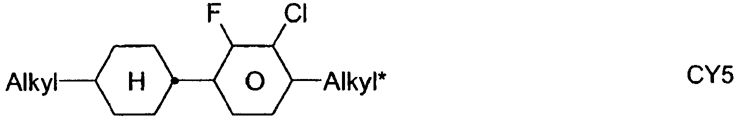 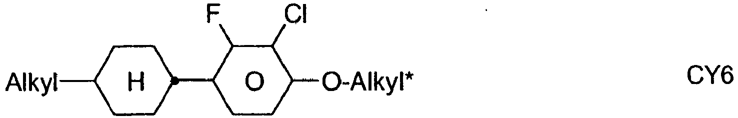 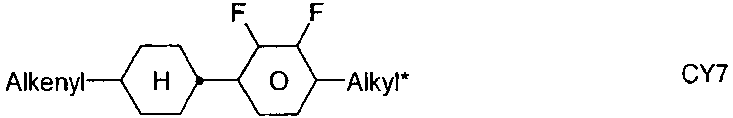 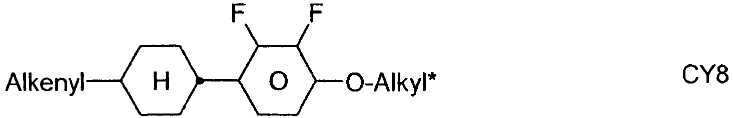 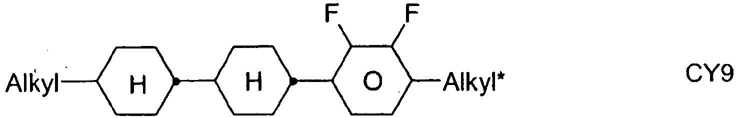 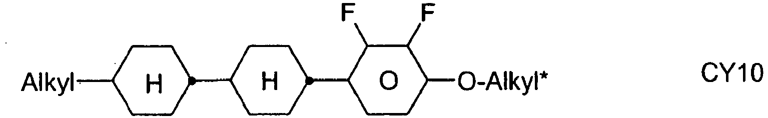 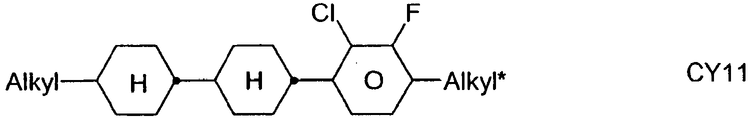 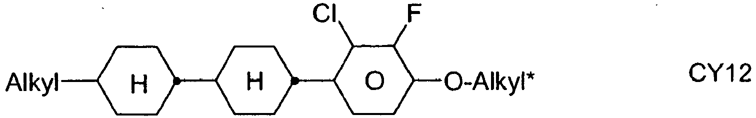 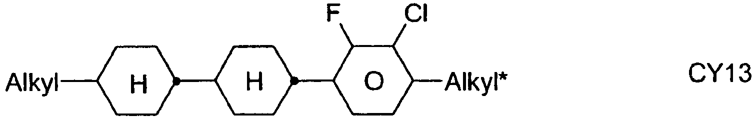 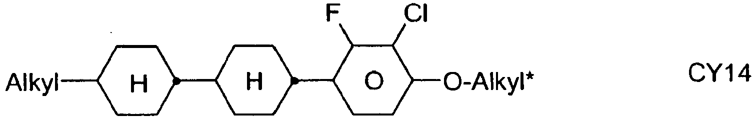 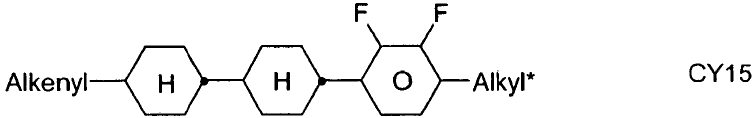 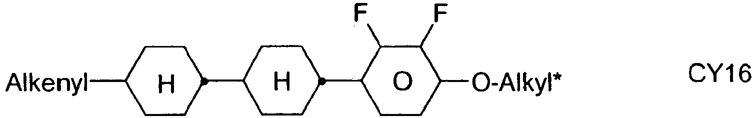 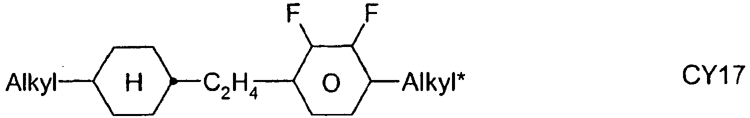 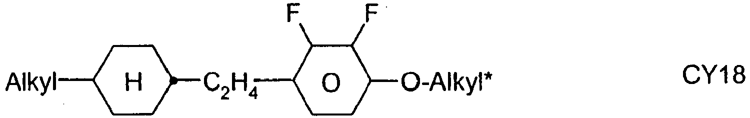 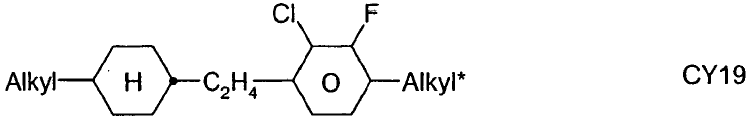 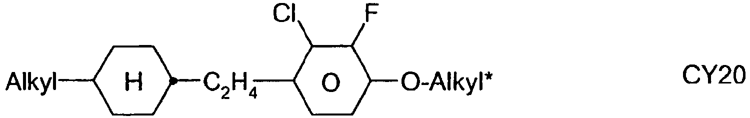 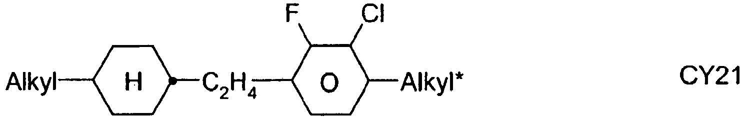 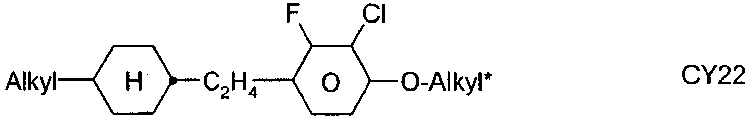 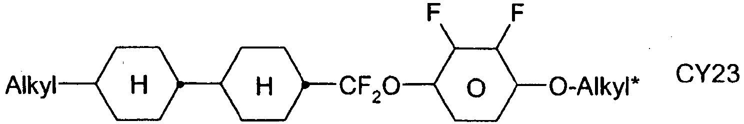 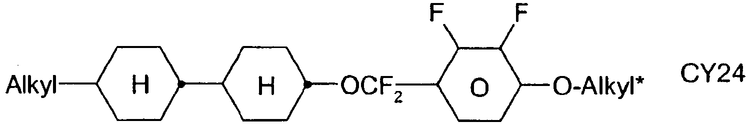 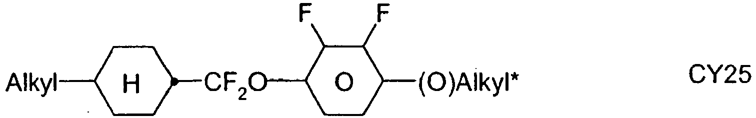 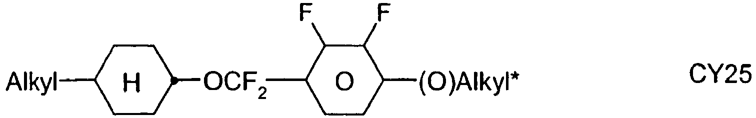 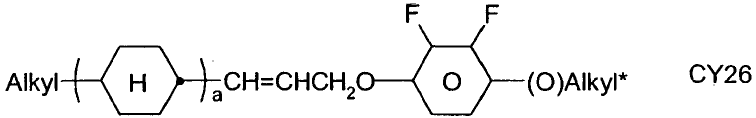 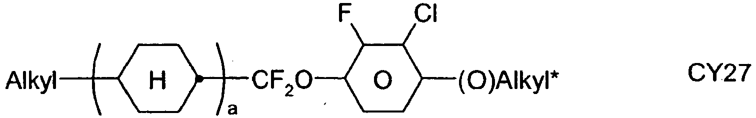 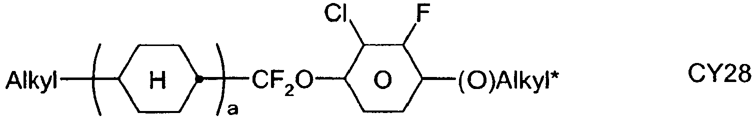 worin a 1 oder 2, Alkyl und Alkyl* jeweils unabhängig voneinander einen geradkettigen Alkylrest mit 1-6 C-Atomen, und Alkenyl einen geradkettigen Alkenylrest mit 2-6 C-Atomen bedeuten. Alkenyl bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.

Die Verbindungen der Formel PY sind vorzugsweise ausgewählt aus den folgenden Unterformeln: 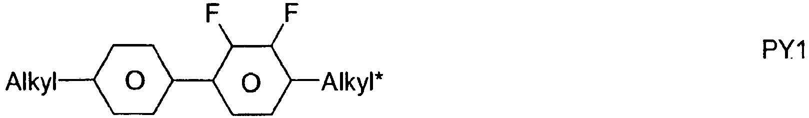 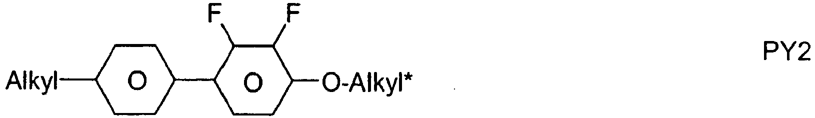 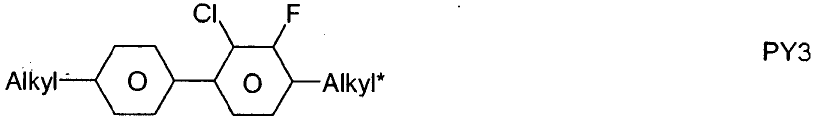 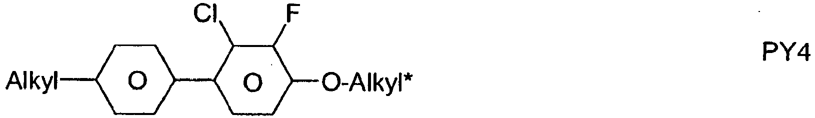 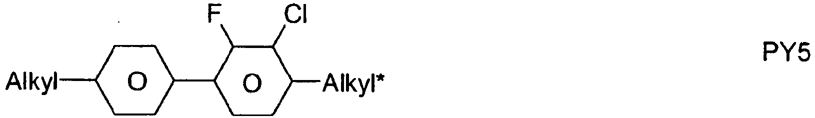 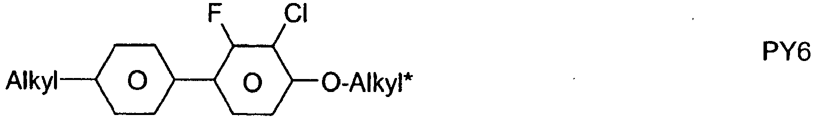 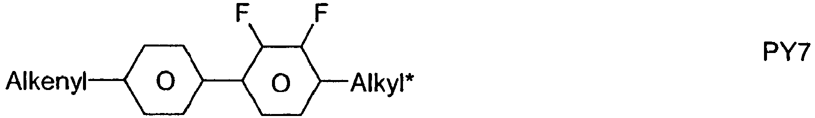 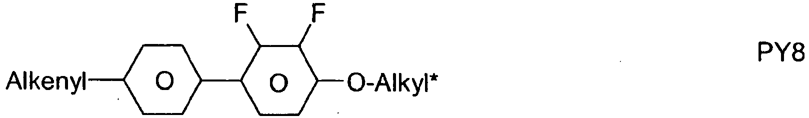 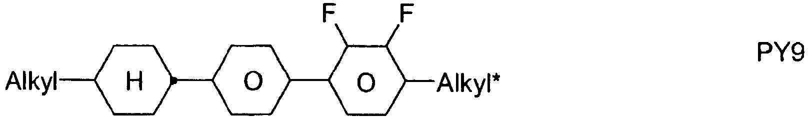 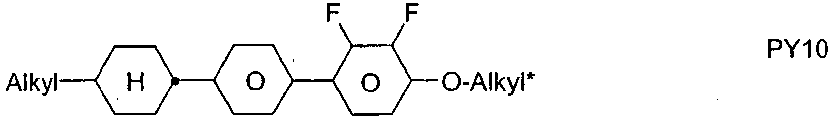 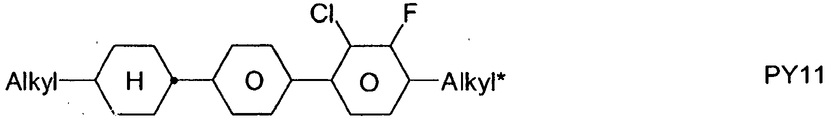 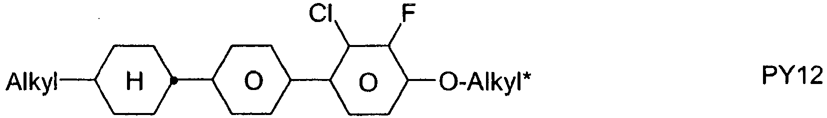 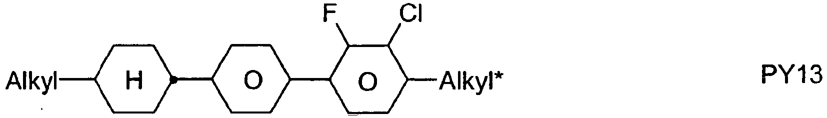 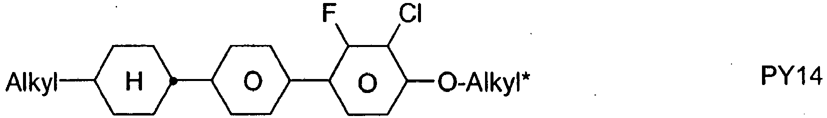 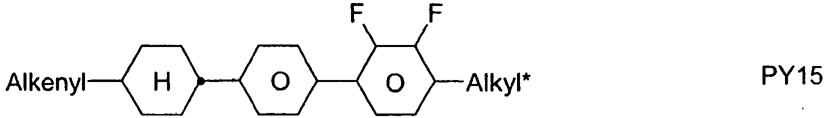 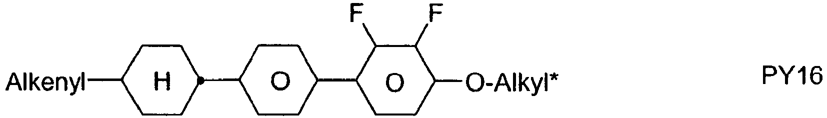 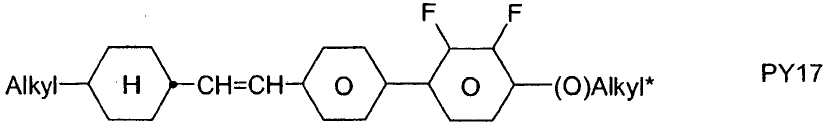 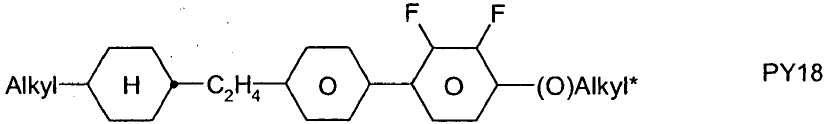 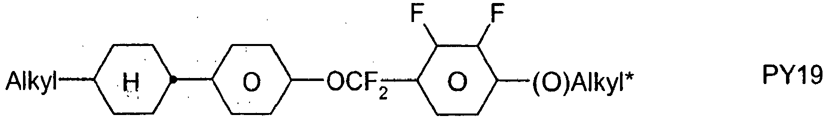 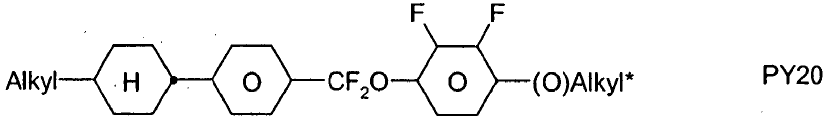 worin Alkyl und Alkyl* jeweils unabhängig voneinander einen geradkettigen Alkylrest mit 1-6 C-Atomen und Alkenyl einen geradkettigen Alkenylrest mit 2-6 C-Atomen bedeuten. Alkenyl bedeutet vorzugsweise CH₂=CH-, CH₂=CHCH₂CH₂-, CH₃-CH=CH-, CH₃₋CH₂-CH=CH-, CH₃-(CH₂)₂-CH=CH-, CH₃-(CH₂)₃-CH=CH- oder CH₃-CH=CH-(CH₂)₂-.
b) FK-Medium, welches zusätzlich eine oder mehrere Verbindungen der folgenden Formel enthält: 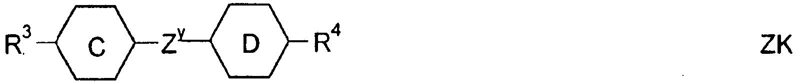 worin die einzelnen Reste folgende Bedeutung besitzen 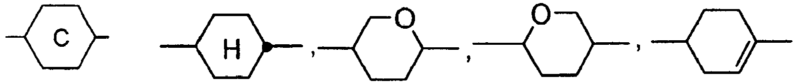 oder

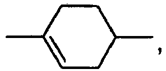

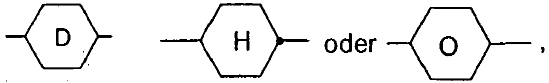

- R³ und R⁴: jeweils unabhängig voneinander Alkyl mit 1 bis 12 C-Atomen, worin auch eine oder zwei nicht benachbarte CH₂-Gruppen durch -O-, -CH=CH-, -CO-, -OCO- oder - COO- so ersetzt sein können, dass O-Atome nicht direkt miteinander verknüpft sind,
- Z^{y}: -CH₂CH₂-, -CH=CH-, -CF₂O-, -OCF₂-, -CH₂O-, -OCH₂-, -COO-, -OCO-, -C₂F₄-, -CF=CF-, -CH=CHCH₂O-, oder eine Einfachbindung, vorzugsweise eine Einfachbindung.

Die Verbindungen der Formel ZK sind vorzugsweise ausgewählt aus den folgenden Unterformeln: 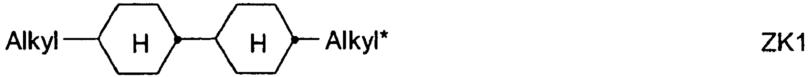 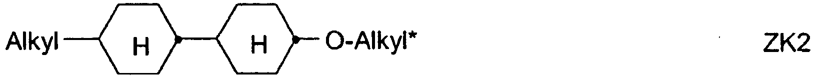 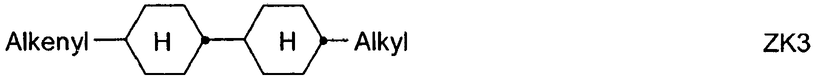 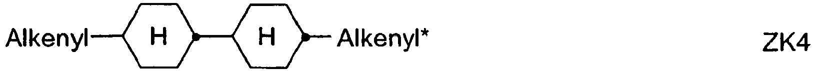 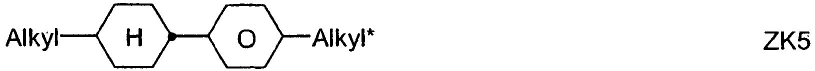 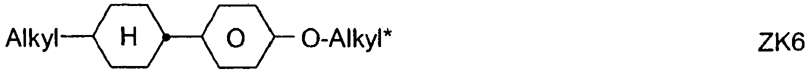 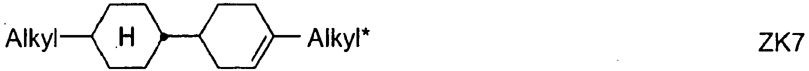 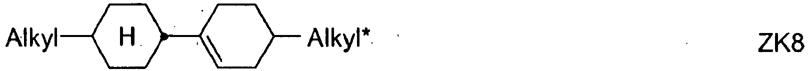 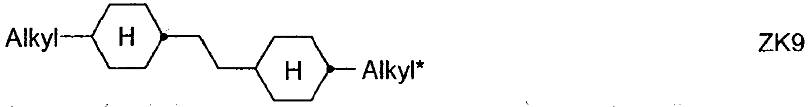 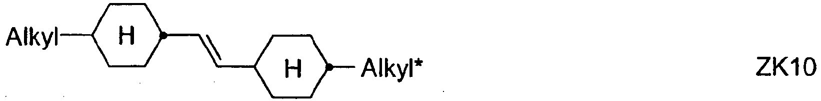 worin Alkyl und Alkyl* jeweils unabhängig voneinander einen geradkettigen Alkylrest mit 1-6 C-Atomen bedeuten, und Alkenyl einen geradkettigen Alkenylrest mit 2-6 C-Atomen bedeuten. Alkenyl bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
c) FK-Medium, welches zusätzlich eine oder mehrere Verbindungen der folgenden Formel enthält: 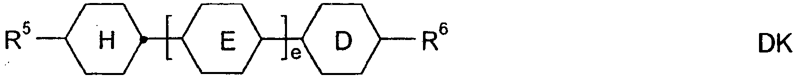 worin die einzelnen Reste bei jedem Auftreten gleich oder verschieden folgende Bedeutung haben:
   - R⁵ und R⁶: jeweils unabhängig voneinander eine der oben für R¹ angegebenen Bedeutungen, 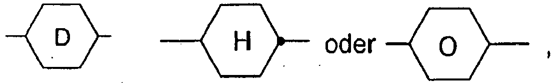 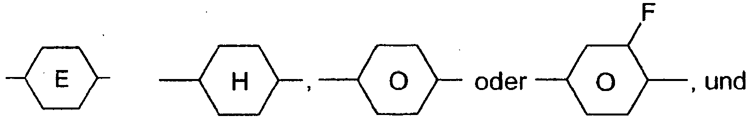
   - e: 1 oder 2.

Die Verbindungen der Formel DK sind vorzugsweise ausgewählt aus den folgenden Unterformeln: 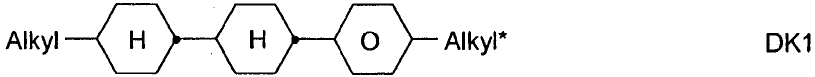 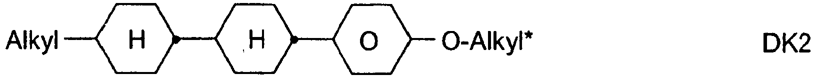 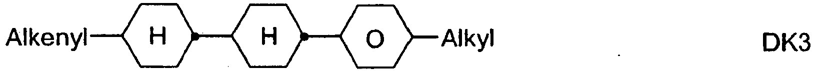 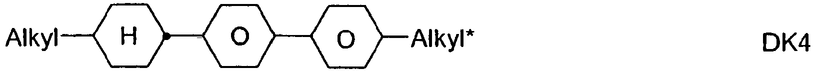 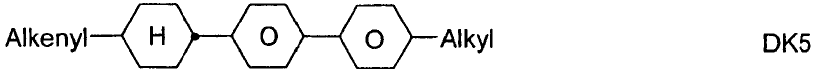 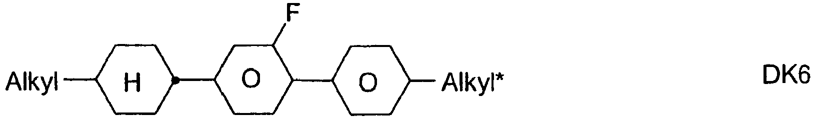 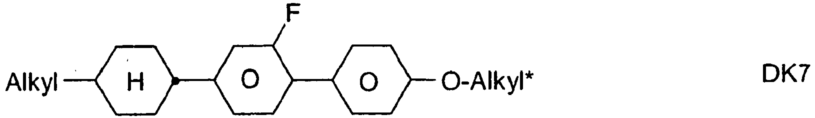 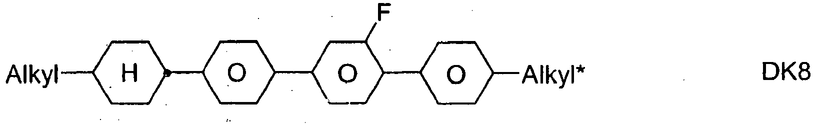 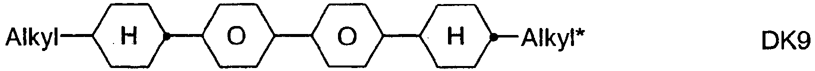 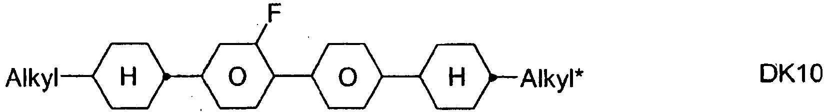 worin Alkyl und Alkyl* jeweils unabhängig voneinander einen geradkettigen Alkylrest mit 1-6 C-Atomen, und Alkenyl und Alkenyl* jeweils unabhängig voneinander einen geradkettigen Alkenylrest mit 2-6 C-Atomen bedeuten. Alkenyl und Alkenyl* bedeuten vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
d) FK-Medium, welches zusätzlich eine oder mehrere Verbindungen der folgenden Formel enthält: 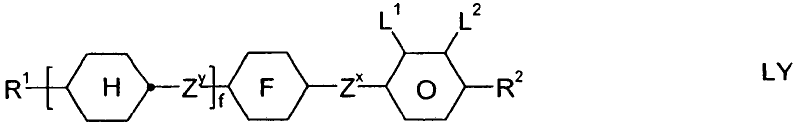 worin die einzelnen Reste folgende Bedeutung besitzen 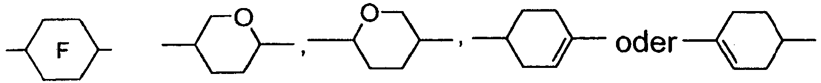
   - f: 0 oder 1,
   - R¹ und: R² jeweils unabhängig voneinander Alkyl mit 1 bis 12 C-Atomen, wobei auch eine oder zwei nicht benachbarte CH₂-Gruppen durch -O-, -CH=CH-, -CO-, -OCO- oder - COO- so ersetzt sein können, dass O-Atome nicht direkt miteinander verknüpft sind,
   - Z^{x} und Z^{y}: jeweils unabhängig voneinander -CH₂CH₂-, -CH=CH-, -CF₂O- -OCF₂-, -CH₂O-, -OCH₂-, -COO-, -OCO-, -C₂F₄-, -CF=CF-, -CH=CHCH₂O-, oder eine Einfachbindung, vorzugsweise eine Einfachbindung,
   - L¹ und L²: jeweils unabhängig voneinander F, Cl, OCF₃, CF₃, CH₃, CH₂F, CHF₂.

Vorzugsweise bedeuten beide Reste L¹ und L² F oder einer der Reste L¹ und L² F und der andere Cl.

Die Verbindungen der Formel LY sind vorzugsweise ausgewählt aus den folgenden Unterformeln 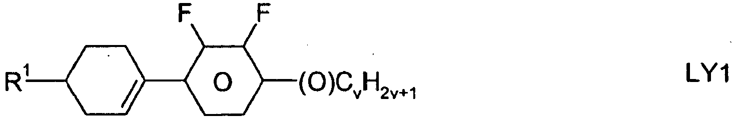 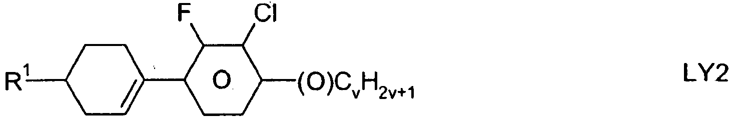 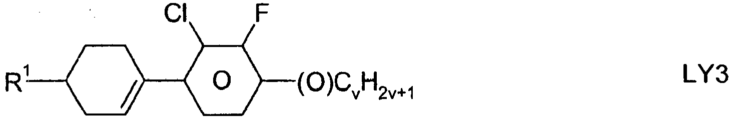 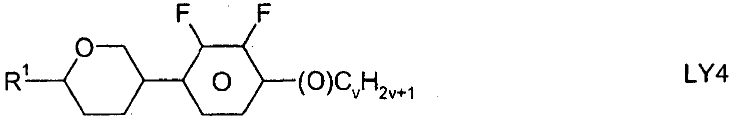 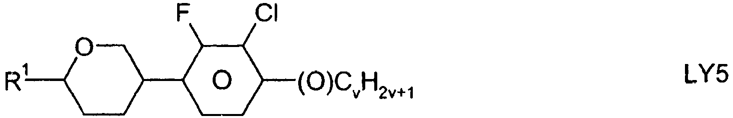 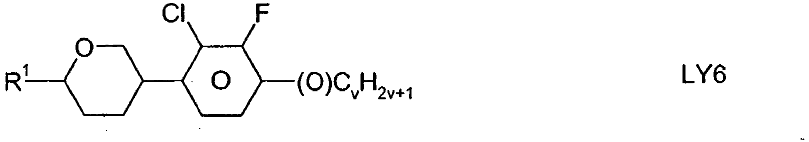 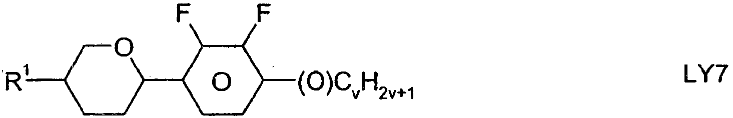 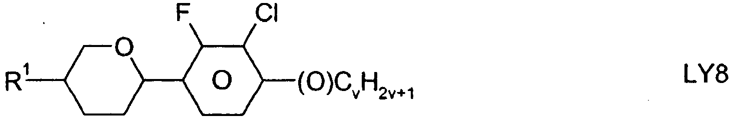 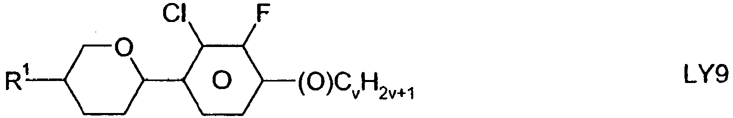 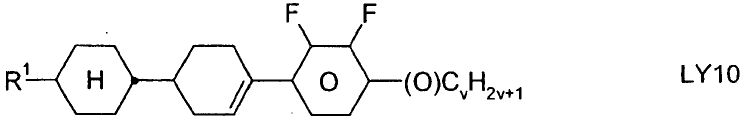 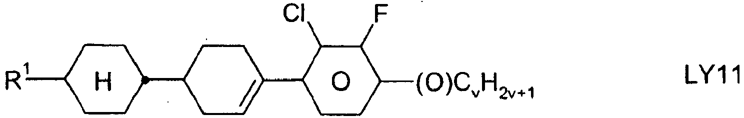 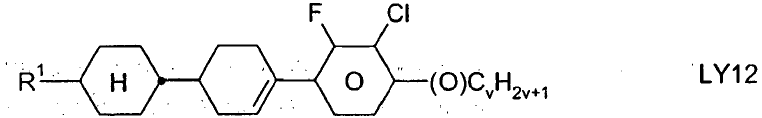 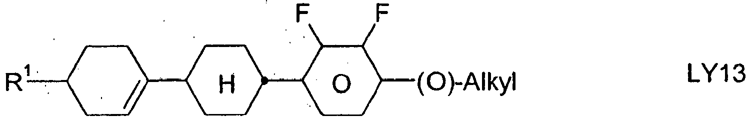 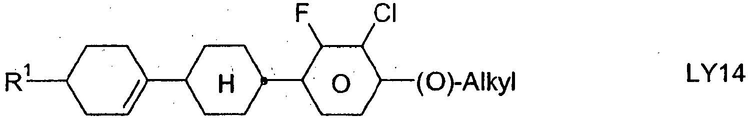 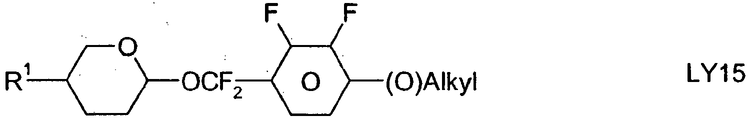 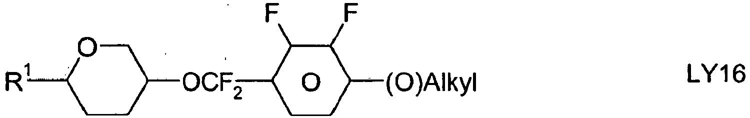 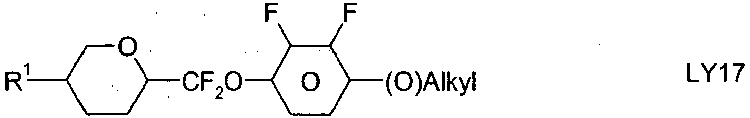 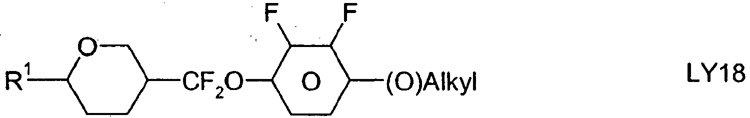 worin R¹ die oben angegebene Bedeutung hat und v eine ganze Zahl von 1 bis 6 bedeutet. R¹ bedeutet vorzugsweise geradkettiges Alkyl oder Alkenyl, insbesondere CH₃, C₂H₅, n-C₃H₇, n-C₄H₉, n-C₅H₁₁, CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.
e) FK-Medium, welches zusätzlich eine oder mehrere Verbindungen ausgewählt aus den folgenden Formeln enthält: 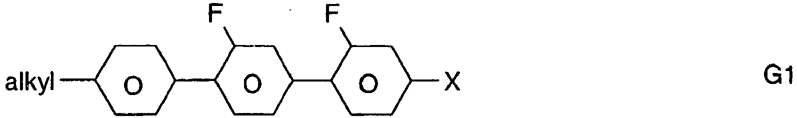 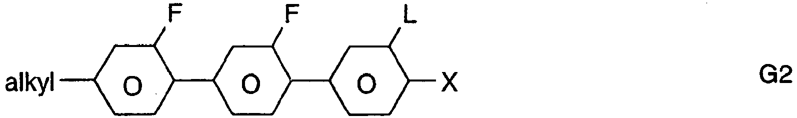 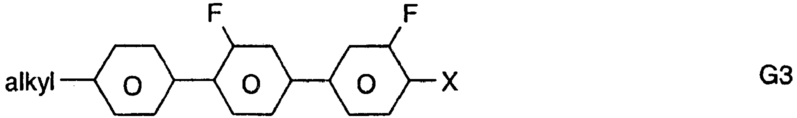 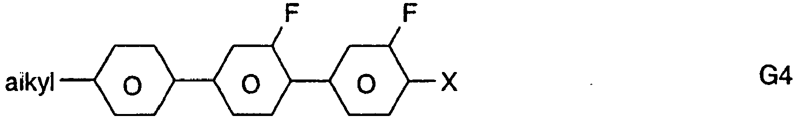 worin alkyl C₁₋₆-alkyl, L H oder F und X F, Cl, OCF₃, OCHF₂ oder OCH=CF₂ bedeutet. Besonders bevorzugt sind Verbindungen der Formel G1, worin X F bedeutet.
f) FK-Medium, welches zusätzlich eine oder mehrere Verbindungen ausgewählt aus den folgenden Formeln enthält: 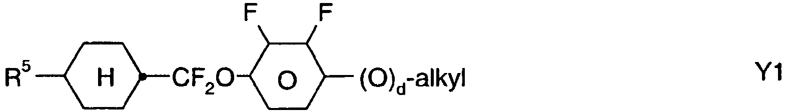 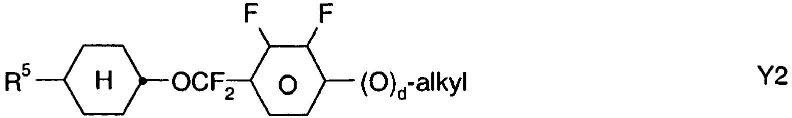 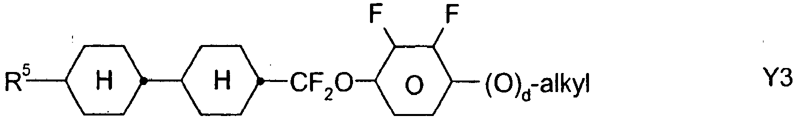 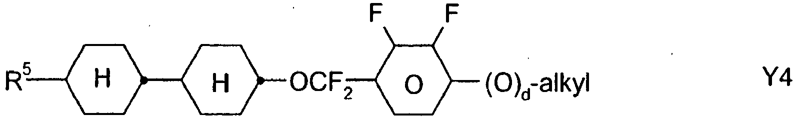 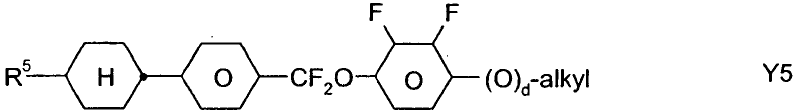 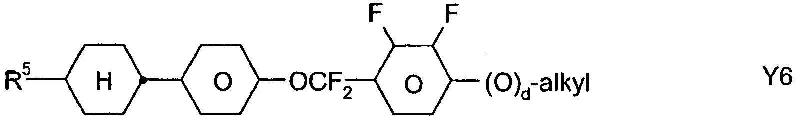 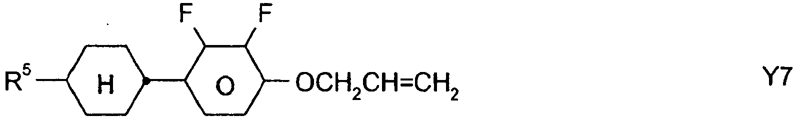 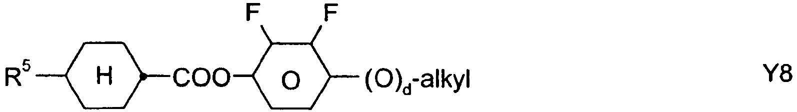 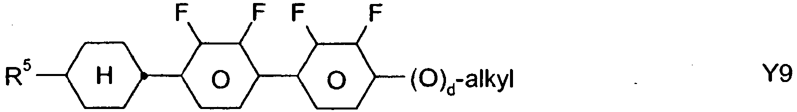 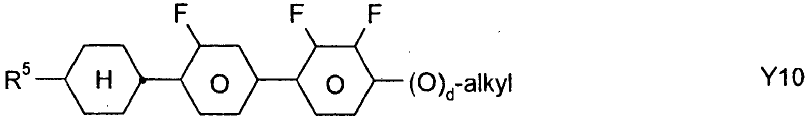 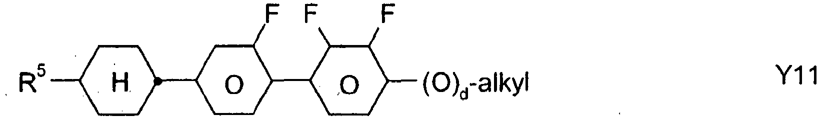 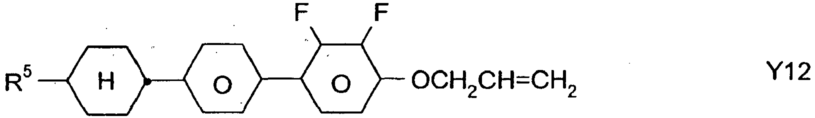 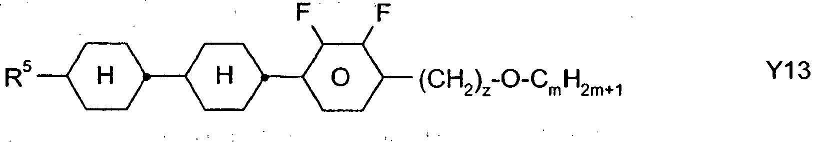 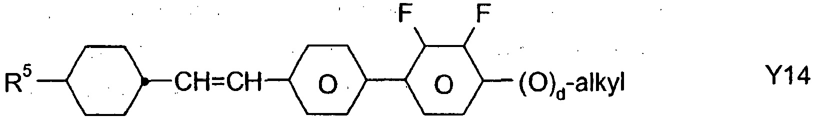 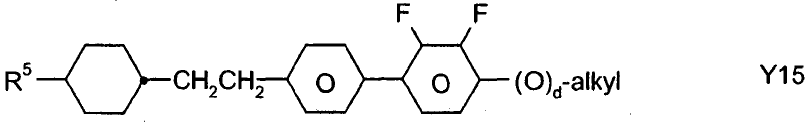 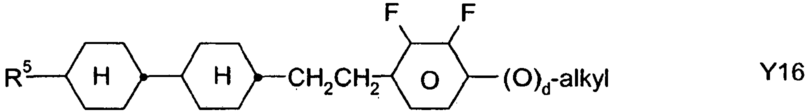 worin R⁵ eine der oben für R¹ angegebenen Bedeutungen besitzt, alkyl C₁₋₆-alkyl, d 0 oder 1, und z und m jeweils unabhängig voneinander eine ganze Zahl von 1 bis 6 bedeuten. R⁵ ist in diesen Verbindungen besonders bevorzugt C₁₋₆-alkyl oder -alkoxy oder C₂₋₆-alkenyl, d ist vorzugsweise 1. Vorzugsweise enthält das erfindungsgemäße FK-Medium eine oder mehrere Verbindungen der oben genannten Formeln in Mengen von ≥ 5 Gew.%.
g) FK-Medium, welches zusätzlich eine oder mehrere Biphenylverbindungen der folgenden Formeln enthält: 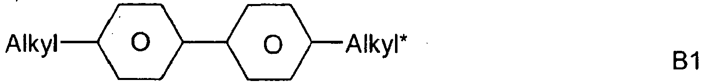 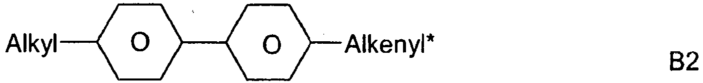 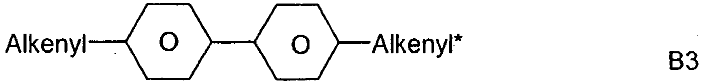
worin Alkyl und Alkyl* jeweils unabhängig voneinander einen geradkettigen Alkylrest mit 1-6 C-Atomen, und Alkenyl und Alkenyl* jeweils unabhängig voneinander einen geradkettigen Alkenylrest mit 2-6 C-Atomen bedeuten. Alkenyl und Alkenyl* bedeuten vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.

Der Anteil der Biphenyle der Formeln B1 bis B3 in der FK-Mischung beträgt vorzugsweise mindestens 3 Gew.%, insbesondere ≥ 5 Gew.%.

Die Verbindungen der Formel B2 sind besonders bevorzugt.

Die Verbindungen der Formel B1 bis B3 sind vorzugsweise ausgewählt aus den folgenden Unterformeln 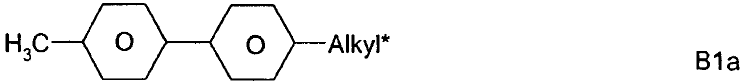 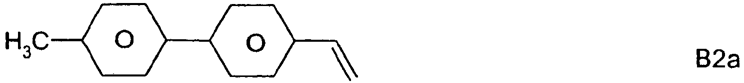 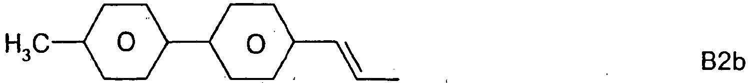 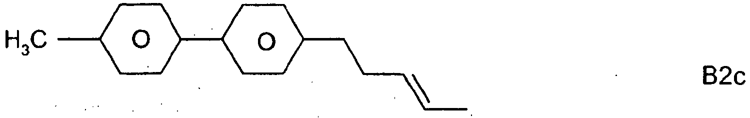 worin Alkyl* einen Alkylrest mit 1-6 C-Atomen bedeutet. Insbesondere bevorzugt enthält das erfindungsgemäße Medium eine oder mehrere Verbindungen der Formeln B1a und/oder B2c.
h) FK-Medium, welches zusätzlich eine oder mehrere Terphenylverbindungen der folgenden Formel enthält: 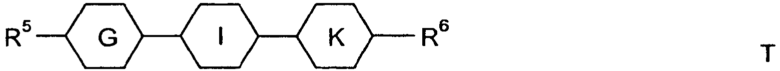 worin R⁵ und R⁶ jeweils unabhängig voneinander eine der oben für R¹ angegebenen Bedeutungen besitzen und 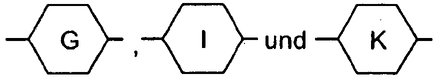 jeweils unabhängig voneinander 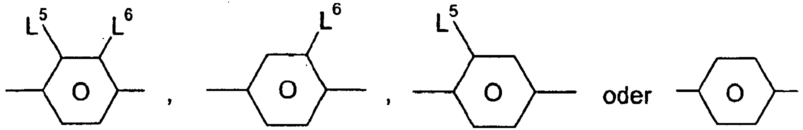 bedeuten, worin L⁵ F oder Cl, vorzugsweise F, und L⁶ F, Cl, OCF₃, CF₃, CH₃, CH₂F oder CHF₂, vorzugsweise F, bedeuten.

Die Verbindungen der Formel T sind vorzugsweise ausgewählt aus den folgenden Unterformeln 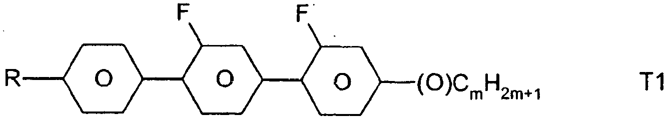 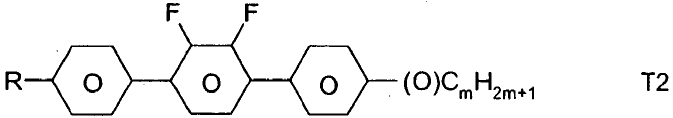 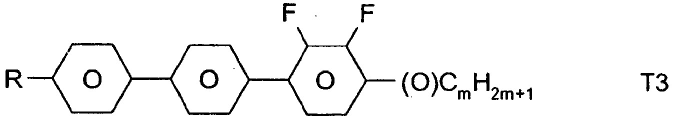 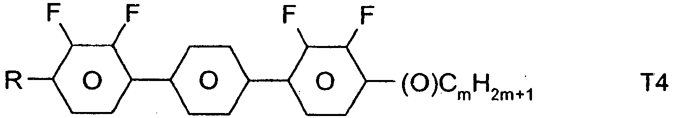 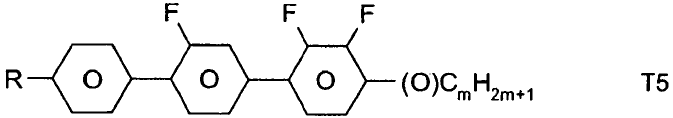 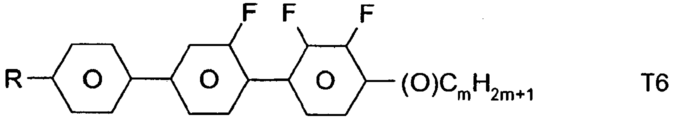 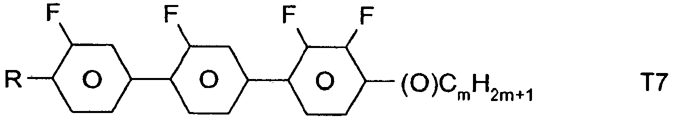 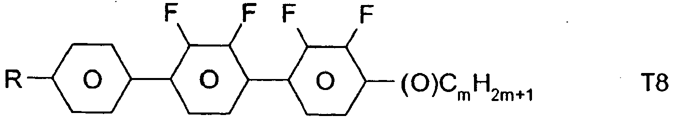 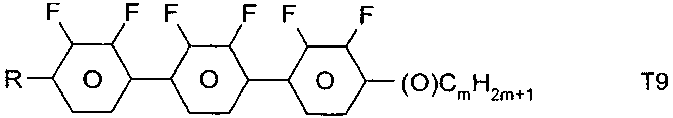 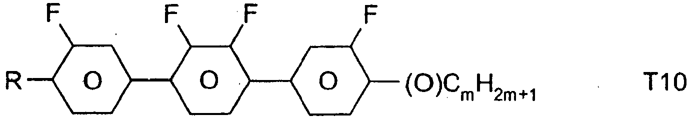 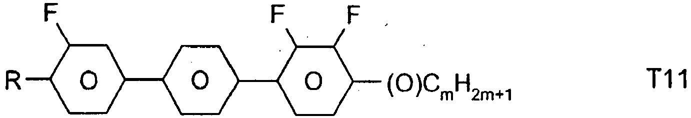 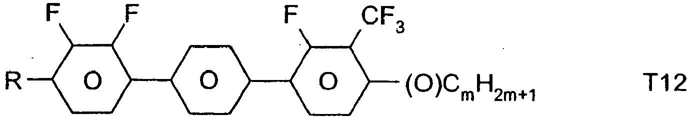 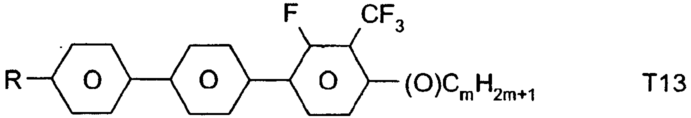 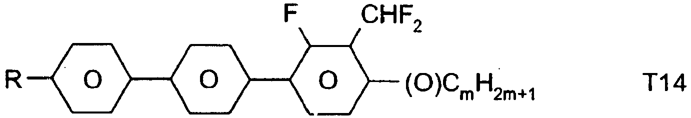 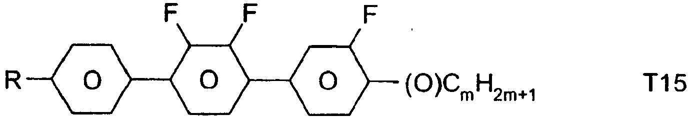 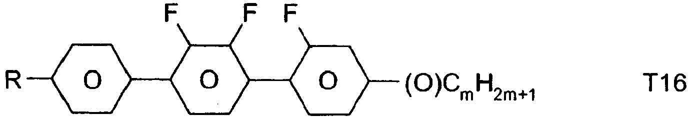 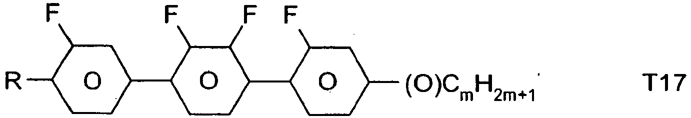 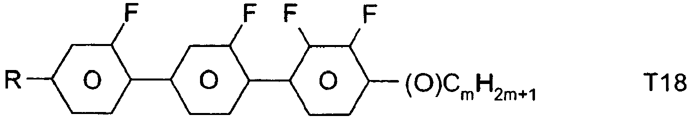 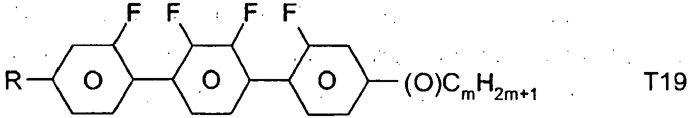 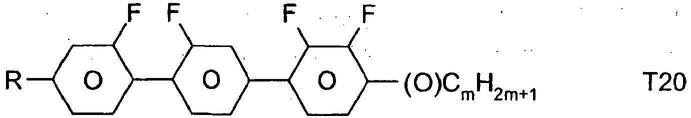 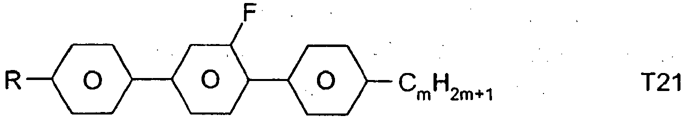 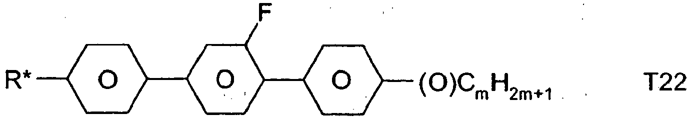 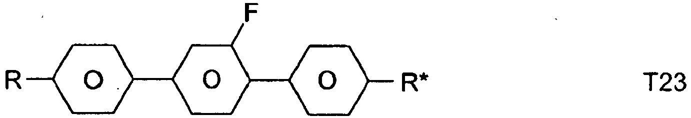 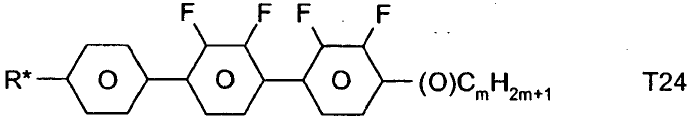 worin R einen geradkettigen Alkyl- oder Alkoxyrest mit 1-7 C-Atomen, R* einen geradkettigen Alkenylrest mit 2-7 C-Atomen, und m eine ganze Zahl von 1 bis 6 bedeutet. R* bedeutet vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.

Vorzugsweise bedeutet R Methyl, Ethyl, Propyl, Butyl, Pentyl, Hexyl Methoxy, Ethoxy, Propoxy, Butoxy oder Pentoxy.

Das erfindungsgemäße FK-Medium enthält die Terphenyle der Formeln T und deren bevorzugte Unterformeln vorzugsweise in einer Menge von 2-30 Gew.%, insbesondere von 5-20 Gew.%.

Besonders bevorzugt sind Verbindungen der Formeln T1, T2, T3 und T21. In diesen Verbindungen bedeutet R vorzugsweise Alkyl, ferner Alkoxy jeweils mit 1-5 C-Atomen.

Vorzugsweise werden die Terphenyle in erfindungsgemäßen Mischungen eingesetzt, wenn der Δn-Wert der Mischung ≥ 0,1 sein soll. Bevorzugte Mischungen enthalten 2-20 Gew.% einer oder mehrerer Terphenyl-Verbindungen der Formel T, vorzugsweise ausgewählt aus der Gruppe der Verbindungen T1 bis T22.
i) FK-Medium, welches zusätzlich eine oder mehrere Verbindungen der folgenden Formeln enthält: 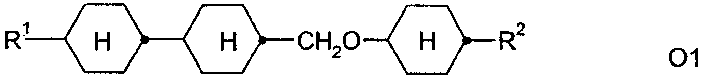 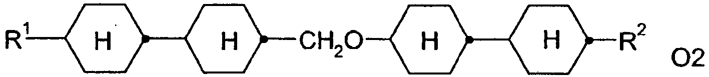 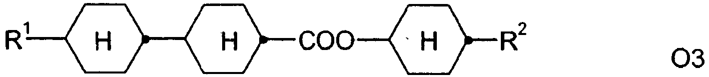 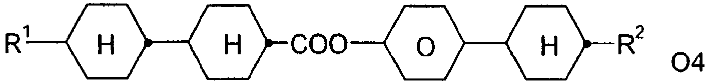 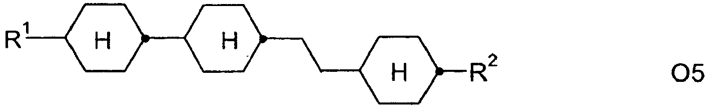 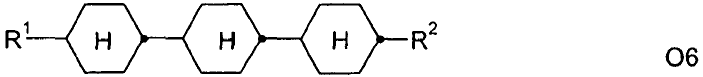 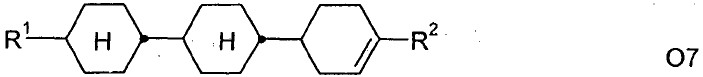 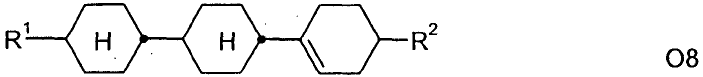 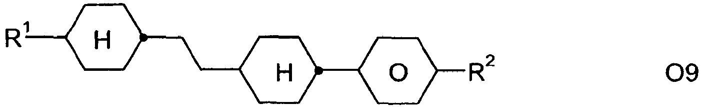 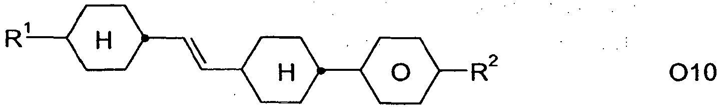 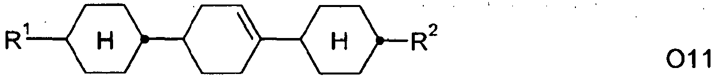 worin R¹ und R² die oben angegebenen Bedeutungen haben, und vorzugsweise jeweils unabhängig voneinander geradkettiges Alkyl oder Alkenyl bedeuten.

Bevorzugte Medien enthalten eine oder mehrere Verbindungen ausgewählt aus den Formeln O1, 03 und 04.
k) FK-Medium, welches zusätzlich eine oder mehrere Verbindungen der folgenden Formel enthält: 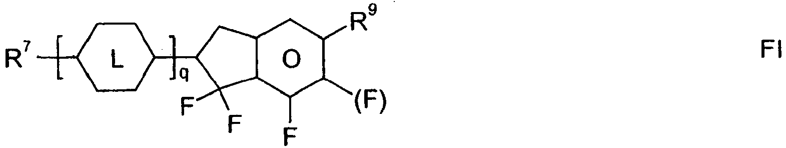 worin 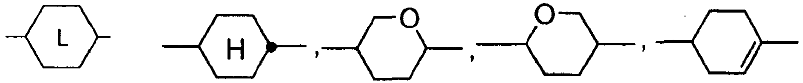 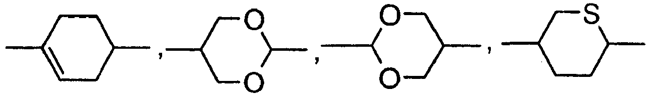 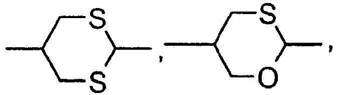
R⁹ H, CH₃, C₂H₅ oder n-C₃H₇ und q 1, 2 oder 3 bedeutet, und R⁷ eine der für R¹ angegebenen Bedeutungen hat, vorzugsweise in Mengen von > 3 Gew.%, insbesondere ≥ 5 Gew.%, und ganz besonders bevorzugt von 5-30 Gew.%.

Besonders bevorzugte Verbindungen der Formel IF sind ausgewählt aus den folgenden Unterformeln: 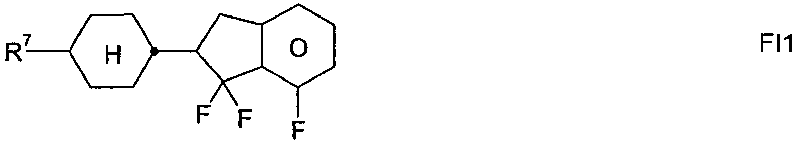 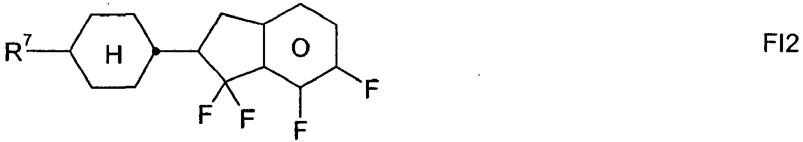 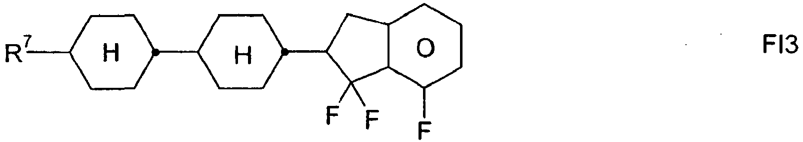 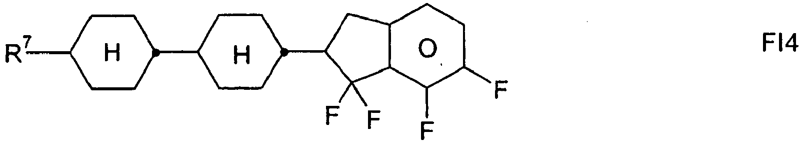 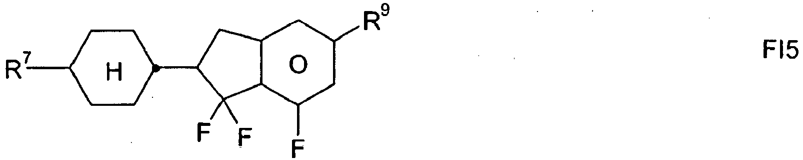 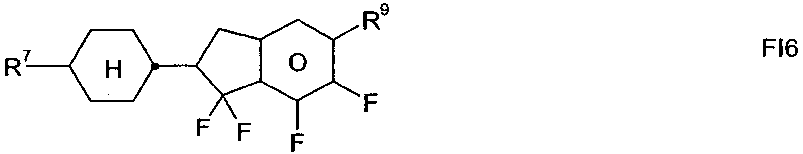 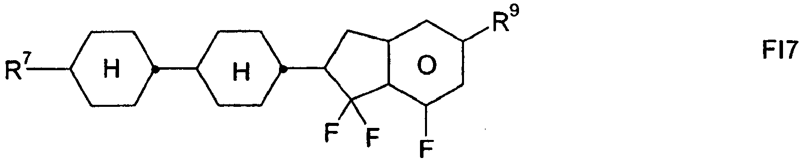 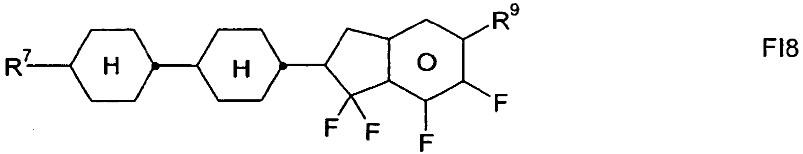 worin R⁷ vorzugsweise geradkettiges Alkyl bedeutet und R⁹ CH₃, C₂H₅ oder n-C₃H₇ bedeutet. Besonders bevorzugt sind die Verbindungen der Formel FI1, FI2 und FI3.
m) FK-Medium, welches zusätzlich eine oder mehrere Verbindungen der folgenden Formeln enthält: 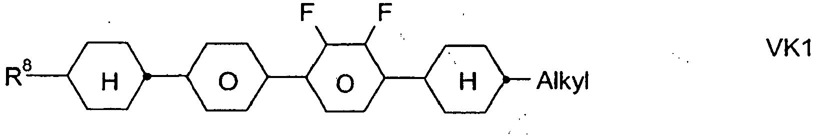 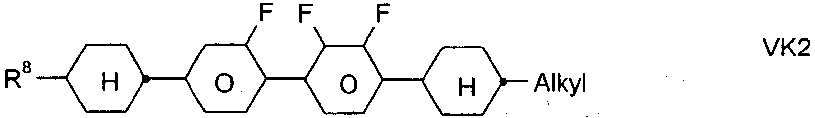 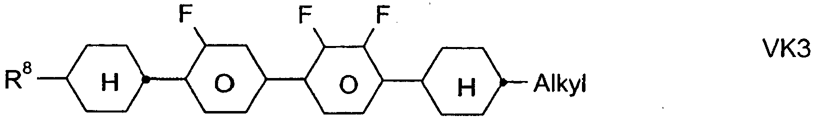 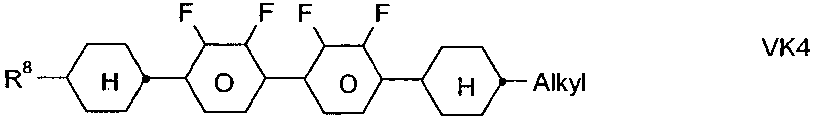 worin R⁸ die für R¹ angegebene Bedeutung hat und Alkyl einen geradkettigen Alkylrest mit 1-6 C-Atomen bedeutet.
n) FK-Medium, welches zusätzlich eine oder mehrere Verbindungen enthält, die eine Tetrahydronaphthyl- oder Naphthyl-Einheit aufweisen, wie z.B. die Verbindungen ausgewählt aus den folgenden Formeln: 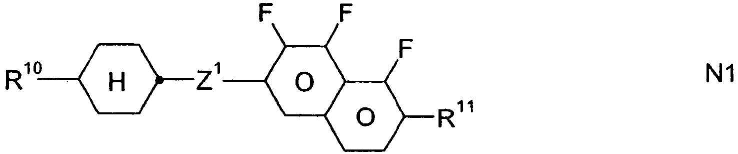 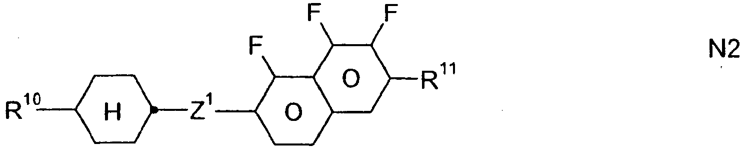 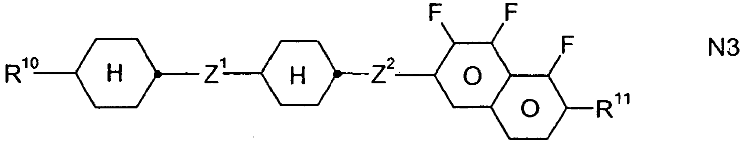 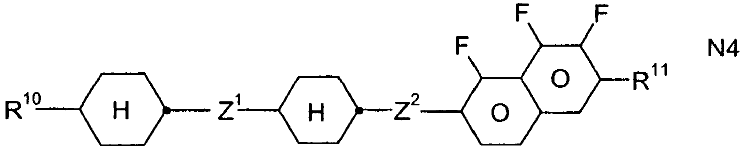 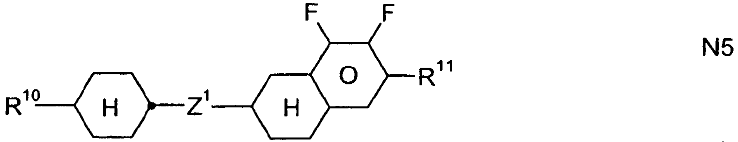 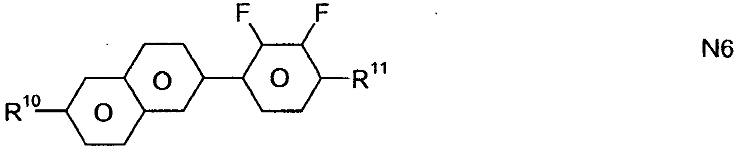 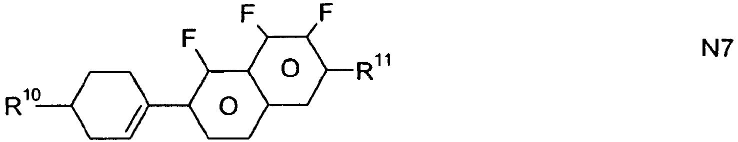 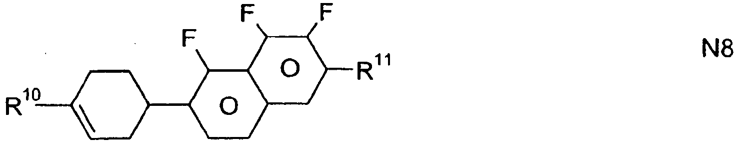 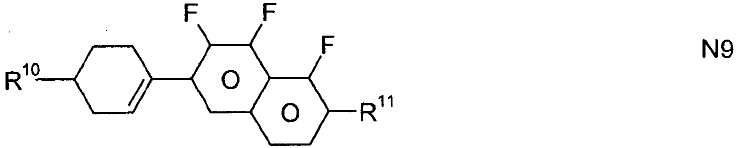 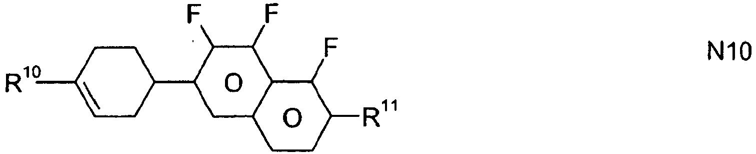 worin R¹⁰ und R¹¹ jeweils unabhängig voneinander eine der für R¹ angegebenen Bedeutungen haben, vorzugsweise geradkettiges Alkyl, geradkettiges Alkoxy oder geradkettiges Alkenyl bedeuten, und Z, Z¹ und Z² jeweils unabhängig voneinander -C₂H₄-, -CH=CH-, - (CH₂)₄-, -(CH₂)₃O-, -O(CH₂)₃-, -CH=CHCH₂CH₂-, -CH₂CH₂CH=CH-, - CH₂O-, -OCH₂-, -COO-, -OCO-, -C₂F₄-, -CF=CF-, -CF=CH-, -CH=CF-, -CH₂- oder eine Einfachbindung bedeuten.
o) FK-Medium, welches zusätzlich eine oder mehrere eine oder mehrere Difluordibenzochromane und/oder Chromane der folgenden Formeln enthält: 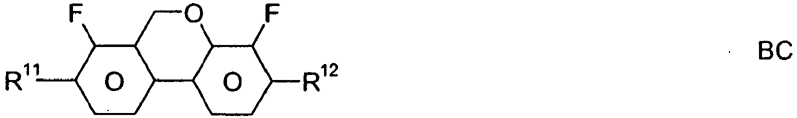 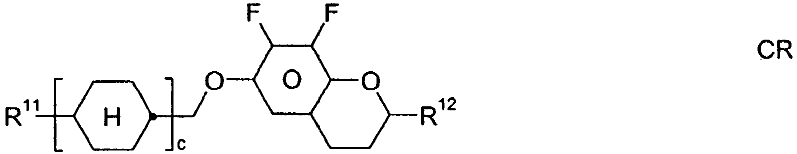 worin R¹¹ und R¹² jeweils unabhängig voneinander die oben angegebene Bedeutung aufweisen, und c 0 oder 1 bedeutet, vorzugsweise in Mengen von 3 bis 20 Gew.%, insbesondere in Mengen von 3 bis 15 Gew.%.

Besonders bevorzugte Verbindungen der Formeln BC und CR sind ausgewählt aus den folgenden Unterformeln: 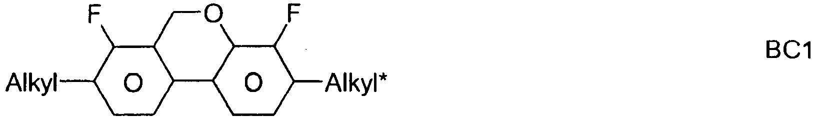 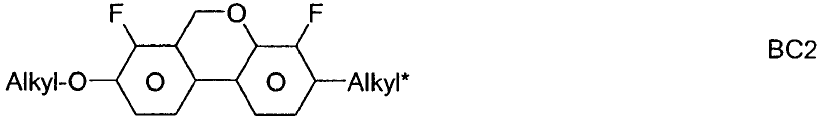 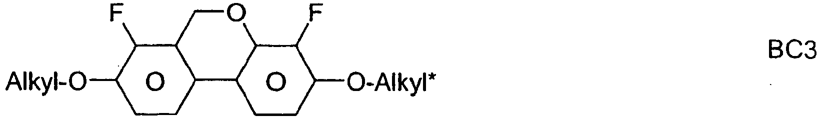 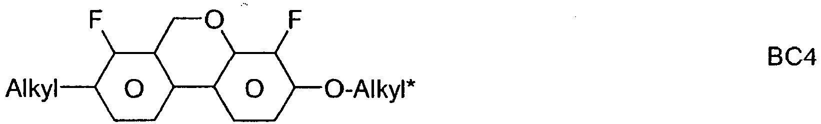 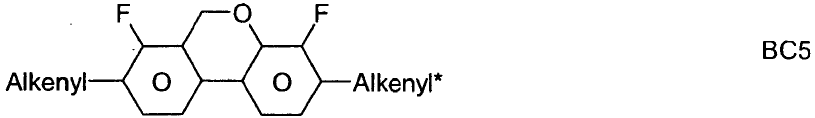 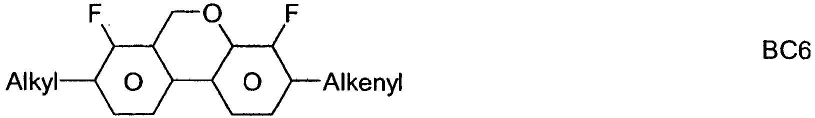 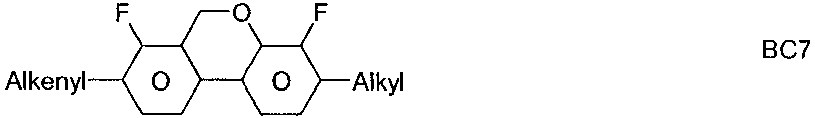 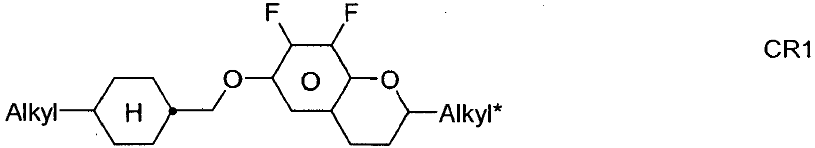 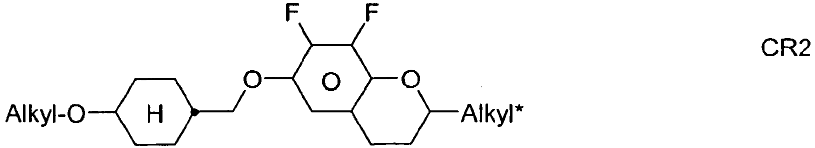 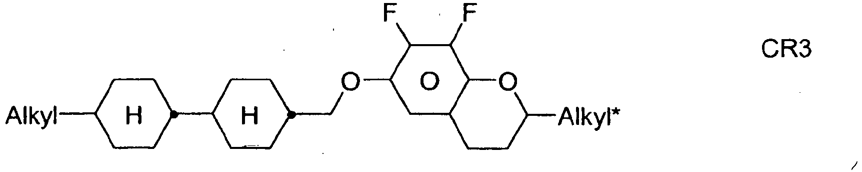 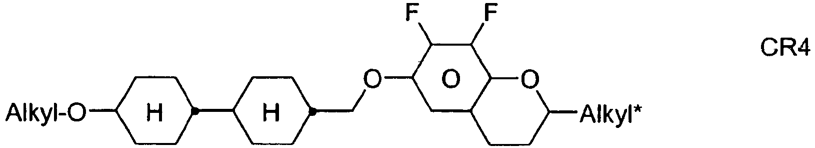 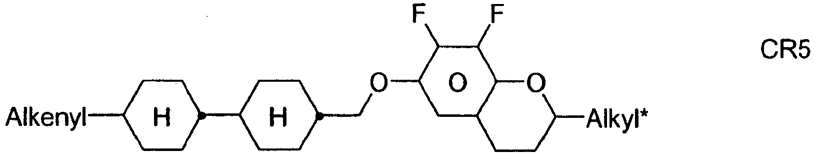 worin Alkyl und Alkyl* jeweils unabhängig voneinander einen geradkettigen Alkylrest mit 1-6 C-Atomen, und Alkenyl und Alkenyl* jeweils unabhängig voneinander einen geradkettigen Alkenylrest mit 2-6 C-Atomen bedeuten. Alkenyl und Alkenyl* bedeuten vorzugsweise CH₂=CH-, CH₂=CHCH₂CH₂-, CH₃-CH=CH-, CH₃-CH₂-CH=CH-, CH₃-(CH₂)₂-CH=CH-, CH₃-(CH₂)₃-CH=CH- oder CH₃-CH=CH-(CH₂)₂-.

Ganz besonders bevorzugt sind Mischungen enthaltend eine, zwei oder drei Verbindungen der Formel BC-2.
p) FK-Medium, welches zusätzlich eine oder mehrere fluorierte Phenanthrene oder Dibenzofurane der folgenden Formeln enthält: 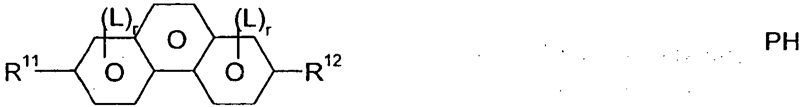 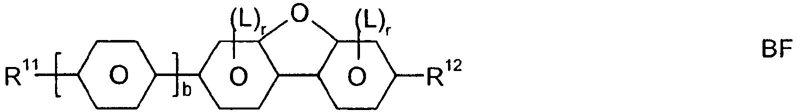 worin R¹¹ und R¹² jeweils unabhängig voneinander die oben angegebenen Bedeutungen besitzen, b 0 oder 1, L F und r 1, 2 oder 3 bedeutet.

Besonders bevorzugte Verbindungen der Formeln PH und BF sind ausgewählt aus den folgenden Unterformeln: 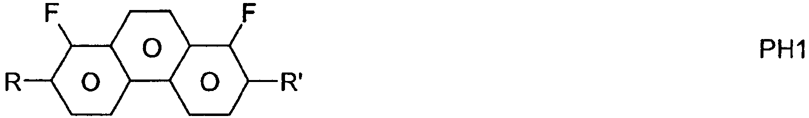 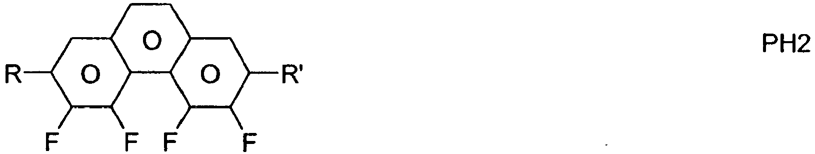 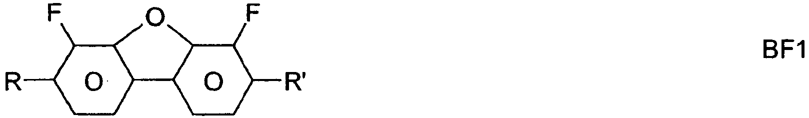 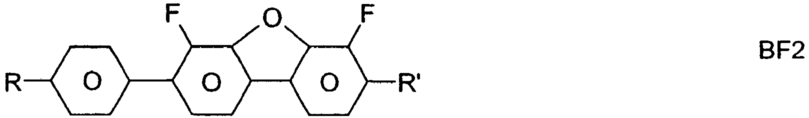 worin R und R' jeweils unabhängig voneinander einen geradkettigen Alkyl- oder Alkoxyrest mit 1-7 C-Atomen bedeuten.
q) FK-Medium, vorzugsweise zur Verwendung in PSA-OCB-Anzeigen, welches eine oder mehrere Verbindungen der folgenden Formeln enthält: 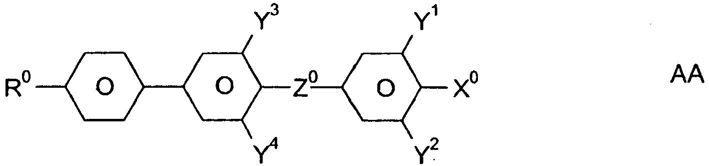 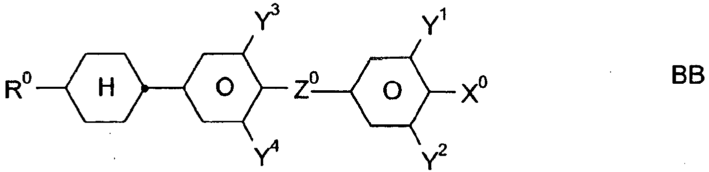 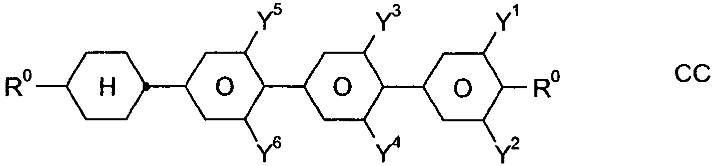 worin
   - R⁰: bei jedem Auftreten gleich oder verschieden n-Alkyl, Alkoxy, Oxaalkyl, Fluoralkyl oder Alkenyl mit jeweils bis zu 9 C-Atomen,
   - X⁰: F, Cl oder jeweils halogeniertes Alkyl, Alkenyl, Alkenyloxy oder Alkoxy mit jeweils bis zu 6 C-Atomen,
   - Z⁰: -CF₂O- oder eine Einfachbindung,
   - Y¹⁻⁶: jeweils unabhängig voneinander H oder F,
   bedeuten.
   X⁰ ist vorzugsweise F, Cl, CF₃, CHF₂, OCF₃, OCHF₂, OCFHCF₃, OCFHCHF₂, OCFHCHF₂, OCF₂CH₃, OCF₂CHF₂, OCF₂CHF₂, OCF₂CF₂CHF₂, OCF₂CF₂CHF₂, OCFHCF₂CF₃, OCFHCF₂CHF₂, OCF₂CF₂CF₃, OCF₂CF₂CClF₂, OCClFCF₂CF₃ oder CH=CF₂, besonders bevorzugt F oder OCF₃.

Die Verbindungen der Formel AA sind vorzugsweise ausgewählt aus den folgenden Formeln: 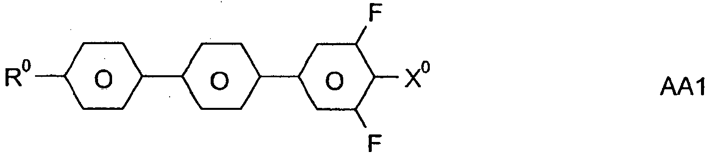 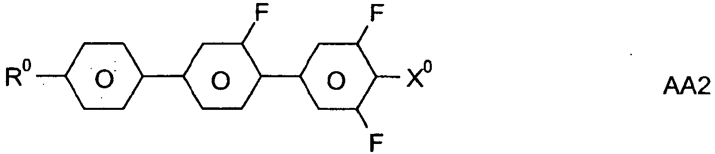 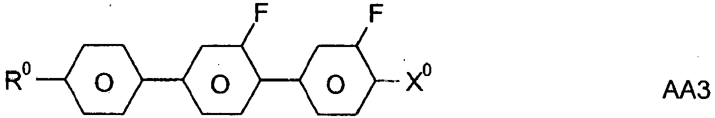 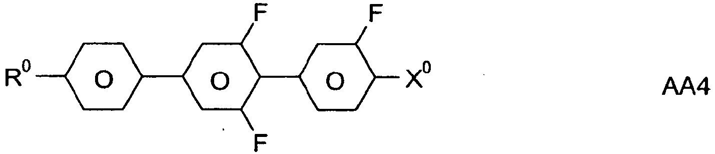 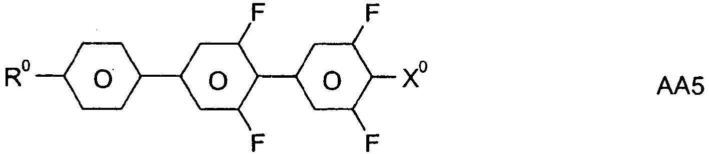 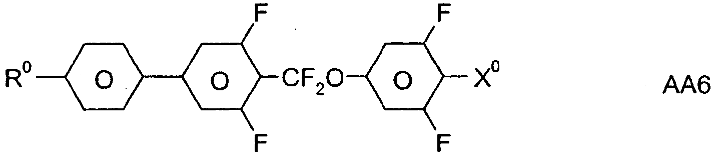 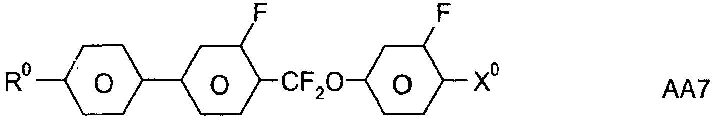 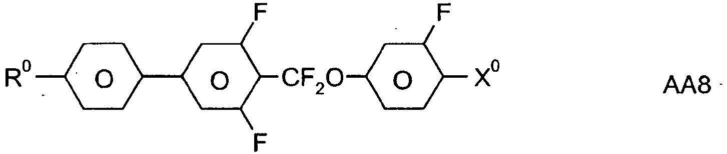 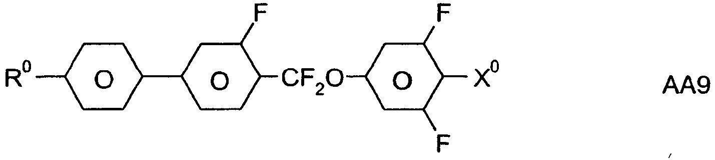 worin R⁰ und X⁰ die oben angegebene Bedeutung besitzen, und X⁰ vorzugsweise F bedeutet. Besonders bevorzugt sind Verbindungen der Formeln AA2 und AA6.

Die Verbindungen der Formel BB sind vorzugsweise ausgewählt aus den folgenden Formeln: 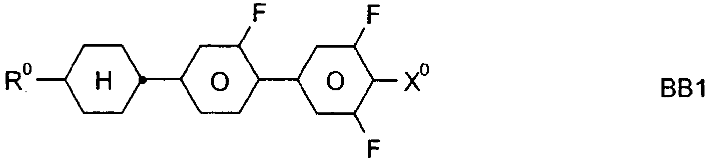 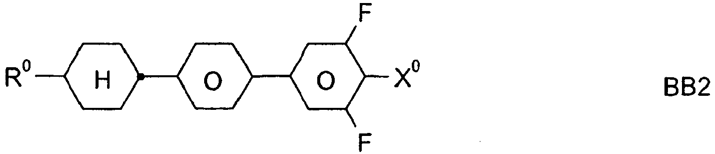 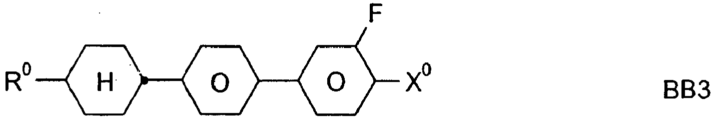 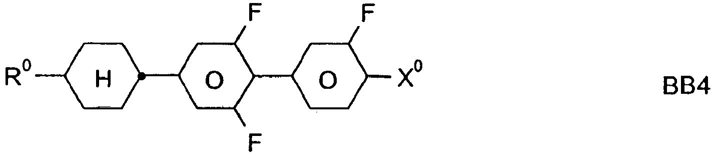 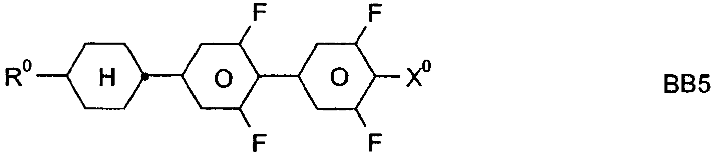 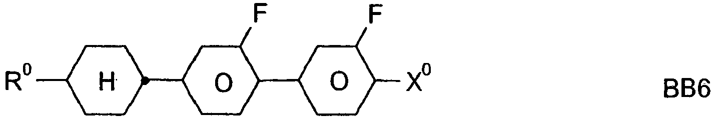 worin R⁰ und X⁰ die oben angegebene Bedeutung besitzen, und X⁰ vorzugsweise F bedeutet. Besonders bevorzugt sind Verbindungen der Formeln BB1, BB2 und BB5.

Die Verbindungen der Formel CC sind vorzugsweise ausgewählt aus den folgenden Formeln: 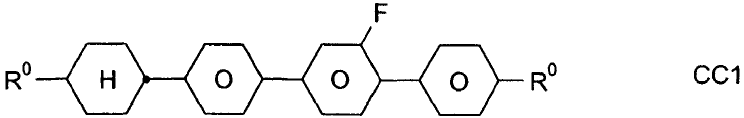 worin R⁰ bei jedem Auftreten gleich oder verschieden die oben angegebene Bedeutung besitzt, und vorzugsweise Alkyl mit 1 bis 6 C-Atomen bedeutet.
r) FK-Medium, welches außer den polymerisierbaren Verbindungen der Formel I oder deren Unterformeln sowie den Comonomeren keine Verbindungen enthält, die eine endständige Vinyloxygruppe (-O-CH=CH₂) aufweisen.
s) FK-Medium, welches 1 bis 5, vorzugsweise 1, 2 oder 3 polymerisierbare Verbindungen enthält.
t) FK-Medium, worin der Anteil an polymerisierbaren Verbindungen im Gesamtgemisch 0,05 bis 5 %, vorzugsweise 0,1 bis 1 % beträgt.
u) FK-Medium, welches 1 bis 8, vorzugsweise 1 bis 5 Verbindungen der Formel CY1, CY2, PY1 und/oder PY2 enthält. Der Anteil dieser Verbindungen im Gesamtgemisch beträgt vorzugsweise 5 bis 60 %, besonders bevorzugt 10 bis 35 %. Der Gehalt dieser einzelnen Verbindungen beträgt vorzugsweise jeweils 2 bis 20 %.
v) FK-Medium, welches 1 bis 8, vorzugsweise 1 bis 5 Verbindungen der Formel CY9, CY10, PY9 und/oder PY10 enthält. Der Anteil dieser Verbindungen im Gesamtgemisch beträgt vorzugsweise 5 bis 60 %, besonders bevorzugt 10 bis 35 %. Der Gehalt dieser einzelnen Verbindungen beträgt vorzugsweise jeweils 2 bis 20 %.
w) FK-Medium, welches 1 bis 10, vorzugsweise 1 bis 8 Verbindungen der Formel ZK enthält, insbesondere Verbindungen der Formel ZK1, ZK2 und/oder ZK6. Der Anteil dieser Verbindungen im Gesamtgemisch beträgt vorzugsweise 3 bis 25 %, besonders bevorzugt 5 bis 45 %. Der Gehalt dieser einzelnen Verbindungen beträgt vorzugsweise jeweils 2 bis 20 %.
x) FK-Medium, worin der Anteil an Verbindungen der Formel CY, PY und ZK im Gesamtgemisch mehr als 70 %, vorzugsweise mehr als 80 % beträgt.
y) FK-Medium, welches zusätzlich ein oder mehrere, vorzugsweise niedermolekulare und/oder nicht polymerisierbare, chirale Dotierstoffe enthält, besonders bevorzugt ausgewählt aus der untenstehenden Tabelle B sowie in den dort angegebenen Konzentrationsbereichen.

Die Kombination von Verbindungen der oben genannten bevorzugten Ausführungsformen a)-y) mit den oben beschriebenen polymerisierten Verbindungen bewirkt in den erfindungsgemäßen FK-Medien niedrige Schwellenspannungen, niedrige Rotationsviskositäten und sehr gute Tieftemperaturstabilitäten bei gleichbleibend hohen Klärpunkten und hohen HR-Werten, und erlaubt die Einstellung eines Pretilt-Winkels in PSA-Anzeigen. Insbesondere zeigen die FK-Medien in PSA-Anzeigen im Vergleich zu den Medien aus dem Stand der Technik deutlich verringerte Schaltzeiten, insbesondere auch der Graustufenschaltzeiten.

Vorzugsweise weist die Flüssigkristallmischung einen nematischen Phasenbereich von mindestens 80 K, besonders bevorzugt von mindestens 100 K, und eine Rotationsviskosität von nicht mehr als 250, vorzugsweise nicht mehr als 200 mPa·s, bei 20°C auf.

Erfindungsgemäße FK-Medien zur Verwendung in Anzeigen des VA-Typs weisen eine negative dielektrische Anisotropie Δε auf, vorzugsweise von etwa -0,5 bis -7,5, insbesondere von etwa -2,5 bis -5,5 bei 20°C und 1 kHz.

Erfindungsgemäße FK-Medien zur Verwendung in Anzeigen des OCB-Typs weisen eine positive dielektrische Anisotropie Δε auf, vorzugsweise von etwa +7 bis +17 bei 20°C und 1 kHz.

Die Doppelbrechung Δn in erfindungsgemäßen FK-Medien zur Verwendung in Anzeigen des VA-Typs liegt vorzugsweise unter 0,16, besonders bevorzugt zwischen 0,06 und 0,14, insbesondere zwischen 0,07 und 0,12.

Die Doppelbrechung Δn in erfindungsgemäßen FK-Medien zur Verwendung in Anzeigen des OCB-Typs liegt vorzugsweise zwischen 0,14 und 0,22, insbesondere zwischen 0,16 und 0,22.

Die Dielektrika können auch weitere, dem Fachmann bekannte und in der Literatur beschriebene Zusätze enthalten. Beispielsweise können 0 bis 15 Gew.-% pleochroitische Farbstoffe zugesetzt werden, ferner Nanopartikel, Leitsalze, vorzugsweise Ethyl-dimethyldodecylammonium-4-hexoxy-benzoat, Tetrabutylammoniumtetraphenylborat oder Komplexsalze von Kronenethern (vgl. z.B. Haller et al., Mol. Cryst. Liq. Cryst. 24, 249-258 (1973)) zur Verbesserung der Leitfähigkeit, oder Substanzen zur Veränderung der dielektrischen Anisotropie, der Viskosität und/oder der Orientierung der nematischen Phasen. Derartige Substanzen sind z.B. in DE-A 22 09 127, 22 40 864, 23 21 632, 23 38 281, 24 50 088, 26 37 430 und 28 53 728 beschrieben.

Die einzelnen Komponenten der bevorzugten Ausführungsformen a)-y) der erfindungsgemäßen FK-Medien sind entweder bekannt, oder ihre Herstellungsweisen sind für den einschlägigen Fachmann aus dem Stand der Technik ohne weiteres abzuleiten, da sie auf in der Literatur beschriebenen Standardverfahren basieren. Entsprechende Verbindungen der Formel CY werden beispielsweise in EP-A-0 364 538 beschrieben. Entsprechende Verbindungen der Formel ZK werden beispielsweise in DE-A-26 36 684 und DE-A-33 21 373 beschrieben.

Die Herstellung der erfindungsgemäß verwendbaren FK-Medien erfolgt in an sich üblicher Weise, beispielsweise indem man eine oder mehrere der oben genannten Verbindungen mit einer oder mehreren polymerisierbaren Verbindungen wie oben definiert, und ggf. mit weiteren flüssigkristallinen Verbindungen und/oder Additiven mischt. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation. Das Verfahren zur Herstellung der erfindungsgemäßen FK-Medien ist ein weiterer Gegenstand der Erfindung.

Es versteht sich für den Fachmann von selbst, daß die erfindungsgemäßen FK-Medien auch Verbindungen enthalten können, worin beispielsweise H, N, O, Cl, F durch die entsprechenden Isotope ersetzt sind.

Der Aufbau der erfindungsgemäßen FK-Anzeigen entspricht der für PSA-Anzeigen üblichen Geometrie, wie er im eingangs zitierten Stand der Technik beschrieben ist. Es sind Geometrien ohne Protrusions bevorzugt, insbesondere diejenigen, bei denen darüber hinaus die Elektrode auf der Colour Filter-Seite unstrukturiert ist und lediglich die Elektrode auf der TFT-Seite Schlitze aufweist. Besonders geeignete und bevorzugte Elektrodenstrukturen für PSA-VA-Anzeigen sind beispielsweise in US 2006/0066793 A1 beschrieben.

Die folgenden Beispiele erläutern die vorliegende Erfindung ohne sie zu begrenzen. Sie zeigen dem Fachmann jedoch bevorzugte Mischungskonzepte mit bevorzugt einzusetzenden Verbindungen und deren jeweiligen Konzentrationen sowie deren Kombinationen miteinander. Außerdem illustrieren die Beispiele, welche Eigenschaften und Eigenschftskombinationen zugänglich sind.

Folgende Abkürzungen werden verwendet:
(n, m, z: jeweils unabhängig voneinander 1, 2, 3, 4, 5 oder 6)

**Tabelle A**

| | |
|---|---|
| 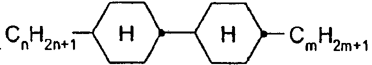 | 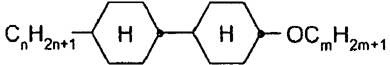 |
| CCH-nm | CCH-nOm |
| 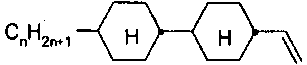 | 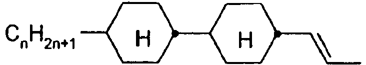 |
| CC-n-V | CC-n-V1 |
| 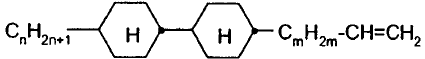 | 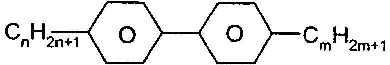 |
| CC-n-mV | PP-n-m |
| 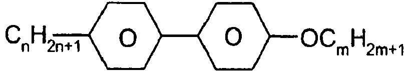 | 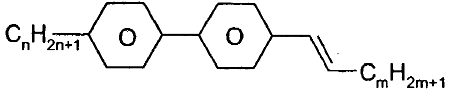 |
| PP-n-Om | PP-n-Vm |
| 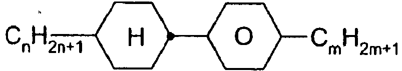 | 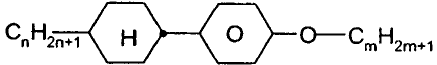 |
| PCH-nm | PCH-nOm |
| 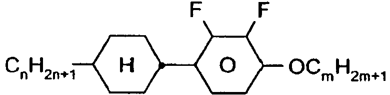 | 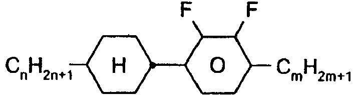 |
| CY-n-Om | CY-n-m |
| 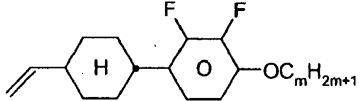 | 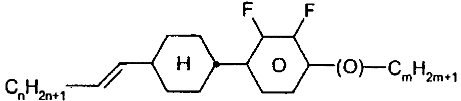 |
| CY-V-Om | CY-nV-(O)m |
| 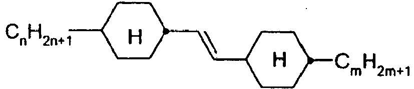 | 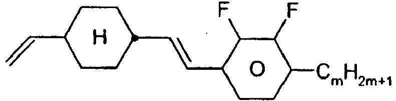 |
| CVC-n-m | CVY-V-m |
| 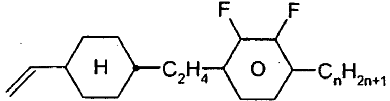 | 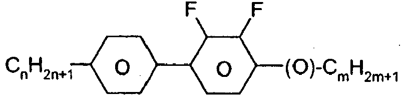 |
| CEY-V-m | PY-n-(O)m |
| 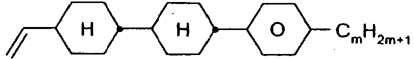 | 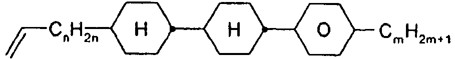 |
| CCP-V-m | CCP-Vn-m |
| 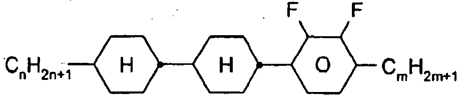 | 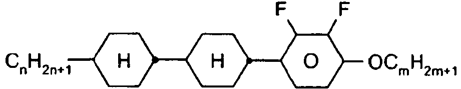 |
| CCY-n-m | CCY-n-Om |
| 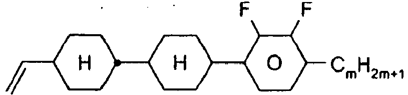 | 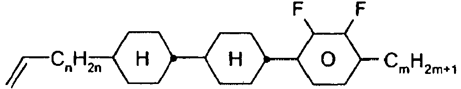 |
| CCY-V-m | CCY-Vn-m |
| 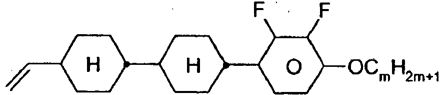 | 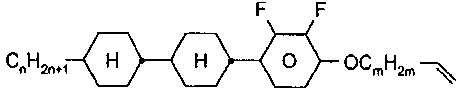 |
| CCY-V-Om | CCY-n-OmV |
| 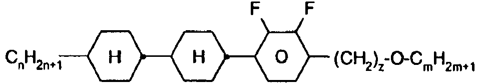 | 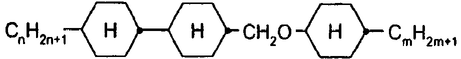 |
| CCY-n-zOm | CCOC-n-m |
| 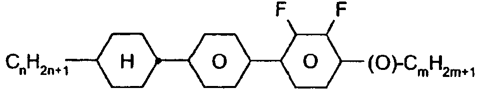 | 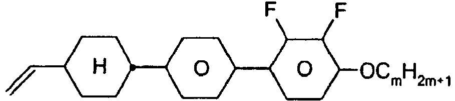 |
| CPY-n-(O)m | CPY-V-Om |
| 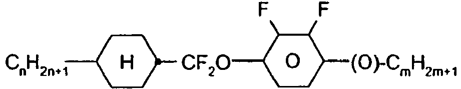 | 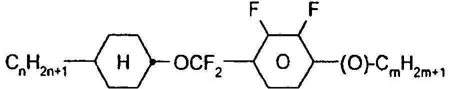 |
| CQY-n-(O)m | CQIY-n-(O)m |
| 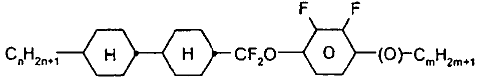 | 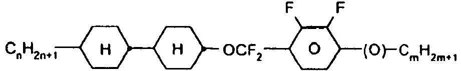 |
| CCQY-n-(0)m | CCQIY-n-(O)m |
| 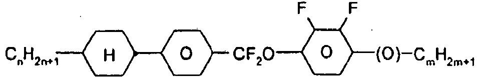 | 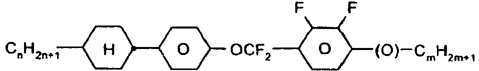 |
| CPQY-n-(O)m | CPQIY-n-Om |
| 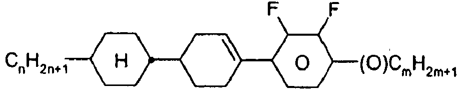 | 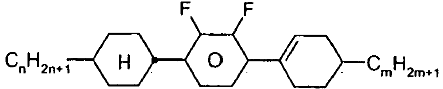 |
| CLY-n-(O)m | CYLI-n-m |
| 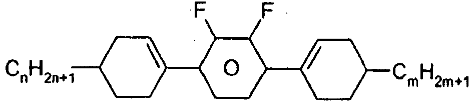 | 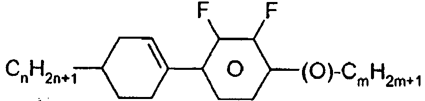 |
| LYLI-n-m | LY-n-(O)m |
| 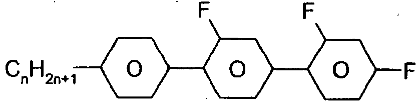 | 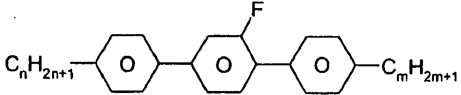 |
| PGIGI-n-F | PGP-n-m |
| 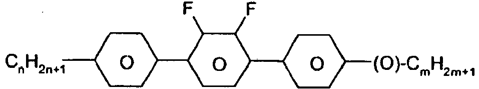 | 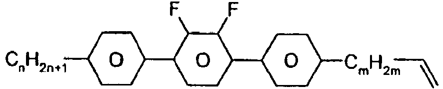 |
| PYP-n-(O)m | PYP-n-mV |
| 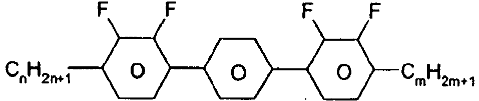 | 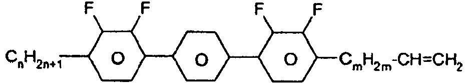 |
| YPY-n-m | YPY-n-mV |
| 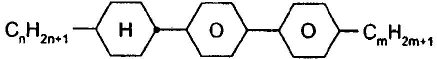 | 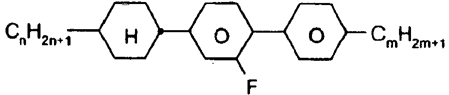 |
| BCH-nm | BCH-nmF |
| 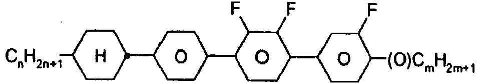 | 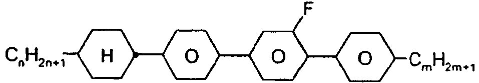 |
| CPYP-n-(O)m | CPGP-n-m |
| 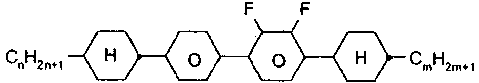 | 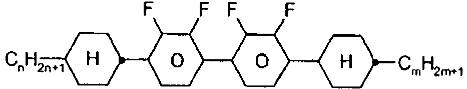 |
| CPYC-n-m | CYYC-n-m |
| 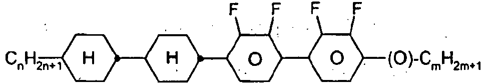 | 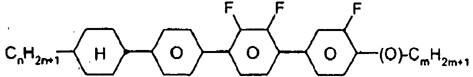 |
| CCYY-n-m | CPYG-n-(O)m |
| 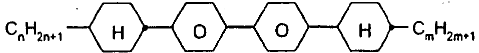 | 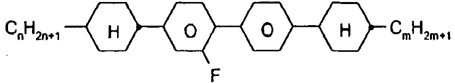 |
| CBC-nm | CBC-nmF |
| 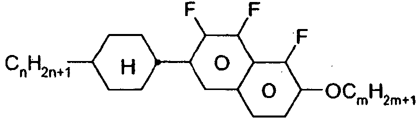 | 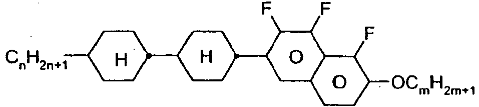 |
| CNap-n-Om | CCNap-n-Om |
| 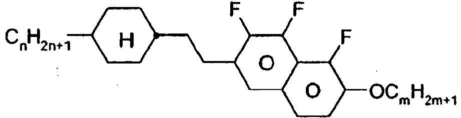 | 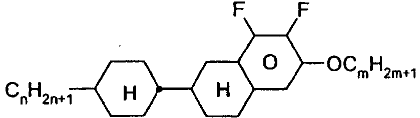 |
| CENap-n-Om | CTNap-n-Om |
| 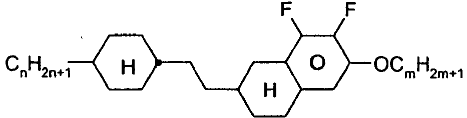 | 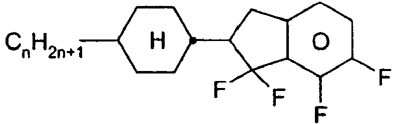 |
| CETNap-n-Om | CK-n-F |
| 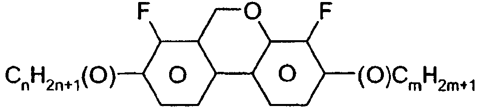 | 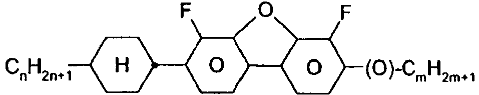 |
| DFDBC-n(O)-(O)m | C-DFDBF-n-(O)m |

In einer bevorzugten Ausführungsform der vorliegenden Erfindung enthalten die erfindungsgemäßen FK-Medien eine oder mehrere Verbindungen ausgewählt aus der Guppe bestehend aus Verbindungen der Tabelle A.

**Tabelle B**

| In der Tabelle B werden mögliche Dotiert toffe angegeben, die den erfindungsgemäßen FK-Medien zugesetzt werden können. | |
|---|---|
| 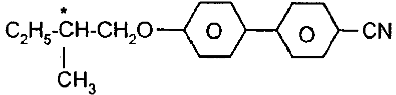 | 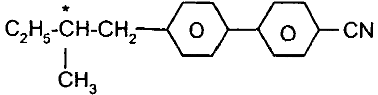 |
| **C 15** | **CB 15** |
| 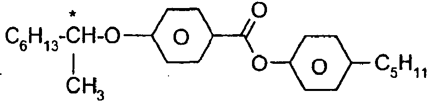 | 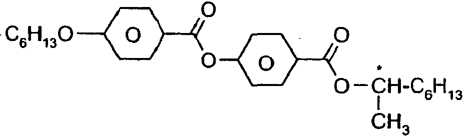 |
| **CM 21** | **R/S-811** |
| 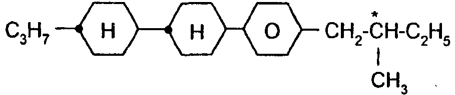 | 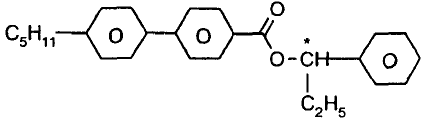 |
| **CM 44** | **CM 45** |
| 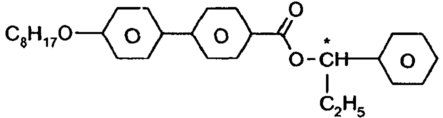 | 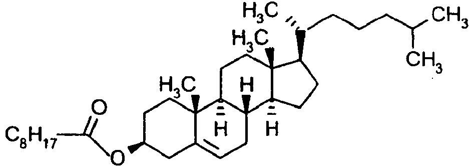 |
| **CM 47** | **CN** |
| 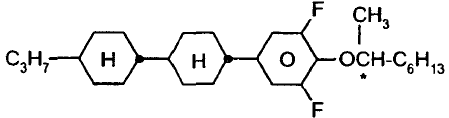 | 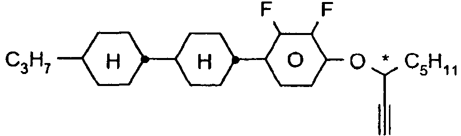 |
| **R/S-2011** | **R/S-3011** |
| 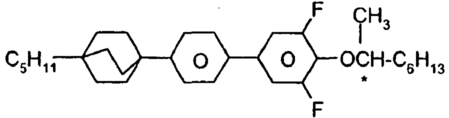 | 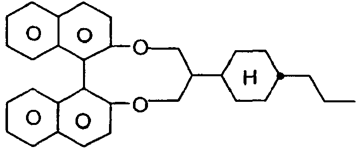 |
| **R/S-4011** | **R/S-5011** |
| 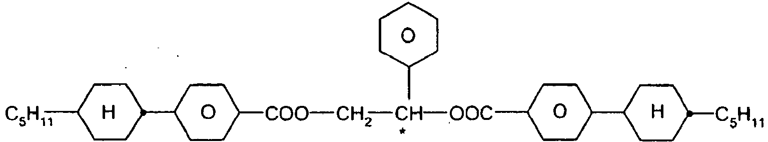 | |
| **R/S-1011** | |

Vorzugsweise enthalten die FK-Medien 0 bis 10 Gew.%, insbesondere 0,01 bis 5 Gew.% und besonders bevorzugt 0,1 bis 3 Gew.% an Dotierstoffen. Vorzugsweise enthalten die FK-Medien einen oder mehrere Dotierstoffe ausgewählt aus der Guppe bestehend aus Verbindungen der Tabelle B.

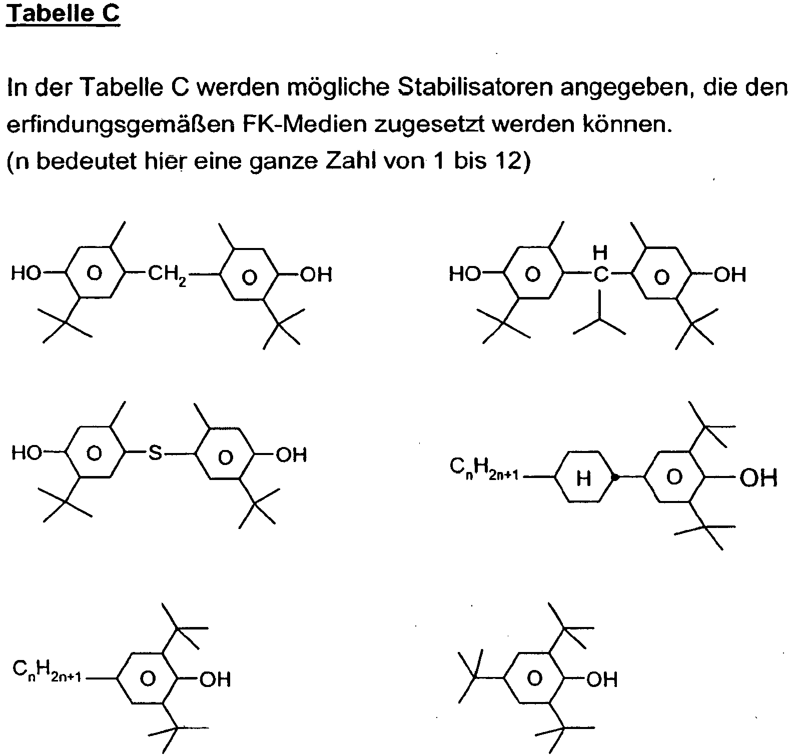

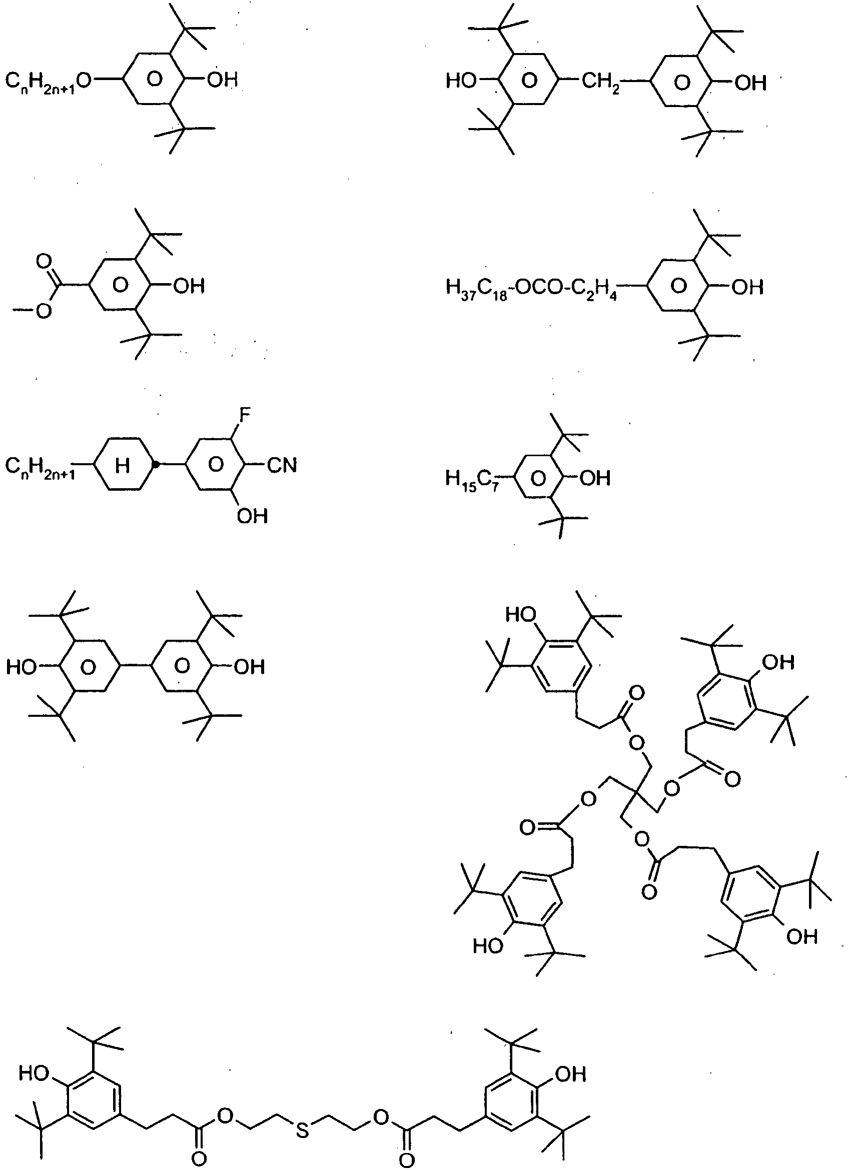

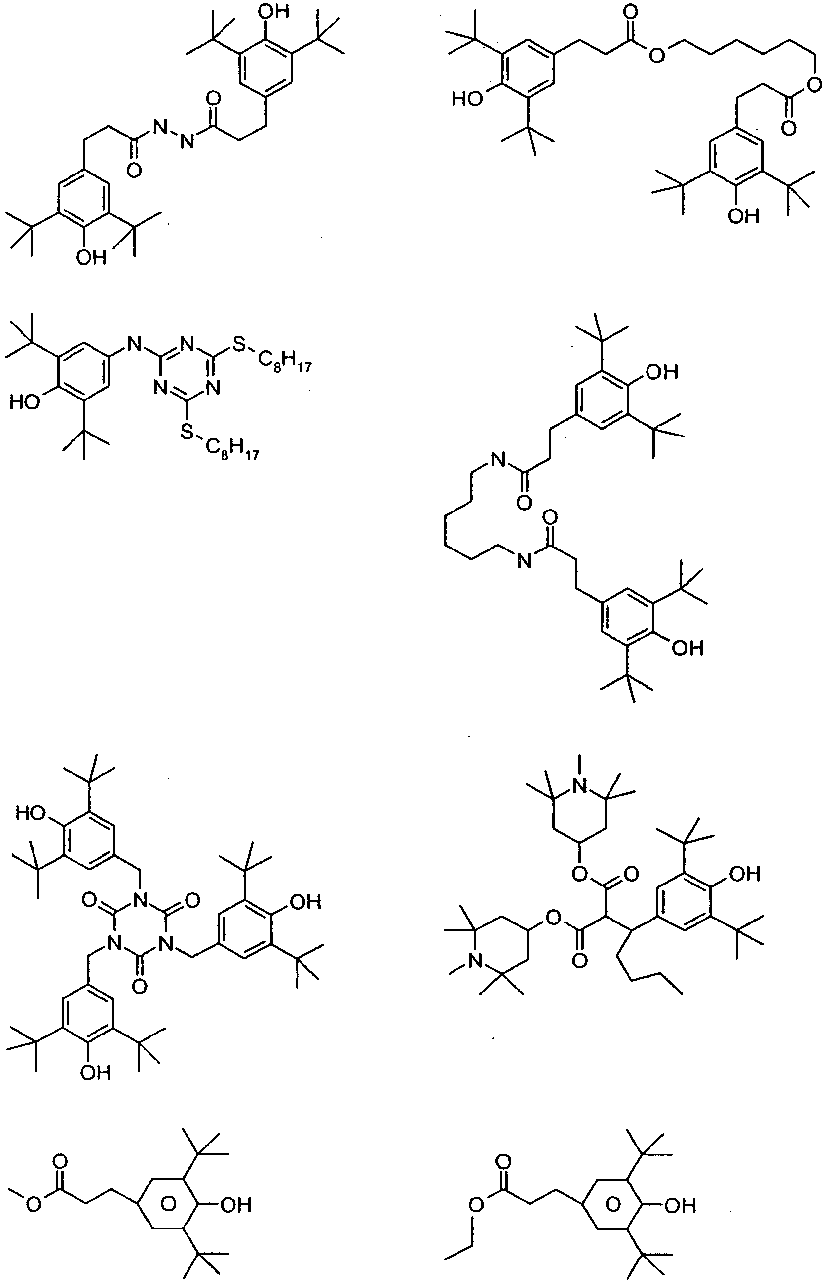

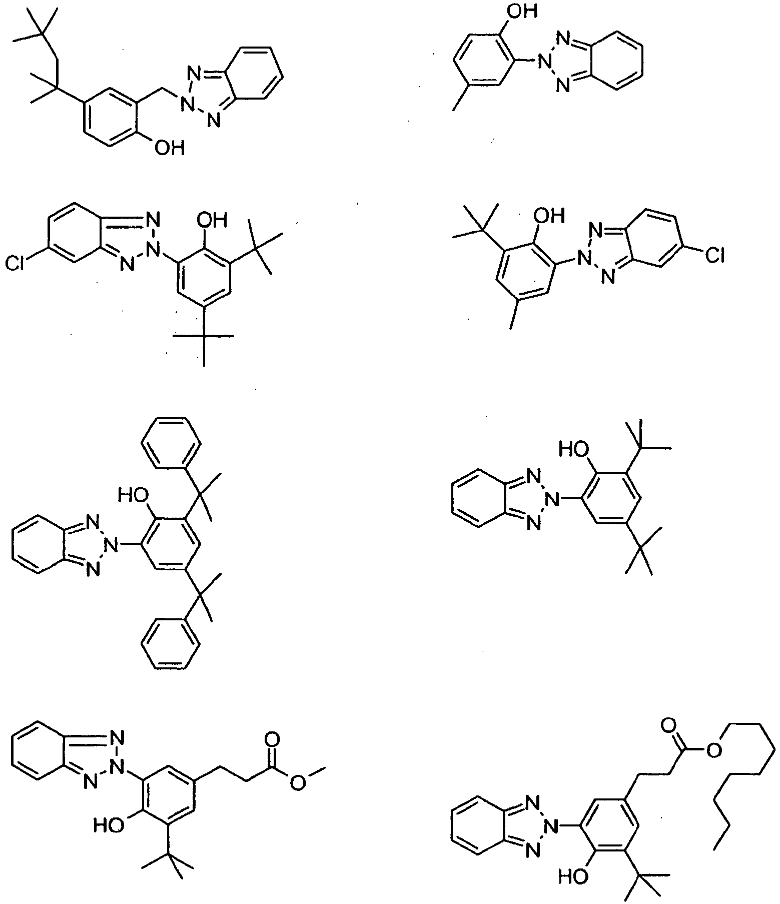

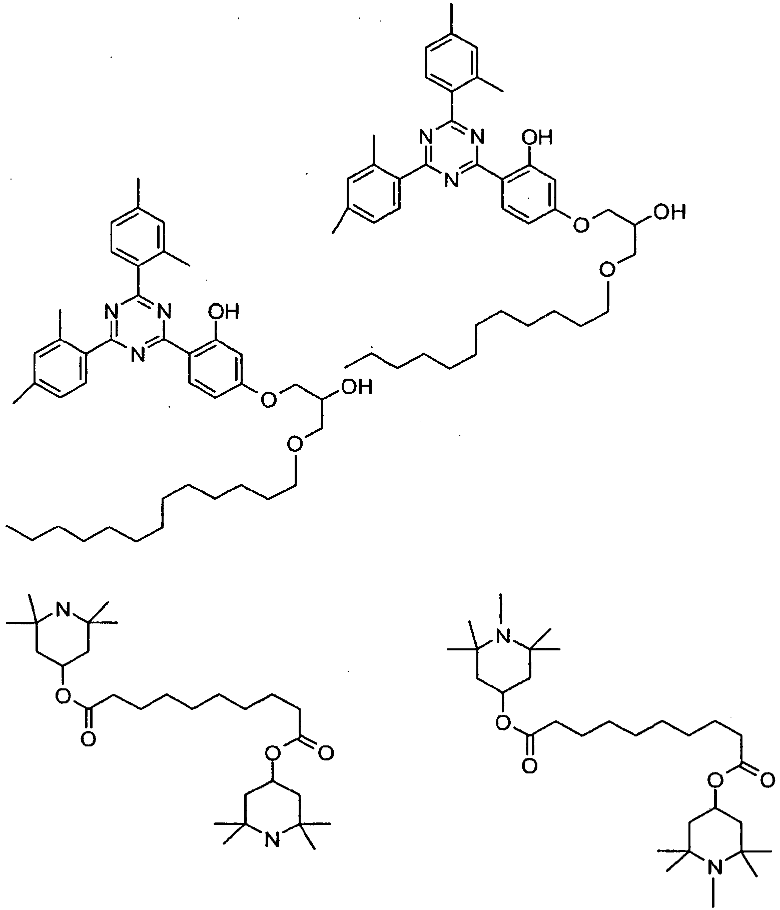

Vorzugsweise enthalten die FK-Medien 0 bis 10 Gew.%, insbesondere 0,01 bis 5 Gew.% und besonders bevorzugt 0,1 bis 3 Gew.% an Stabilisatoren. Vorzugsweise enthalten die FK-Medien einen oder mehrere Stabilisatoren ausgewählt aus der Gruppe bestehend aus Verbindungen der Tabelle C.

Außerdem werden folgende Abkürzungen und Symbole verwendet:
- Vₒ: Schwellenspannung, kapazitiv [V] bei 20°C,
- nₑ: außerordentlicher Brechungsindex bei 20°C und 589 nm,
- nₒ: ordentlicher Brechungsindex bei 20°C und 589 nm,
- Δn: optische Anisotropie bei 20°C und 589 nm,
- ε_{⊥}: dielektrische Suszeptibilität senkrecht zum Direktor bei 20°C und 1 kHz,
- e_{∥}: dielektrische Suszeptibilität parallel zum Direktor bei 20°C und 1 kHz,
- Δε: dielektrische Anisotropie bei 20°C und 1 kHz,
- Kp., T(N,I): Klärpunkt [°C],
- γ₁: Rotationsviskosität bei 20°C [mPa·s],
- K₁: elastische Konstante, "splay"-Deformation bei 20°C [pN],
- K₂: elastische Konstante, "twist"-Deformation bei 20°C [pN],
- K₃: elastische Konstante, "bend"-Deformation bei 20°C [pN],
- LTS: "low temperature stability" (Phase), bestimmt in Testzellen,
- HR₂₀: "voltage holding ratio" bei 20°C [%] und
- HR₁₀₀: "voltage holding ratio" bei 100°C [%].

Soweit nicht explizit anders vermerkt, sind in der vorliegenden Anmeldung alle Konzentrationen in Gewichtsprozent angegeben und beziehen sich auf die entsprechende Gesamtmischung ohne Lösungsmittel.

Soweit nicht explizit anders vermerkt, sind in der vorliegenden Anmeldung alle angegebenen Werte für Temperaturen, wie z. B. der Schmelzpunkt T(C,N), der Übergang von der smektischen (S) zur nematischen (N) Phase T(S,N) und der Klärpunkt T(N,I), in Grad Celsius (°C) angegeben. Fp. bedeutet Schmelzpunkt, Kp. = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar.

Alle physikalischen Eigenschaften werden und wurden nach "Merck Liquid Crystals, Physical Properties of Liquid Crystals", Status Nov. 1997, Merck KGaA, Deutschland bestimmt und gelten für eine Temperatur von 20°C und Δn wird bei 589 nm und Δε bei 1 kHz bestimmt, sofern nicht jeweils explizit anders angegeben.

Der Begriff "Schwellenspannung" bezieht sich für die vorliegende Erfindung auf die kapazitive Schwelle (V₀), auch Freedericksz-Schwelle genannt, sofern nicht explizit anders angegeben. In den Beispielen kann auch, wie allgemein üblich, die optische Schwelle für 10 % relativen Kontrast (V₁₀) angegeben werden.

Die zur Messung der kapazitiven Schwellenspannung verwendete Anzeige weist zwei planparallele Trägerplatten im Abstand von 4 µm und Elektrodenschichten mit darüberliegenden Orientierungsschichten aus geriebenem Polyimid auf den Innenseiten der Trägerplatten auf, welche eine homöotrope Randorientierung der Flüssigkristallmoleküle bewirken.

Die polymerisierbaren Verbindungen werden in der Anzeige durch UV-Bestrahlung bei vorgegebener Zeit polymerisiert, wobei gleichzeitig eine Spannung an die Anzeige angelegt wird (üblicherweise 10V bis 30V Wechselstrom, 1 kHz). In den Beispielen wurde, falls nicht anders angegeben, eine Quecksilberdampflampe mit 28 mW/cm² verwendet, die Intensität wurde mit einem Standard-UV-Meter (Fabrikat Ushio UNI meter) gemessen, der mit einem Bandpassfilter bei 365nm ausgerüstet ist.

Der Tiltwinkel wird per Drehkristall-Experiment (Autronic-Melchers TBA-105) bestimmt. Ein kleiner Wert (d.h. eine große Abweichung vom 90°-Winkel) entspricht dabei einem großen Tilt.

Der HR -Wert wird wie folgt gemessen: Zur FK-Host-Mischung werden 0.3% einer polymerisierbaren monomeren Verbindung zugesetzt, und die dadurch entstandene Mischung in TN-VHR-Testzellen gefüllt (90° gerieben, Orientierungsschicht TN-Polyimid, Schichtdicke d≈4µm). Der HR-Wert wird nach 5min bei 100°C vor und nach 2h UV-Belastung (suntest) bei 1V, 60Hz, 64µs pulse bestimmt (Messgerät: Autronic-Melchers VHRM-105).

### Beispiel 1

Verbindung (A) wird gemäß Schema 1 und 3 hergestellt.

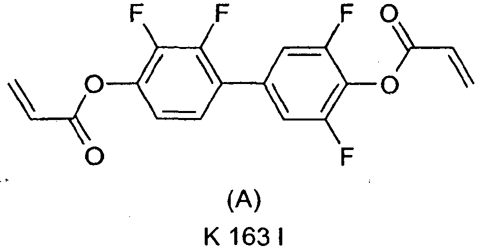

### Schritt a):

76g 2,3-Difluorphenol (1) werden in 400ml Dichlormethan bei Raumtemperatur (RT) unter Rühren und N₂-Atmosphäre vorgelegt, 99ml Triethylamin und 3.5g 4-Dimethylaminopyridin zugegeben, und das Reaktionsgemisch mit einem Trockeneis/Alkohol- Bad auf -15°C eingekühlt. Bei dieser Temperatur wird eine Lösung von 100g Dimethyl-tert.-butylchlorsilan in 100mL Dichlormethan so zugetropft, dass die Temperatur nicht über 0°C ansteigt (es bildet sich eine Suspension). Anschliessend wird das Kältebad entfernt und über Nacht nachgerührt. Es wird vom ausgefallenen Ammoniumchlorid abgesaugt, das Reaktionsgemisch mit 500mL Wasser versetzt und in einen Scheidetrichter überführt, geschüttelt, die wässrige Phase abgelassen und 2x mit tert. Butylmethyl(MTB)-Ether extrahiert. Die vereinigten organischen Phasen werden 2x mit Wasser und 1 x mit Natriumchloridlösung gewaschen, über Natriumsulfat getrocknet, filtriert und eingeengt. Man erhält 147,1g einer gelben Flüssigkeit (2).

### Schritt b):

In einer 4-Liter-Vierhalsapparatur werden 142.5g tert-Butyl-(2,3-difluorphenoxy)-dimethylsilan (2) in 800 ml THF vorgelegt und unter Stickstoff auf -70°C eingekühlt. Dann werden bei -70°C 320.8 ml 1.6M BuLi in Hexan zugetropft, 45 Minuten nachgerührt und zum Schluß 57 ml Trimethylborat bei -70°C zugetropft, wobei ein weißer Niederschlag ausfällt. Nach beendetem Zutropfen wird noch 30 Minuten bei -70°C nachgerührt, dann lässt man den Ansatz auf RT abkühlen, wobei sich der weiße Niederschlag bei ca. 10°C komplett auflöst und eine fast farblose klare Lösung entsteht. Es wird mit 120ml vollentsalztem (VE)-Wasser hydrolysiert und mit 240ml halbkonz. HCl auf pH6 gestellt. Die organische Phase wird abgetrennt, mit Wasser ausgeschüttelt, getrocknet und einrotiert. Die erhaltene braune Masse wird 1:2 aus Hexan bei 0°C ausgerührt, abgesaugt und getrocknet. Man erhält 60,3g beiges Pulver (3).

### Schritt c):

20.2g 2,3-Difluor-4-tert-butyldimethylsiloxyphenylboronsäure (3), 21.2g 4-Brom-2,6-difluor-phenol (4), 21.5 ml Natriummetaboratoctahydrat, 2.2g Bis(triphenylphosphino)palladium(II)chlorid, 0.13g Hydraziniumhydroxid und 200ml THF werden bei RT vereinigt, zum Rückfluß erhitzt und über Nacht unter Rückfluß gerührt. Anschliessend versetzt man das Reaktionsgemisch mit VE-Wasser und lässt auf RT abkühlen. Es wird mit MTB-Ether versetzt, die wäßrige Phase mit MTB-Ether extrahiert, die vereinigten organischen Phasen über Na₂SO₄ getrocknet, abfiltriert und einrotiert. Die braune Masse wird mit Heptan/Essigester 1:1 gefrittet und die entsprechenden Fraktionen einrotiert. Man erhält 27,1 g weiße Kristalle (5).

### Schritt d):

13.5g 2,3,3',5'-Tetrafluoro-4,4'-dihydroxybiphenyl (5) und 8.75ml Chlorproprionsäurechlorid werden in 70ml Dichlormethan vorgelegt, 30ml Triethylamin bei 20°C zugetropft (Eiskühlung) und über Nacht bei RT nachgerührt. Das Reaktionsgemisch wird auf 300ml Wasser gegossen, mit Salzsäure auf pH 2 gestellt und dann abgetrennt. Es wird 2x mit CH₂Cl₂ nachextrahiert, die organische Phase 2x mit Wasser extrahiert, über Na₂SO₄ getrocknet, filtriert und einrotiert. Nach Chromatographie in n-Heptan:Essigester 3:1 und Umkristallisation aus Essigester erhält man 2,1 g weiße Kristalle.

### Beispiel 2

Aus dem nach Beispiel 1, Schritt a)-c) erhaltenen Diol (5) wird Verbindung (B) gemäß Schema 3 hergestellt.

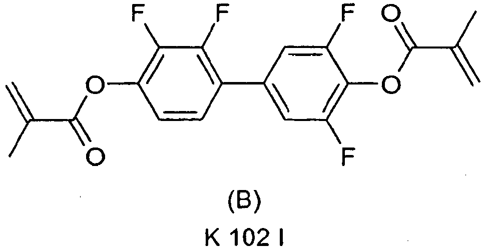

### Schritt h):

13.5g 2,3,3',5'-Tetrafluoro-4,4'-dihydroxybiphenyl (5), 9.1ml Methacrylsäure und 0.42g Dimethylaminopyridin werden in 350ml Toluol vorgelegt. Es wird eine Lösung aus 23.1g DCC in 100ml THF bei maximal 10°C zugetropft und über Nacht gerührt. Am nächsten Tag werden 0.65g Oxalsäuredihydrat dazugegeben und 1 Stunde bei RT nachgerührt. Es wird abgesaugt, mit Toluol gewaschen und einrotiert. Die erhaltenen leicht gelben Kristalle werden über eine Kieselgelsäule mit n-Heptan : Essigester 2:1 gegeben. Die danach erhaltenen weißen Kristalle werden aus 200ml Heptan + Celite heiß über einen 250ml-Seitzfilter filtriert, bei Raumtemperatur umkristallisiert, abgesaugt und im Vakuumschrank getrocknet. Nach mehrfacher Umkristallisation aus Methanol erhält man 6,4g weiße Kristalle.

### Beispiel 3 - Mischungsbeispiel

Die nematische FK-Host-Mischung N1 wird wie folgt formuliert

| | | | |
|---|---|---|---|
| CCH-501 | 9,00 % | Kp. | + 70,0 |
| CCH-35 | 14,00 % | Δn | 0,0825 |
| PCH-53 | 8,00 % | Δε | - 3,5 |
| CY-3-O4 | 14,00 % | ε_{∥} | 3,5 |
| CY-5-O4 | 13,00 % | K₃/K₁ | 1,00 |
| CCY-3-O2 | 8,00 % | γ₁ | 141 |
| CCY-5-O2 | 8,00 % | V₀ | 2,06 |
| CCY-2-1 | 9,00 % | | |
| CCY-3-1 | 9,00 % | | |
| CPY-2-O2 | 8,00 % | | |

Zur FK-Mischung N1 werden 0.3% der polymerisierbaren monomeren Verbindung B aus Beispiel 2 zugesetzt, und die dadurch entstandene Mischung in VA-e/o-Testzellen gefüllt (90° gerieben, Orientierungsschicht VA-Polyimid, Schichtdicke d≈4µm). Unter Anlegen einer Spannung von 10V (Wechselstrom) wird die Zelle 20 Minuten lang mit UV-Licht der Intensität 28mW/cm² bestrahlt, dadurch erfolgt Polymerisation der monomeren Verbindung.

In einem zweiten Versuch wird der FK/Monomer-Mischung noch zusätzlich 0.006% des Photoinitiators Irgacure-651 zugesetzt und die Belichtungszeit auf 2 Minuten verkürzt. Vor und nach der UV-Bestrahlung wird per Drehkristall-Experiment (Autronic-Melchers TBA-105) der Tiltwinkel bestimmt. Die Ergebnisse sind in Tabelle 1 zusammengefasst.

**Tabelle 1**

| Monomer | Initiator | Tilt vor UV | Tilt nach UV |
|---|---|---|---|
| (B) | nein | 89.9° | 86.9° |
| (B) | ja | 89.9° | 86.0° |

Wie aus Tabelle 1 ersichtlich ist, kann mit der erfindungsgemäßen Verbindung B ein ausreichend großer Tilt (d.h. kleiner Tiltwinkel) nach Polymerisation erreicht werden.

## Patentansprüche

1. Verbindung der Formel I 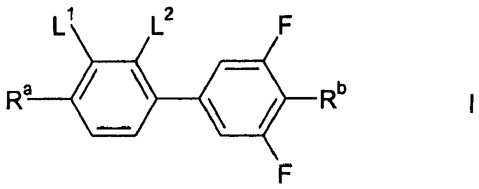 worin die einzelnen Reste folgende Bedeutung besitzen
R^{a} und R^{b} P-Sp-,
P bei jedem Auftreten gleich oder verschieden Vinyloxy, Acrylat, Methacrylat, Fluoracrylat, Chloracrylat, Oxetan oder Epoxy,
Sp eine Einfachbindung,
L¹ und L² jeweils unabhängig voneinander H oder F.

2. Verbindung nach Anspruch 1, **dadurch gekennzeichnet, dass** einer oder beide Reste L¹ und L² F bedeuten.

3. Verbindung nach Anspruch 1 oder 2, ausgewählt aus folgenden Unterformeln 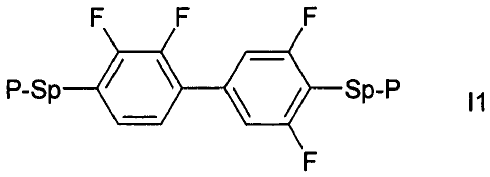 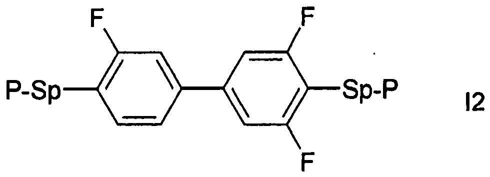 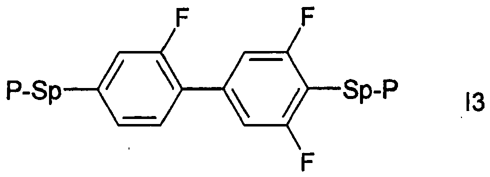 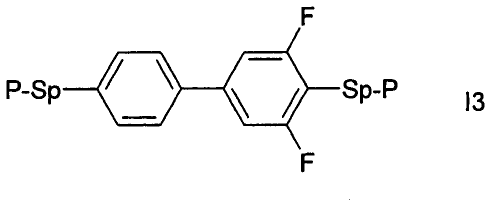 worin P und Sp jeweils unabhängig voneinander die in Anspruch 1 angegebene Bedeutung besitzen.

4. Verfahren zur Herstellung von Verbindungen nach einem oder mehreren der Ansprüche 1 bis 3, enthaltend folgende Schritte:
a) Reaktion der OH-Gruppe von 2,3-Difluorphenol mit einer Schutzgruppe, zum Beispiel ein Trialkylsilylether,
b) Metallierung des Produkts aus Schritt a) in para-Position zur geschützten Phenolgruppe und anschließende Reaktion mit einer Boronsäure oder einem Boronsäureester,
c) Kopplung des Produkts aus Schritt b) mit 4-Halogen-2,6-difluorphenol in Gegenwart eines Übergangsmetallkatalysators und Abspaltung der Schutzgruppe zu 2,3,3',5'-Tetrafluoro-biphenyl-4,4'-diol,
und Schritt g) oder Schritt h):
g) Reaktion der phenolischen OH-Gruppen mit Chlorpropionsäurechlorid und anschließender HCl-Abspaltung,
oder
h) Veretherung oder Veresterung der phenolischen OH-Gruppen mit einer Säure, einem Säurederivat oder einer halogenierten Verbindung enthaltend eine Gruppe P.

5. Verfahren zur Herstellung von Verbindungen nach einem oder mehreren der Ansprüche 1 bis 3, enthaltend folgende Schritte:
d) Reaktion der OH-Gruppe von 4-Halogen-2,6-Difluorphenol mit einer Schutzgruppe, zum Beispiel einem Benzylhalogenid,
e) Dehalogenierung des Produkts aus Schritt d) in para-Position zur geschützten Phenolethergruppe und anschließende Reaktion mit einer Boronsäure oder einem Boronsäureester,
f) Kopplung des Produkts aus Schritt e) mit 4-Halogen-2-fluorphenol bzw. 4-Halogen-3-fluorphenol in Gegenwart eines Übergangsmetallkatalysators und Abspaltung der Schutzgruppe zu 2,3',5'-Trifluoro-biphenyl-4,4'-diol bzw. 3,3',5'-Trifluoro-biphenyl-4,4'-diol,
und Schritt g) oder Schritt h):
g) Reaktion der phenolischen OH-Gruppen mit Chlorpropionsäurechlorid und anschließender HCl-Abspaltung,
oder
h) Veretherung oder Veresterung der phenolischen OH-Gruppen mit einer Säure, einem Säurederivat oder einer halogenierten Verbindung enthaltend eine Gruppe P.

6. Verbindung der Formel la 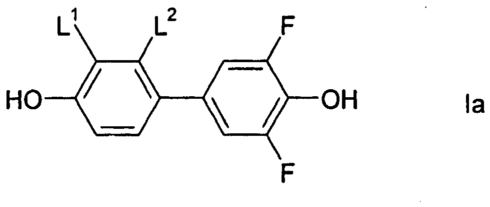 worin L¹ und L² die in Anspruch 1 oder 2 angegebene Bedeutung besitzen.

7. Verbindung nach Anspruch 6, ausgewählt aus folgenden Unterformeln

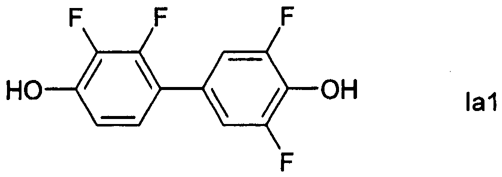

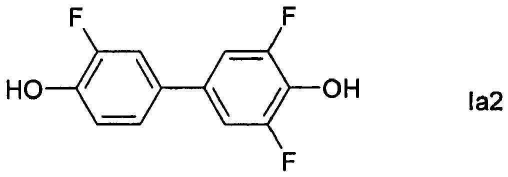

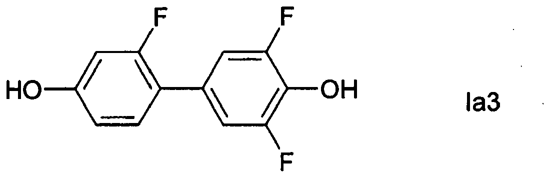

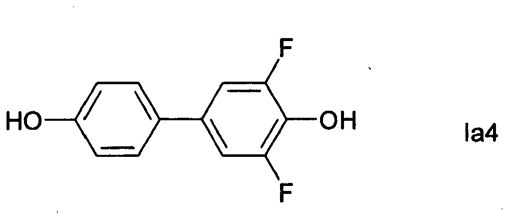

8. Flüssigkristall (FK)-Medium enthaltend eine oder mehrere Verbindungen nach einem oder mehreren der Ansprüche 1 bis 3 und eine oder mehrere zusätzliche Verbindungen, welche auch mesogen, flüssigkristallin und/oder polymerisierbar sein können.

9. FK-Medium enthaltend
- eine polymerisierbare Komponente A), enthaltend eine oder mehrere Verbindungen nach einem oder mehreren der Ansprüche 1 bis 3,
sowie
- eine flüssigkristalline Komponente B) enthaltend eine oder mehrere niedermolekulare Verbindungen,

10. FK-Medium nach Anspruch 8 oder 9, **dadurch gekennzeichnet, dass** es eine oder mehrere Verbindungen der folgenden Formel enthält: 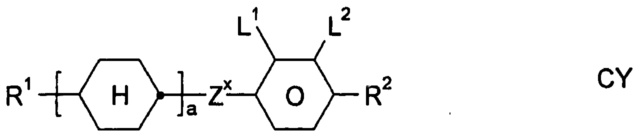 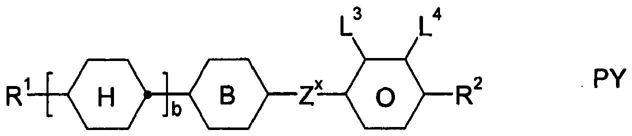 worin die einzelnen Reste folgende Bedeutung besitzen
a 1 oder 2,
b 0 oder 1, 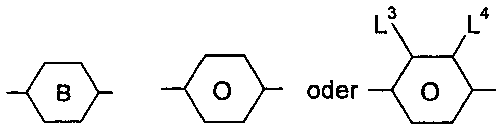
R¹ und R² jeweils unabhängig voneinander Alkyl mit 1 bis 12 C-Atomen, wobei auch eine oder zwei nicht benachbarte CH₂-Gruppen durch -O-, -CH=CH-, -CO-, -OCO- oder - COO- so ersetzt sein können, dass O-Atome nicht direkt miteinander verknüpft sind,
Z^{x} -CH=CH-, -CH₂O-, -OCH₂-, -CF₂O-, -OCF₂-, -O-, -CH₂-, -CH₂CH₂-, oder eine Einfachbindung, vorzugsweise eine Einfachbindung,
L¹⁻⁴ jeweils unabhängig voneinander F, Cl, OCF₃, CF₃, CH₃, CH₂F, CHF₂.

11. FK-Medium nach einem oder mehreren der Ansprüche 8 bis 10, **dadurch gekennzeichnet, dass** es eine oder mehrere Verbindungen der folgenden Formel enthält: 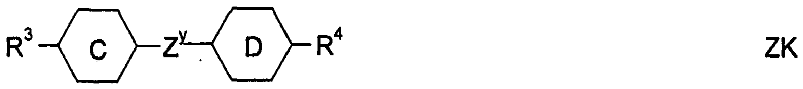 worin die einzelnen Reste folgende Bedeutung besitzen 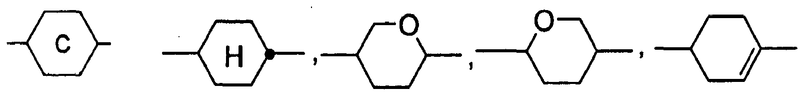 oder 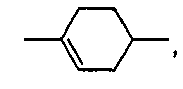 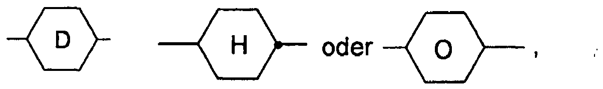
R³ und R⁴ jeweils unabhängig voneinander Alkyl mit 1 bis 12 C-Atomen, worin auch eine oder zwei nicht benachbarte CH₂-Gruppen durch -O-, -CH=CH-, -CO-, -OCO- oder - COO- so ersetzt sein können, dass O-Atome nicht direkt miteinander verknüpft sind,
Z^{y} -CH₂CH₂-, -CH=CH-, -CF₂O-, -OCF₂-, -CH₂O-, -OCH₂-, -COO-, -OCO-, -C₂F₄-, -CF=CF- oder eine Einfachbindung.

12. Polymer erhältlich durch Polymerisation einer oder mehrerer Verbindungen nach einem oder mehreren der Ansprüche 1 bis 3 oder eines FK-Mediums nach einem oder mehreren der Ansprüche 8 bis 11.

13. Polymerfolie erhältlich durch Polymerisation einer Schicht, enthaltend eine oder mehrere Verbindungen nach einem oder mehreren der Ansprüche 1 bis 3 oder ein FK-Medium nach einem oder mehreren der Ansprüche 8 bis 11, im einheitlich ausgerichteten Zustand in der FK-Phase.

14. Verwendung von Verbindungen, FK-Medien, Polymeren und Polymerfolien nach einem oder mehreren der Ansprüche 1 bis 13 in elektrooptischen Anzeigen, Flüssigkristall (FK)-Anzeigen, optischen Filmen, Polarisatoren, Kompensatoren, Strahlenteilern, Reflektivpolarisatoren, Orientierungsschichten, Farbfilteren, holographischen Elementen, Heissprägefolien, Adhäsionsfolien, optischen Datenspeichern, in der nichtlinearen Optik, Effektpigmenten, dekorativen Elementen, Sicherheitselementen, Sicherheitsmarkierungen, elektrischen Halbleitern, organischen Feldeffekttransistoren (OFET), integrierten Schaltkreise (IC), Dünnfilmtransistoren (TFT), Radiofrequenz-Identifikationselementen (RFID), organischen Leuchtdioden (OLED), elektrolumineszierenden Anzeigen, Beleuchtungselementen, photovoltaischen Vorrichtungen, optischen Sensoren, Photoleitern, elektrophotographischen Anwendungen, oder kosmetischen Formulierungen oder Anwendungen.

15. FK-Anzeige enthaltend eine oder mehrer Verbindungen, ein FK-Medium, ein Polymer oder eine Polymerfolie nach einem oder mehreren der Ansprüche 1 bis 13.

16. FK-Anzeige des PS- (polymer stabilized) oder PSA- (polymer sustained alignment) Typs, enthaltend eine FK-Zelle bestehend aus zwei Substraten, wobei mindestens ein Substrat lichtdurchlässig ist und mindestens ein Substrat eine Elektrodenschicht aufweist, sowie einer zwischen den Substraten befindlichen Schicht eines FK-Mediums enthaltend eine polymerisierte Komponente und eine niedermolekulare Komponente, wobei die polymerisierte Komponente erhältlich ist durch Polymerisation einer oder mehrerer polymerisierbarer Verbindungen zwischen den Substraten der FK-Zelle im FK-Medium unter Anlegen einer elektrischen Spannung, **dadurch gekennzeichnet, dass** die FK-Anzeige eine oder mehrere Verbindungen nach einem oder mehreren der Ansprüche 1 bis 3 oder ein FK-Medium nach einem oder mehreren der Ansprüche 8 bis 11 enthält.

17. FK-Anzeige nach Anspruch 15 oder 16, **dadurch gekennzeichnet, dass** sie eine PSA-VA-, PSA-OCB-, PS-IPS-, PS-FFS- oder PS-TN-Anzeige ist.

## Claims

1. Compound of the formula I 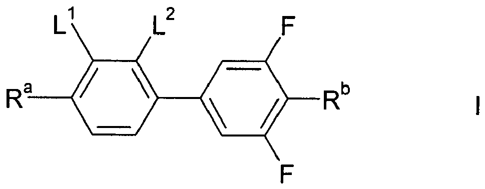 in which the individual radicals have the following meaning:
R^{a} and R^{b} denote P-Sp-,
P on each occurrence, identically or differently, denotes vinyloxy, acrylate, methacrylate, fluoroacrylate, chloroacrylate, oxetane or epoxy,
Sp denotes a single bond,
L¹ and L² each, independently of one another, denote H or F.

2. Compound according to Claim 1, **characterised in that** one or both radicals L¹ and L² denote F.

3. Compound according to Claim 1 or 2, selected from the following sub-formulae: 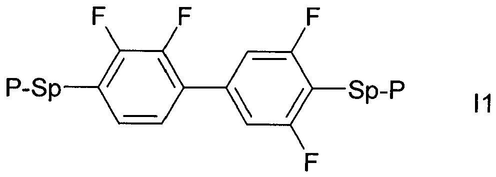 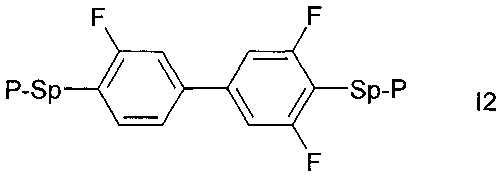 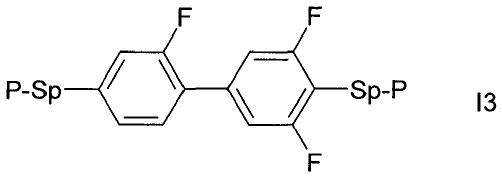 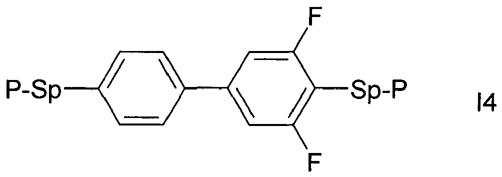 in which P and Sp each, independently of one another, have the meaning indicated in Claim 1.

4. Process for the preparation of compounds according to one or more of Claims 1 to 3, comprising the following steps:
a) reaction of the OH group of 2,3-difluorophenol with a protecting group, for example a trialkylsilyl ether,
b) metallation of the product from step a) in the para-position to the protected phenol group and subsequent reaction with a boronic acid or a boronic acid ester,
c) coupling of the product from step b) to 4-halo-2,6-difluorophenol in the presence of a transition-metal catalyst and removal of the protecting group to give 2,3,3',5'-tetrafluorobiphenyl-4,4'-diol,
and step g) or step h):
g) reaction of the phenolic OH groups with chloropropionyl chloride and subsequent elimination of HCl,
or
h) etherification or esterification of the phenolic OH groups using an acid, an acid derivative or a halogenated compound containing a group P.

5. Process for the preparation of compounds according to one or more of Claims 1 to 3, comprising the following steps:
d) reaction of the OH group of 4-halo-2,6-difluorophenol with a protecting group, for example a benzyl halide,
e) dehalogenation of the product from step d) in the para-position to the protected phenol ether group and subsequent reaction with a boronic acid or a boronic acid ester,
f) coupling of the product from step e) to 4-halo-2-fluorophenol or 4-halo-3-fluorophenol in the presence of a transition-metal catalyst and removal of the protecting group to give 2,3',5'-trifluoro-biphenyl-4,4'-diol or 3,3',5'-trifluorobiphenyl-4,4'-diol respectively,
and step g) or step h):
g) reaction of the phenolic OH groups with chloropropionyl chloride and subsequent elimination of HCl,
or
h) etherification or esterification of the phenolic OH groups using an acid, an acid derivative or a halogenated compound containing a group P.

6. Compound of the formula la 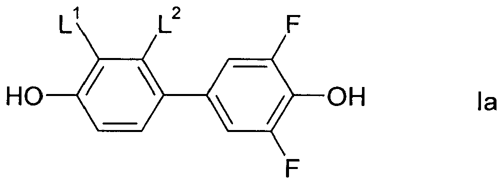 in which L¹ and L² have the meaning indicated in Claim 1 or 2.

7. Compound according to Claim 6, selected from the following sub-formulae:

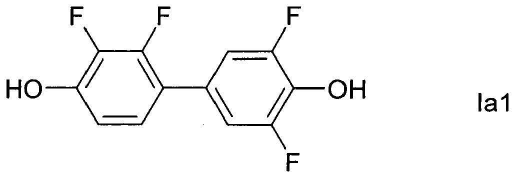

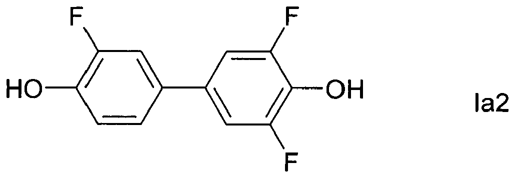

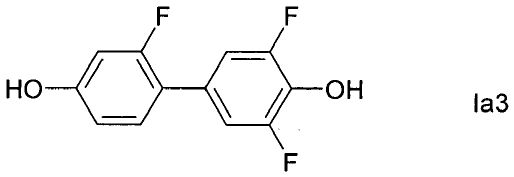

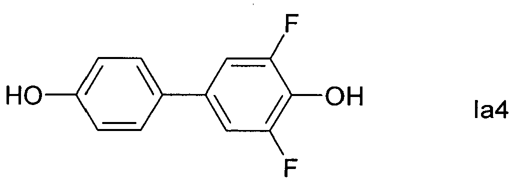

8. Liquid-crystal (LC) medium comprising one or more compounds according to one or more of Claims 1 to 3 and one or more additional compounds, which may also be mesogenic, liquid-crystalline and/or polymerisable.

9. LC medium comprising
- a polymerisable component A) comprising one or more compounds according to one or more of Claims 1 to 3,
and
- a liquid-crystalline component B) comprising one or more low-molecular-weight compounds.

10. LC medium according to Claim 8 or 9, **characterised in that** it comprises one or more compounds of the following formulae: 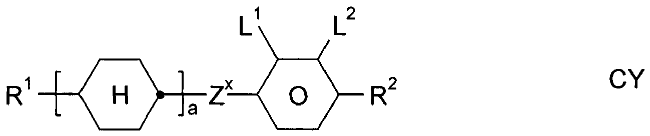 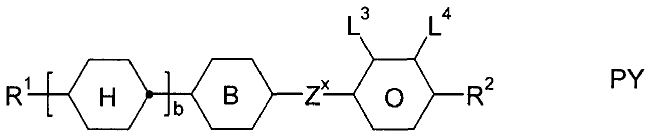 in which the individual radicals have the following meaning:
a denotes 1 or 2,
b denotes 0 or 1, 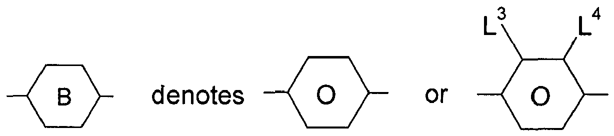
R¹ and R² each, independently of one another, denote alkyl having 1 to 12 C atoms, in which, in addition, one or two non-adjacent CH₂ groups may be replaced by -O-, -CH=CH-, -CO-, -OCO- or -COO- in such a way that O atoms are not linked directly to one another,
Z^{x} denotes -CH=CH-, -CH₂O-, -OCH₂-, -CF₂O-, -OCF₂-, -O-, -CH₂-, -CH₂CH₂- or a single bond, preferably a single bond,
L¹⁻⁴ each, independently of one another, denote F, Cl, OCF₃, CF₃, CH₃, CH₂F, CHF₂.

11. LC medium according to one or more of Claims 8 to 10, **characterised in that** it comprises one or more compounds of the following formula: 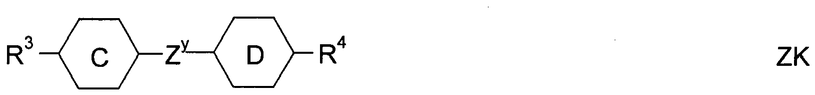 in which the individual radicals have the following meaning: 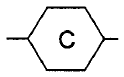 denotes 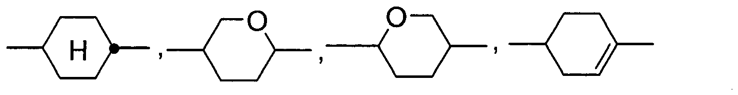 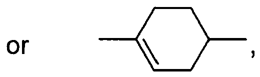 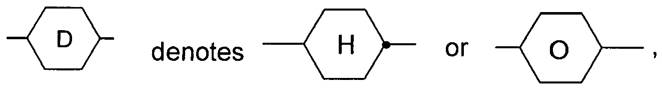
R³ and R⁴ each, independently of one another, denote alkyl having 1 to 12 C atoms, in which, in addition, one or two non-adjacent CH₂ groups may be replaced by -O-, -CH=CH-, -CO-, -OCO- or -COO- in such a way that O atoms are not linked directly to one another,
Z^{y} denotes -CH₂CH₂-, -CH=CH-, -CF₂O-, -OCF₂-, -CH₂O-, -OCH₂-, -COO-, -OCO-, -C₂F₄-, -CF=CF- or a single bond.

12. Polymer obtainable by polymerisation of one or more compounds according to one or more of Claims 1 to 3 or of an LC medium according to one or more of Claims 8 to 11.

13. Polymer film obtainable by polymerisation of a layer comprising one or more compounds according to one or more of Claims 1 to 3 or an LC medium according to one or more of Claims 8 to 11 in the uniformly aligned state in the LC phase.

14. Use of compounds, LC media, polymers and polymer films according to one or more of Claims 1 to 13 in electro-optical displays, liquid-crystal (LC) displays, optical films, polarisers, compensators, beam splitters, reflective polarisers, alignment layers, coloured filters, holographic elements, heat-sealing films, adhesion films, optical data storage media, in nonlinear optics, effect pigments, decorative elements, security elements, security markings, electrical semiconductors, organic field-effect transistors (OFETs), integrated circuits (ICs), thin-film transistors (TFTs), radio frequency identification elements (RFIDs), organic light-emitting diodes (OLEDs), electroluminescent displays, illumination elements, photovoltaic devices, optical sensors, photoconductors, electrophotographic applications, or cosmetic formulations or applications.

15. LC display containing one or more compounds, an LC medium, a polymer or a polymer film according to one or more of Claims 1 to 13.

16. LC display of the PS (polymer-stabilised) or PSA (polymer-sustained alignment) type, containing an LC cell consisting of two substrates, where at least one substrate transmits light and at least one substrate has an electrode layer, and a layer of an LC medium comprising a polymerised component and a low-molecular-weight component which is located between the substrates, where the polymerised component is obtainable by polymerisation of one or more polymerisable compounds between the substrates of the LC cell in the LC medium with application of an electrical voltage, **characterised in that** the LC display contains one or more compounds according to one or more of Claims 1 to 3 or an LC medium according to one or more of Claims 8 to 11.

17. LC display according to Claim 15 or 16, **characterised in that** it is a PSA-VA, PSA-OCB, PS-IPS, PS-FFS or PS-TN display.

## Revendications

1. Composé de la formule I : 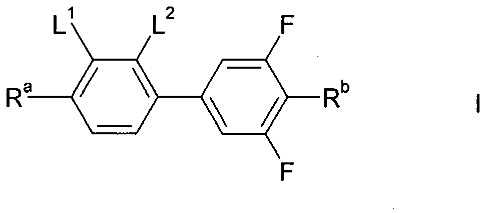 dans laquelle les radicaux individuels présentent la signification qui suit :
R^{a} et R^{b} représentent P-Sp-,
P représente, pour chaque occurrence, de manière identique ou différente, vinyloxy, acrylate, méthacrylate, fluoroacrylate, chloroacrylate, oxétane ou époxy,
Sp représente une liaison simple,
L¹ et L² représentent, chacun indépendamment de l'autre, H ou F.

2. Composé selon la revendication 1, **caractérisé en ce que** l'un des radicaux L¹ et L² ou les deux représente(nt) F.

3. Composé selon la revendication 1 ou 2, choisi parmi les sous-formules qui suivent : 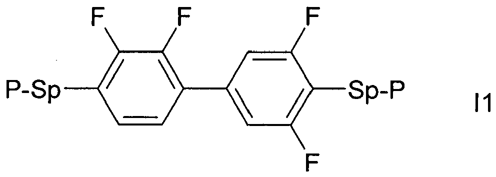 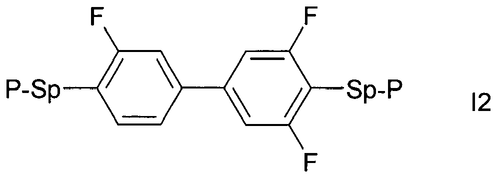 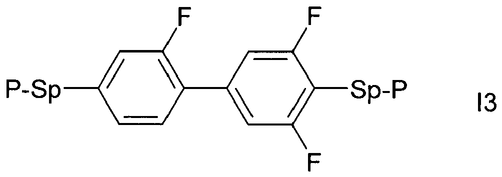 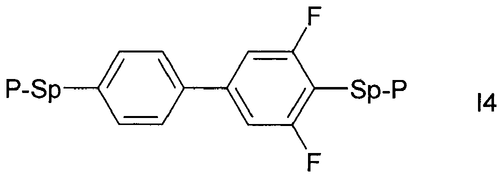 dans lesquelles P et Sp présentent, chacun indépendamment de l'autre, la signification indiquée selon la revendication 1.

4. Procédé pour la préparation de composés selon une ou plusieurs des revendications 1 à 3, comprenant les étapes qui suivent :
a) réaction du groupe OH du 2,3-difluorophénol avec un groupe de protection, par exemple un éther de trialkylsilyle,
b) métallation du produit issu de l'étape a) à la position para sur le groupe phénol protégé et ensuite, réaction avec un acide boronique ou un ester d'acide boronique,
c) couplage du produit issu de l'étape b) sur du 4-halo-2,6-difluorophénol en présence d'un catalyseur constitué par un métal de transition et enlèvement du groupe de protection pour obtenir du 2,3,3',5'-tétrafluorobiphényl-4,4'-diol,
et étape g) ou étape h) :
g) réaction des groupes OH phénoliques avec du chlorure de chloropropionyle et ensuite, élimination du HCl,
ou
h) éthérification ou estérification des groupes OH phénoliques en utilisant un acide, un dérivé d'acide ou un composé halogéné contenant un groupe P.

5. Procédé pour la préparation de composés selon une ou plusieurs des revendications 1 à 3, comprenant les étapes qui suivent :
d) réaction du groupe OH du 4-halo-2,6-difluorophénol avec un groupe de protection, par exemple un halogénure de benzyle,
e) déshalogénisation du produit issu de l'étape d) à la position para sur le groupe éther de phénol protégé et ensuite, réaction avec un acide boronique ou un ester d'acide boronique,
f) couplage du produit issu de l'étape e) sur du 4-halo-2-fluoro-phénol ou du 4-halo-3-fluorophénol en présence d'un catalyseur constitué par un métal de transition et enlèvement du groupe de protection pour obtenir respectivement du 2,3',5'-trifluoro-biphényl-4,4'-diol ou du 3,3',5'-trifluorobiphényl-4,4'-diol,
et étape g) ou étape h) :
g) réaction des groupes OH phénoliques avec du chlorure de chloropropionyle et ensuite, élimination du HCl,
ou
h) éthérification ou estérification des groupes OH phénoliques en utilisant un acide, un dérivé d'acide ou un composé halogéné contenant un groupe P.

6. Composé de la formule la : 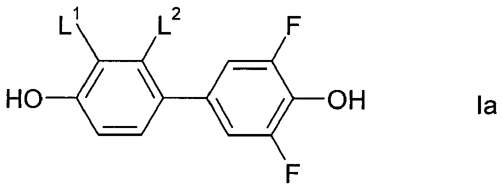 dans laquelle L¹ et L² présentent la signification indiquée selon la revendication 1 ou 2.

7. Composé selon la revendication 6, choisi parmi les sous-formules qui suivent :

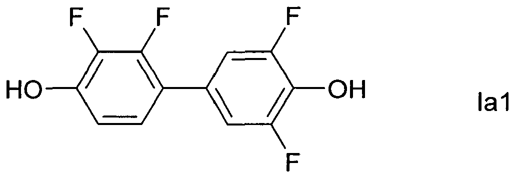

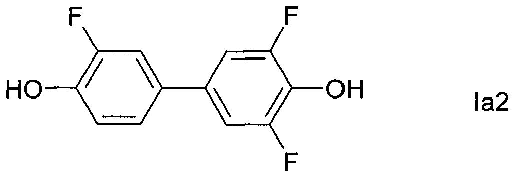

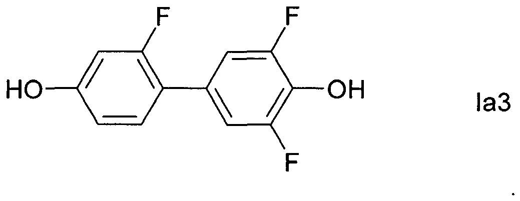

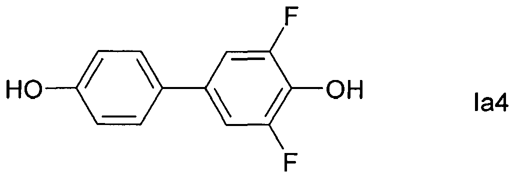

8. Milieu cristallin liquide (LC) comprenant un ou plusieurs composé(s) selon une ou plusieurs des revendications 1 à 3 et un ou plusieurs composé(s) additionnel(s), lesquels peuvent également être méso-gènes, cristallins liquides et/ou polymérisables.

9. Milieu LC comprenant :
- un composant polymérisable A) comprenant un ou plusieurs composé(s) selon une ou plusieurs des revendications 1 à 3,
et
- un composant cristallin liquide B) comprenant un ou plusieurs composés de poids moléculaire faible.

10. Milieu LC selon la revendication 8 ou 9, **caractérisé en ce qu'**il comprend un ou plusieurs composé(s) des formules qui suivent : 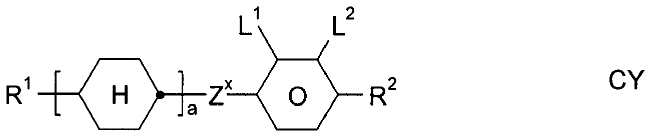 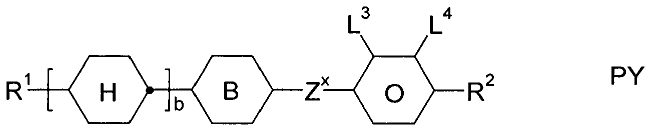 dans lesquelles les radicaux individuels présentent la signification qui suit :
a représente 1 ou 2,
b représente 0 ou 1, 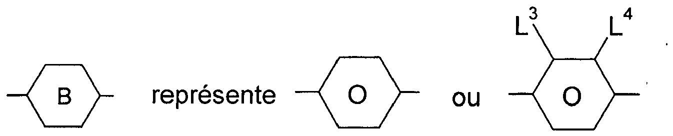
R¹ et R² représentent, chacun indépendamment de l'autre, alkyle comportant de 1 à 12 atome(s) de C, où, en outre, un ou deux groupe(s) CH₂ non adjacents peut/peuvent être remplacé(s) par -O-, -CH=CH-, -CO-, -OCO- ou -COO- de telle sorte que des atomes de O ne soient pas liés directement les uns aux autres,
Z^{x} représente -CH=CH-, -CH₂O-, -OCH₂-, -CF₂O-, -OCF₂-, -O-, -CH₂-, -CH₂CH₂- ou une liaison simple, de façon préférable, une liaison simple,
L¹⁻⁴ représentent, chacun indépendamment des autres, F, Cl, OCF₃, CF₃, CH₃, CH₂F, CHF₂.

11. Milieu LC selon une ou plusieurs des revendications 8 à 10, **caractérisé en ce qu'**il comprend un ou plusieurs composé(s) de la formule qui suit : 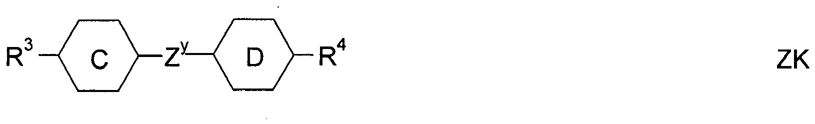 dans laquelle les radicaux individuels présentent la signification qui suit : 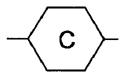 représente 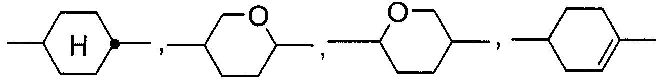 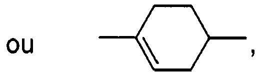 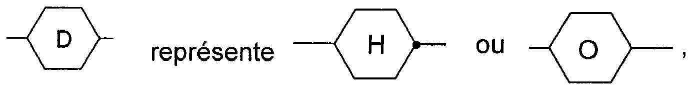
R³ et R⁴ représentent, chacun indépendamment de l'autre, alkyle comportant de 1 à 12 atomes de C, où, en outre, un ou deux groupe(s) CH₂ non adjacents peut/peuvent être remplacé(s) par -O-, -CH=CH-, -CO-, -OCO- ou -COO- de telle sorte que des atomes de O ne soient pas liés directement les uns aux autres,
Z^{y} représente -CH₂CH₂-, -CH=CH-, -CF₂O-, -OCF₂-, -CH₂O-, -OCH₂-, -COO-, -OCO-, -C₂F₄-, -CF=CF- ou une liaison simple.

12. Polymère pouvant être obtenu par polymérisation d'un ou de plusieurs composé(s) selon une ou plusieurs des revendications 1 à 3 ou d'un milieu LC selon une ou plusieurs des revendications 8 à 11.

13. Film en polymère pouvant être obtenu par polymérisation d'une couche comprenant un ou plusieurs composé(s) selon une ou plusieurs des revendications 1 à 3 ou un milieu LC selon une ou plusieurs des revendications 8 à 11 dans l'état aligné de façon uniforme dans la phase LC.

14. Utilisation de composés, de milieux LC, de polymères et de films en polymère selon une ou plusieurs des revendications 1 à 13 dans des affichages électro-optiques, des affichages à cristaux liquides (LC), des films optiques, des polariseurs, des compensateurs, des séparateurs de faisceau, des polariseurs réfléchissants, des couches d'alignement, des filtres couleur, des éléments holographiques, des films de scellement thermique, des films d'adhérence, des milieux de stockage de données optiques, dans des optiques non linéaires, des pigments d'effet, des éléments décoratifs, des éléments de sécurité, des marquages de sécurité, des semiconducteurs électriques, des transistors à effet de champ organiques (OFET), des circuits intégrés (IC), des transistors à film mince (TFT), des éléments d'identification radiofréquence (RFID), des diodes émettrices de lumière organiques (OLED), des affichages électroluminescents, des éléments d'éclairage, des dispositifs photovoltaïques, des capteurs optiques, des photoconducteurs, des applications électrophotographiques ou des formulations ou applications cosmétiques.

15. Affichage LC contenant un ou plusieurs composé(s), un milieu LC, un polymère ou un film en polymère selon une ou plusieurs des revendications 1 à 13.

16. Affichage LC du type PS (stabilisé par polymère) ou PSA (à alignement soutenu par polymère), contenant une cellule LC constituée par deux substrats, où au moins un substrat laisse passer la lumière et au moins un substrat comporte une couche d'électrode, et une couche en un milieu LC comprenant un composant polymérisé et un composant de poids moléculaire faible qui est située entre les substrats, où le composant polymérisé peut être obtenu par polymérisation d'un ou de plusieurs composé(s) polymérisable(s) entre les substrats de la cellule LC dans le milieu LC moyennant l'application d'une tension électrique, **caractérisé en ce que** l'affichage LC contient un ou plusieurs composé(s) selon une ou plusieurs des revendications 1 à 3 ou un milieu LC selon une ou plusieurs des revendications 8 à 11.

17. Affichage LC selon la revendication 15 ou 16, **caractérisé en ce qu'**il s'agit d'un affichage PSA-VA, PSA-OCB, PS-IPS, PS-FFS ou PS-TN.
